(19) Europäisches Patentamt
European Patent Office
Office européen des brevets

(11) Veröffentlichungsnummer: **0 283 829 B1**

(12) **EUROPÄISCHE PATENTSCHRIFT**

(45) Veröffentlichungstag der Patentschrift: **04.08.93**

(51) Int. Cl.[5]: **C12N 15/30**, A61K 39/015, C07K 13/00, C12N 1/20, C07K 15/00, C12P 21/00, C12P 21/02

(21) Anmeldenummer: **88103564.6**

(22) Anmeldetag: **08.03.88**

Die Akte enthält technische Angaben, die nach dem Eingang der Anmeldung eingereicht wurden und die nicht in dieser Patentschrift enthalten sind.

(54) **Plasmodium falciparum Merozoitenantigen-Peptide.**

(30) Priorität: **19.03.87 GB 8706599**

(43) Veröffentlichungstag der Anmeldung:
**28.09.88 Patentblatt 88/39**

(45) Bekanntmachung des Hinweises auf die Patenterteilung:
**04.08.93 Patentblatt 93/31**

(84) Benannte Vertragsstaaten:
**AT BE CH DE ES FR GB IT LI NL SE**

(56) Entgegenhaltungen:

**THE EMBO JOURNAL, Band 4, Nr. 13B, Dezember 1985, Seiten 3823-3829, IRL PressLtd., Oxford, GB; M. MACKAY et al.: "Polymorphism of the precursor for themajor surface antigens of Plasmodium falciparum merozoites: studies at thegenetic level"**

(73) Patentinhaber: **F. HOFFMANN-LA ROCHE AG**
**Postfach 3255**
**CH-4002 Basel(CH)**

(72) Erfinder: **Certa, Ulrich, Dr.**
**Steinbühlweg 21**
**CH-4123 Allschwil(CH)**
Erfinder: **Gentz, Reiner, Dr.**
**Am Hochgericht 34**
**W-7888 Rheinfelden(DE)**
Erfinder: **Takacs, Béla, Dr.**
**Dornacherstrasse 53**
**CH-4147 Aesch(CH)**

(74) Vertreter: **Mezger, Wolfgang, Dr. et al**
**Grenzacherstrasse 124 Postfach 3255**
**CH-4002 Basel (CH)**

**EP 0 283 829 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**NUCLEIC ACIDS RESEARCH, Band 14, Nr. 8, April 1986, Seiten 3311-3323; J.L.WEBER et al.: "Variation in the gene encoding a major merozoite surface antigenof the human malaria parasite Plasmodium falciparum"**

**NATURE, Band 317, 19. September 1985, Seiten 270-273; A.A. HOLDER et al.:"Primary structure of the precursor to the three major surface antigens ofPlasmodium falciparum merozoites"**

**THE EMBO JOURNAL, Band 4, Nr. 4, April 1985, Seiten 1007-1012, IRL Press Ltd,Oxford, GB; A. CHEUNG et al.: "Cloning and expression in Escherichia coli of asurface antigen of Plasmodium falciparum merozoites"**

## Beschreibung

Die vorliegende Erfindung betrifft Polypeptide mit einer Aminosäuresequenz die vom 190 kD (1kD-1000 Dalton) Vorläufer der vorwiegenden Merozoiten-Oberflächenantigene des K1 Isolates von P. falciparum abgeleitet ist. Diese Polypeptide sind fähig eine Immunantwort gegen verschiedene Isolate von P. falciparum hervorzurufen. Insbesondere betrifft die vorliegende Erfindung synthetische Polypeptide der Formel

A-B-C (I)

worin

A ein Affinitätspeptidrest ist oder fehlen kann

B

ThrLeuCysAspAsnIleHisGlyPheLysTyrLeuIleAspGlyTyrGluGluIle
AsnGluLeuLeuTyrLysLeuAsnPheTyrPheAspLeuLeuArgAlaLysLeuAsn
AsnValCysAlaAsnAspTyrCysGlnIleProPheAsnLeuLysIleArgAlaAsn
GluLeuAspValLeuLysLysLeuValPheGlyTyrArgLysProLeuAspAsnIle
LysAspAsnValGlyLysMetGluAspTyrIleLysLysAsnLysLysThrIleGlu
AsnIleAsnGluLeuIleGluGluSerLysLysThrIleAspLysAsnLysAsnAla
ThrLysGluGluGluLysLysLysLeuTyrGlnAlaGlnTyrAspLeuPheIleTyr

AsnLysGlnLeuGluGluAlaHisAsnLeuIleSerValLeuGluLysArgIleAsp
ThrLeuLysLysAsnGluAsnIleLysGluLeuLeuAspLysIleAsnGluIleLys
AsnProProPro

oder ein Fragment davon, oder

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

oder ein Fragment davon ist, oder eine Kombination dieser Sequenzen oder Fragmente, vorausgesetzt, dass die obengenannten Fragmente mindestens ein Epitop des 190kD Vorläufers der vorwiegenden Merozoiten-Oberflächenantigene von P. falciparum darstellen oder enthalten und

C ein Peptidrest ist oder fehlen kann, dadurch gekennzeichnet dass das Polypeptid fähig ist eine Immunantwort gegegn verschiedene Isolate von P. falciparum hervorzurufen.

Die Erfindung betrifft ferner immunogene Kompositionen und Vakzine, die ein Polypeptid, wie es oben definiert ist, enthalten, DNA Fragmente, die für diese Polypeptide kodieren, replizierbare, mikrobielle Vektoren, die eine solche DNA Sequenz enthalten und Mikroorganismen, die mit solchen Vektoren

transformiert sind. Zudem betrifft die Erfindung Verfahren zur Herstellung der oben genannten Polypeptide und Mikroorganismen und von Antikörpern, die gegen die obengenannten Antikörper gerichtet sind, sowie die Verwendung der Polypeptide zur Herstellung einer immunogenen Komposition zur Immunisierung von Säugern gegen Malaria.

Malaria wird beim Menschen durch vier Spezies von Plasmodium verursacht, nämlich durch P. falciparum, P. vivax, P. ovale und P. malariae. Gemäss einem Bericht der Weltgesundheitsorganisation (WHO) aus dem Jahre 1986, gibt es weltweit fast 100 Millionen Fälle von Malariainfektionen. Davon haben etwa 1 Million, meistens Fälle von Kleinkindern, die mit P. falciparum infiziert sind, tödlichen Ausgang. Wegen dem Auftauchen von drogenresistenten Parasiten und von Insektizidresistenz vermittelnden Moskitovektoren, breitet sich die Malaria weiter aus. So berichteten die Gesundheitsbehörden Indiens 1962 von 100'000 Fällen von Malaria und 1980 schon von 3 Millionen Fällen, meistens verursacht durch P. vivax (vergl. Bruce-Chwatt, Essential Malariology, 2. Auflage, Heinemann, London [1985]).

Neuere technische Entwicklungen haben die Hoffnung genährt, dass es bald möglich sein wird, Antimalaria-Vakzine herzustellen, die der wachsenden Ausbreitung der Malaria entgegen wirken. Erstens können neue Methoden bei der Entwicklung von Malariavakzinen angewendet werden, so z.B. die Klonierung von Genen, die Verwendung von monoklonalen Antikörpern zur Antigenidentifizierung sowie die Immunisierung mit synthetischen Peptiden. Zweitens liefern Langzeitkulturen von P. falciparum in menschlichen roten Blutzellen (Trager et al., Science 193, 673-675 [1976] auf bequeme Art und Weise Material zum Studium des Parasiten. In neuerer Zeit wurde es möglich, alle Stadien im Lebenszyklus des Parasiten im Labor zu züchten (Ponnudurai et al., Trans. R. Soc. Trop. Med. Hyg. 76, 812-818 [1982]; Mazier et al., Science 227, 440-442 [1985]).

Einen Teil seines Lebenszylkus verbringt P. falciparum in menschlichen roten Blutzellen. Ein Merozoit dringt in die Wirtzelle ein, vergrössert sich und durchläuft später mehrere Kernteilungen, um dann einen Schizonten zu bilden. Die Reifung des Schizonten ergibt eine Vielzahl von Merozoiten, die in den Blutstrom abgegeben werden und nach einer kurzen Zeit wieder in neue Erythrocyten eindringen.

Auf der Oberfläche der Merozoiten und Schizonten wurde ein Protein entdeckt, das als Vakzin gegen Malaria aktiv sein könnte. Wenn es synthetisiert wird, hat das Protein ein scheinbares Molekulargewicht von 190'000-200'000 D (Perrin et al., Clin. exp. Immunol. 41, 91-96 [1980]; Holder et al., J. Exp. Med. 156, 1528-1538 [1982]; Hall et al., Mol. Biochem. Parasitol. 7, 247-265 [1983] and Mol. Biochem. Parasitol. 11, 61-80 [1984]). Es wurde GP185, p190, 195-kD Protein oder polymorphes Schizontantigen (PSA) genannt. Zusätzlich sei bemerkt, dass ein grosser Teil des Proteins verlorengeht, wenn Merozoiten in neue Blutzellen eindringen (Holder et al., supra; Hall et al., supra [1983]).

Analoga des p190 sind in allen bekannten Spezies von Plasmodium vorhanden. In allen getesteten Fällen konnten Antikörper, die gegen das p190 gerichtet sind, das Eindringen der Parasiten in vitro verhindern (Epstein et al., J. Immunol. 127, 212-217 [1981]: Perrin et al., J. Exp. Med. 160, 441-451 [1984]; Boyle et al., Infect. Immun. 38, 94-102 [1982]). Injektion des gereinigten p190 Proteins in Saimiri Affen, führte zu einem mindestens teilweisen Schutz gegen Malaria (Hall et al., supra [1984]; Perrin et al., supra [1984]). Von drei verschiedenen Parasitenisolaten wurde das Gen. das das p190 Protein kodiert, isoliert und sequenziert, nämlich vom thailändischen Isolat K1 (Mackay et al., EMBO J. 4, 3823-3829 [1985]), vom malaysischen Isolat CAMP (Weber et al., Nucleic Acids Res. 14, 3311-3323 [1986]) und vom westafrikanischen Isolat Wellcome (Holder et al., Nature 317, 270-273 [1985]). Sequenzvergleiche zwischen diesen Allelen haben gezeigt, dass ein gewisser Grad an Polymorphismus zwischen den verschiedenen Isolaten besteht. Die Tripeptid-Wiederholungen, die im Bereich des Aminoterminus jedes Allels gefunden werden, sind verschieden in jedem der studierten Fälle.

Für die Entwicklung von Vakzinen ist es nötig, Epitope auf dem p190 Polypeptid zu finden, die in den meisten oder vorzugsweise in allen Isolaten vorhanden sind, damit ein Schutz gegen eine grosse Anzahl von Parasitenvarianten erhalten werden kann.

Die erfindungsgemässen Polypeptide enthalten eine Aminosäuresequenz, die vom 190 kD Vorläufer der vorwiegenden Merozoitenoberflächenantigene von P. falciparum hergeleitet ist, und die mindestens ein Epitop, das in verschiedenen Isolaten, z.B. dem K1, CAMP und dem Wellcome-Isolat vorhanden ist, darstellt.

Die Polypeptide können einen Affinitätspeptidrest enthalten. Solche Affinitätspeptidreste enthalten eine Aminosäuresequenz welche selektiv an ein Affinitätschromatographierharz binden. Bevorzugte Affinitätspolypeptidreste enthalten zwei oder mehrere benachbarte Histinreste. Besonders bevorzugt sind die Affinitätspeptidreste MetHisHisAlaProGlySerGly, MetHisHisAlaProGlySer und MetHisHisAlaPro. Polypeptide, die ein Affinitätspeptidrest mit zwei oder mehreren benachbarten Histinresten enthalten, binden selektiv an Nitrilotriessigsäure-Nickelchelatharze und können deshalb durch Affinitätschromatographie von Proteinen, die nicht zwei oder mehrere benachbarte Histine enthalten, abgetrennt werden.

Bevorzugte Polypeptide der vorliegenden Erfindung sind:

MetHisHisAlaProGlySerGlyThrLeuCysAspAsnIleHisGlyPheLysTyrLeu
IleAspGlyTyrGluGluIleAsnGluLeuLeuTyrLysLeuAsnPheTyrPheAspLeu
LeuArgAlaLysLeuAsnAsnValCysAlaAsnAspTyrCysGlnIleProPheAsnLeu
LysIleArgAlaAsnGluLeuAspValLeuLysLysLeuValPheGlyTyrArgLysPro
LeuAspAsnIleLysAspAsnValGlyLysMetGluAspTyrIleLysLysAsnLysLys
ThrIleGluAsnIleAsnGluLeuIleGluGluSerLysLysThrIleAspLysAsnLys
AsnAlaThrLysGluGluGluLysLysLysLeuTyrGlnAlaGlnTyrAspLeuPheIle
TyrAsnLysGlnLeuGluGluAlaHisAsnLeuIleSerValLeuGluLysArgIleAsp
ThrLeuLysLysAsnGluAsnIleLysGluLeuLeuAspLysIleAsnGluIleLysAsn
ProProProAlaGlyGlyLeuLeuLeuIleAspProValMetThrSerGluLeuHisLeu

AspLeuPheArgThrLeuGlyCysArgArgAlaPhePheIleGlyGluAsnProSer (p190-1);

MetHisHisAlaProGlySerAlaGluIleAlaGluThrGluAsnThrLeuGluAsnThr LysIleLeuLeuLysHisTyrLysGlyLeuValLysTyrTyrAsnGlyGluSerSerPro LeuLysThrLeuSerGluGluSerIleGlnThrGluAspAsnTyrAlaSerLeuGluAsn PheLysValLeuSerLysLeuGluGlyLysLeuLysAspAsnLeuAsnLeuGluLysLys LysLeuSerTyrLeuSerArgGlyLeuHisHisLeuIleAlaGluLeuLysGluValIle LysAsnLysAsnTyrThrGlyAsnSerProSerValAsnAsnThrAspValAsnAsnAla LeuGluSerTyrLysLysPheLeuProGluGlyThrAspValAlaThrValValSerGlu SerGlySerValAspLeuGlnProSerLeuAspSerCys (p190-2a);

MetHisHisAlaProAlaGluIleAlaGluThrGluAsnThrLeuGluAsnThrLysIle LeuLeuLysHisTyrLysGlyLeuValLysTyrTyrAsnGlyGluSerSerProLeuLys ThrLeuSerGluGluSerIleGlnThrGluAspAsnTyrAlaSerLeuGluAsnPheLys ValLeuSerLysLeuGluGlyLysLeuLysAspAsnLeuAsnLeuGluLysLysLysLeu SerTyrLeuSerArgGlyLeuHisHisLeuIleAlaGluLeuLysGluValIleLysAsn LysAsnTyrThrGlyAsnSerProSerValAsnAsnThrAspValAsnAsnAlaLeuGlu SerTyrLysLysPheLeuProGluGlyThrAspValAlaThrValValSerGluSerGly SerValAspLeuGlnProSerLeuAspSerCys (p190-2b);

MetHisHisAlaProGlySerAlaGluIleAlaGluThrGluAsnThrLeuGluAsnThr LysIleLeuLeuLysHisTyrLysGlyLeuValLysTyrTyrAsnGlyGluSerSerPro LeuLysThrLeuSerGluGluSerIleGlnThrGluAspAsnTyrAlaSerLeuGluAsn PheLysValLeuSerLysLeuGluGlyLysLeuLysAspAsnLeuAsnLeuGluLysLys LysLeuSerTyrLeuSerArgGlyLeuHisHisLeuIleAlaGluLeuLysGluValIle LysAsnLysAsnTyrThrGlyAsnSerProSerValAsnAsnThrAspValAsnAsnAla LeuGluSerTyrLysLysPheLeuProGluGlyThrAspValAlaThrValValSerGlu SerGlySerGlyThrLeuCysAspAsnIleHisGlyPheLysTyrLeuIleAspGlyTyr GluGluIleAsnGluLeuLeuTyrLysLeuAsnPheTyrPheAspLeuLeuArgAlaLys LeuAsnAsnValCysAlaAsnAspTyrCysGlnIleProPheAsnLeuLysIleArgAla AsnGluLeuAspValLeuLysLysLeuValPheGlyTyrArgLysProLeuAspAsnIle LysAspAsnValGlyLysMetGluAspTyrIleLysLysAsnLysLysThrIleGluAsn IleAsnGluLeuIleGluGluSerLysLysThrIleAspLysAsnLysAsnAlaThrLys GluGluGluLysLysLysLeuTyrGlnAlaGlnTyrAspLeuPheIleTyrAsnLysGln LeuGluGluAlaHisAsnLeuIleSerValLeuGluLysArgIleAspThrLeuLysLys AsnGluAsnIleLysGluLeuLeuAspLysIleAsnGluIleLysAsnProProProAla

GlyGlyLeuLeuLeuIleAspProValMetThrSerGluLeuHisLeuAspLeuPheArg ThrLeuGlyCysArgArgAlaPhePheIleGlyGluAsnProSer (p190-3).

Die Erfindung betrifft auch Homologe dieser Polypeptide und Polypeptide, die von den obigen Polypeptiden durch Aminosäurensubstitutionen hergeleitet sind, vorausgesetzt dass diese Polypeptide immer noch fähig sind, in einem Wirt eine Immunantwort, gegen verschiedene Isolate von P. falciparum oder mindestens gegen das K1, das CAMP, das MAD-20 und das Wellcome Isolat von P. falciparum, hervorzurufen. Solche Polypeptide können für die Immunisierung von Säugern gegen Malaria verwendet werden.

Gewisse Substitutionen in der Aminosäuresequenz eines Polypeptids haben keinen Einfluss auf dessen biologische Aktivität. Beispiele solcher Aminosäuresubstitutionen sind beschrieben, z.B. von Doolittle in "The Proteins", Neurath, H. und Hill, R.L., Eds., Academic Press, New York [1979]. Die häufigsten Aminosäuresubstitutionen sind: Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu und vice versa.

In der obenerwähnten allgemeinen Formel A-B-C entspricht nur Sequenz B einem Teil des 190 kD Proteins von P. falciparum. A und C können entweder fehlen oder eine Aminosäuresequenz darstellen, wie sie oben definiert ist. Zuden können sie aus jeglicher Aminosäuresequenz aufgebaut sein, sofern dies keine Nachteile bei der Verwendung der Polypeptide als Vakzine für die Immunisierung von Säugern gegen Malaria hat. So kann A eine Teilsequenz oder eine verlängerte Sequenz der Aminosäuresequenz MetHisHisAlaProGlySerGly sein, solange diese Sequenz mindestens zwei benachbarte Histidine enthält. Diese Sequenz A stellt ein Affinitätspeptid dar, das als Mittel zur Reinigung der erfindungsgemässen Polypeptide unter Verwendung der Harze und der Methoden wie sie in Beispiel 2, F und G beschrieben sind, verwendet werden kann.

Gleicherweise kann zusätzlich zu den bevorzugten Sequenzen für C, wie z.B.

AlaGlyGlyLeuLeuLeuIleAspProValMet-ThrSerGluLeuHisLeuAspLeuPheArgThrLeuGlyCysArgArgAlaPhePheIleGlyGluAsnProSer

oder ValAspLeuGlnProSerLeuAspSerCys, dieser Peptidrest auch aus einer Aminosäuresequenz zusammengesetzt sein, die nicht mit der Verwendung der erfindungsgemässen Polypeptide interferiert. Die spezifischen Sequenzen von C, wie sie oben definiert sind, sind vom Expressionsvektor hergeleitet, in den die P. falciparum-spezifische Sequenz B hineinkloniert worden war. A und C sind deshalb nur aus technischen Gründen vorhanden und können innerhalb grosser Bereiche variieren.

Die erfindungsgemässen Polypeptide können kovalent an ein Trägermaterial gebunden oder daran adsorbiert sein. Geeignete Trägermaterialien sind natürliche oder synthetische Polymerverbindungen, wie z.B. Copolymere einer oder mehrerer Aminosäuren (z.B. Polylysin) oder Zucker (z.B. Polysaccharid). Weitere geeignete Trägermaterialien sind natürliche oder synthetische Polypeptide wie Hämocyanine (z.B. KLH = keyhole limpet hemocyanin), Serumproteine (z.B. Gammaglobuline, Serumalbumine) und Toxoide (z.B. Diphtherie- und Tetanustoxoid). Weitere geeignete Trägermaterialien sind dem Fachmann bekannt.

Die kovalente Bindung der erfindungsgemässen Polypeptide an die Trägermaterialien kann auf bekannte Art und Weise erfolgen, z.B. direkt durch die Bildung einer Peptid- oder Esterbindung zwischen freien Carboxyl-, Amino- oder Hydroxylgruppen der Polypeptide und den entsprechenden Gruppen auf dem Trägermaterial, oder indirekt durch Verwendung eines konventionellen, bifunktionellen Reagenzes, wie z.B. eines Carbodiimids, vorzugsweise durch Verwendung von 1,3-Dicyclohexylcarbodiimid oder 1-Ethyl-3-(3-dimethylaminopropyl)carbodiimid, eines $C_{2-7}$-Dialkanals wie z.B. Glutaraldehyd (Avrameas, Immunochem. 6, 43-52 [1969] oder von m-Maleimidobenzoyl-N-hydroxysuccinimidester (MBS).

Das Trägermaterial mit den daran gebundenen Polypeptiden kann mittels bekannter Methoden (z.B. Dialyse oder Säulenchromatographie) von nichtgebundenen Polypeptiden und gegebenenfalls von überschüssigen Kupplungs-Reagentien gereinigt werden.

Die Peptide der vorliegenden Erfindung können mittels konventioneller Methoden der Peptidsynthese, in flüssiger oder, vorzugsweise an fester Phase, wie der Methode von Merrifield (J. Am. Chem. Soc. 85, 2149-2154 [1963], oder mittels anderer gleichwertiger Methoden des Standes der Technik, hergestellt werden.

Die Festphasensynthese beginnt mit der C-terminalen Aminosäure des zu synthetisierenden Peptids, die in geschützter Form an ein passendes Trägermaterial gekoppelt wird. Das Ausgangsmaterial kann durch Bindung einer aminogruppengeschützten Aminosäure über eine Benzylesterbrücke an chlormethyliertes oder hydroxymethyliertes Trägermaterial oder durch Amidbindung an ein Benzhydrylamin (BHA)- oder Methylbenzhydrylamin (MBHA)-Trägermaterial hergestellt werden. Diese Trägermaterialien sind käuflich

erhältlich und ihre Bereitstellung und Verwendung sind wohlbekannt.

Allgemeine Methoden zum Schützen und Entfernen der Schutzgruppen von Aminosäuren, die in dieser Erfindung verwendet werden können, sind beschrieben in "The Peptides", Vol. 2 (herausgegeben von E. Gross und J. Meienhofer, Academic Press, New York, 1-284 [1979]. Geeignete Schutzgruppen umfassen z.B. die tertiäre Butyloxycarbonyl (Boc)-, die Benzyl (Bzl)-, die 2-Chlorbenzyloxycarbonyl (2Cl-Z)-, die Dichlorbenzyl (Dcb)- und die 3,4-Dimethylbenzyl-(Dmb)-gruppe.

Nach Entfernen der α-Aminoschutzgruppe der an den Träger gebundenen C-terminalen Aminosäure werden die geschützten Aminosäuren in der gewünschten Reihenfolge schrittweise angekoppelt. Das vollständige Peptid kann so synthetisiert werden. Als Alternative dazu können kleine Peptide aufgebaut werden, die dann zum gewünschten Peptid zusammengefügt werden. Geeignete Kopplungsreagenzien gehören zum Stand der Technik, wobei Dicyclohexylcarbodiimid (DCC) besonders geeignet ist.

Jede(s) geschützte Aminosäure oder Peptid wird im Ueberschuss in das Festphasensynthesereaktionsgefäss gegeben und die Kopplungsreaktion kann in Dimethylformamid (DMF) oder Methylenchlorid ($CH_2Cl_2$) oder einer Mischung aus beiden durchgeführt werden. In Fällen von unvollständiger Kopplung wird die Kopplungsreaktion wiederholt bevor die N-terminale α-Aminoschutzgruppe zwecks Kopplung der nächsten Aminosäure entfernt wird. Die Ausbeute jedes Kopplungsschrittes kann verfolgt werden und zwar bevorzugt nach der Ninhydrinmethode. Die Kopplungsreaktionen und die Waschschritte können automatisiert werden.

Die Abspaltung des Peptids vom Trägermaterial kann durch in der Peptidchemie wohlbekannte Methoden erreicht werden, z.B. durch Umsetzung mit Fluorwasserstoff (HF) in Gegenwart von p-Kresol und Dimethylsulfid während 1 Stunde bei 0 °C, gefolgt möglicherweise von einer zweiten Umsetzung mit HF in Gegenwart von p-Kresol während 2 Stunden bei 0 °C. Die Abspaltung der Peptide von chlormethylierten oder hydroxymethylierten Trägermaterialien ergibt Peptide mit freiem C-Terminus; die Abspaltung der Peptide von Benzylhydrylamin- oder Methylbenzylhydrylamin-Trägern ergibt Peptide mit amidiertem C-Terminus.

Andererseits können die Polypeptide der vorliegenden Erfindung auch unter Verwendung der Methoden der DNA-Rekombinationstechnik (Manniatis et al. in "Molecular Cloning - A Laboratory Manual". Cold Spring Harbor Laboratory [1982]) hergestellt werden. So kann z.B. ein DNA-Fragment, das für ein solches Polypeptid codiert, mittels konventioneller chemischer Methoden synthetisiert werden, z.B. mittels der Phosphotriestermethode wie sie von Narang et al. in Meth. Enzymol. 68, 90-108 [1979] beschrieben ist oder mittels der Phosphodiestermethode (Brown et al., Meth. Enzymol. 68, 109-151 [1979]). Bei beiden Methoden werden zuerst längere Oligonukleotide synthetisiert, die in vorgegebener Art und Weise aneinander gehängt werden und so das gewünschte DNA-Fragment ergeben. Die Nukleotidsequenz des DNA-Fragmentes kann identisch sein zu derjenigen Nukleotidsequenz, die das natürliche Polypeptid im Plasmodium Parasiten codiert oder kann verschieden davon sein. Da der genetische Code degeneriert ist, besteht andererseits die Möglichkeit, dass eine teilweise oder vollständig unterschiedliche Nukleotidsequenz das gleiche Polypeptid codiert. Gegebenenfalls können für die Nukleotidsequenz solche Codons gewählt werden, die auch vom Wirtsorganismus, der zur Expression des Polypeptids verwendet wird, bevorzugt verwendet werden (Grosjean et al., Gene 18, 199-209 [1982]. Dabei muss aber darauf geachtet werden, dass das so erhaltene DNA-Fragment keine Teilsequenzen enthält, die die Konstruktion des Expressionsvektors, z.B. durch Einführung einer unerwünschten Restriktionsenzymschnittstelle, erschweren, oder die die Expression des DNA-Fragmentes negativ beeinflussen.

In einem anderen Verfahren, das in der DNA-Rekombinations-Technologie verwendet wird, kann das DNA-Fragment, das für ein erfindungsgemässes Polpeptid kodiert, aus der genomischen DNA eines Isolates von P. falciparum isoliert werden. Dies kann durch teilweises Verdauen der genomischen DNA mit einer geeigneten Restriktionsendonuklease, z.B. mit EcoR1, gemacht werden. Die verdaute DNA kann auf einem 0.7%-igen Agarosegel aufgetrennt und Fragmente mit einer Grösse von 1,5 bis 8 x $10^3$ Basenpaaren können isoliert werden. Die isolierten Fragmente können in geeignete Vektoren, z.B. in den Lambdavektor gtII (ATCC-No. 37194, auch erhältlich als Protoclone GT® Lambda GTII von Genofit SA, Genève) gemäss den Angaben des Herstellers kloniert werden. Die rekombinate Phagen-DNA kann in vitro gepackt werden (Gigapack®, Vector Cloning Systems, San Diego) und der resultierende Phage kann in einen geeigneten Wirt, z.B. in E.coli Y1088 (ATCC-No. 37195), transfiziert werden. Etwa 100'000 rekombinante Phagen können dann durch Hybridisierung mit radioaktiv markierten, komplementären Oligonukleotidproben auf die Anwesenheit von P. falciparum-spezifischer DNA überprüft werden. Als Proben können Teilsequenzen aus den bekannten Nukleotidsequenzen verschiedener Isolate von P. falciparum dienen. Komplementäre Oligonukleotidproben können wie oben beschrieben chemisch synthetisiert werden. Beispiele für solche Oligonukleotidproben sind das Oligonukleotid 1 (35-mer) mit der Sequenz

TTTTATTTTGATTTATTAAGAGCAAAATTAAATAA

und das Oligonukleotid 2 (33-mer) mit der Sequenz

GAAAACACAAAAATATTATTGAAACATTATAAA

die von der Nukleotidsequenz des K1 Isolates von P. falciparum (Mackay et al., supra) abgeleitet sind.

Rekombinante Phagen, die selektiv mit einer gegebenen Oligonukleotidprobe hybridisieren, können in bekannter Weise isoliert werden. Die Phagen können hochgewachsen werden und die DNA kann isoliert werden. Das P. falciparum-Fragment kann isoliert werden und in einen geeigneten Vektor, z.B. das Plasmid pUC19 (Pharmacia) subkloniert werden. Das erhaltene rekombinante Plasmid kann weiter subkloniert werden und ergibt das DNA Fragment, das für den Teil B des erfindungsgemässen Polypeptids kodiert.

In einem solchen Subklonierungsverfahren wird das DNA Fragment, das gemäss einer der beiden oben beschriebenen Methoden hergestellt worden ist, in einen replizierbaren mikrobiellen Vektor eingebaut. Die vorliegende Erfindung betrifft auch solche replizierbare, mikrobielle Vektoren, die ein DNA Fragment, wie es oben beschrieben ist, enthalten. Solche Vektoren können z.B. als DNA Proben in diagnostischen Tests verwendet werden. Die Genprobentechnologie ist in einem Uebersichtsartikel von Klausner et al. in Bio/Technology, August 1983, Seiten 471-478 beschrieben. Durch die Anwendung dieser Technologie kann ein Fachmann ohne weiteres DNA-Proben, die auf einem DNA-Fragment, wie es oben beschrieben ist, basieren, herstellen und einen Vektor, der als Klonierungsvektor in der vorliegenden Erfindung geeignet ist, auswählen.

Für die Herstellung der erfindungsgemässen Polypeptide in einem Wirtsorganismus muss das DNA-Fragment mit einer Nukleotidsequenz, die von einem der Isolate von P. falciparum hergeleitet ist, ein Startkodon (z.B. ATG) und ein Terminationskodon (TAA, TAG oder TGA) enthalten. Falls diese Signale nicht auf dem ursprünglichen DNA-Fragment vorhanden sind, können sie in das oben genannte DNA-Fragment gemäss Methoden, die dem Fachmann bekannt sind, eingefügt werden. Bei einer dieser Methoden wird das DNA-Fragment in einen Vektor eingebaut, der diese Signale in einer solchen Weise besitzt, dass das Start- und das Terminationskodon in Serie ist mit der Sequenz, die für die Untersequenz B des Polypeptids der Erfindung kodiert. Ein oder mehrere Nukleotidtripletts können zwischen dem Startkodon und der Sequenz, die für die Subsequenz B des Polypeptids kodiert, vorhanden sein. Diese Nukleotide bilden zusammen mit dem Startkodon die Teilsequenz A des erfindungsgemässen Polypeptids. Gleicherweise können ein oder mehrere Nukleotidtripletts zwischen der Sequenz, die für die Teilsequenz B und das Terminationskodon kodiert, vorhanden sein. Diese Nukleotide kodieren dann für die Teilsequenz C des erfindungsgemässen Polypeptids. Ein DNA-Fragment, das für ein erfindungsgemässes Polypeptid der Formel A-B-C kodiert, kann gemäss dem Fachmann bekannter Methoden erhalten werden. Die vorliegende Erfindung betrifft auch solche DNA-Sequenzen. Bevorzugte DNA-Sequenzen, die für die Polypeptide p190-a, p190-2a, p190-2b oder p190-3 kodieren sind:

ATG CAT CAC GCC CCC GGA TCC GGA ACT TTG TGT GAT AAT ATT CAT GGT TTC AAA TAT TTA ATT GAT GGA TAT GAA GAA ATT AAT GAA TTA TTA TAT AAA TTA AAC TTT TAT TTT GAT TTA TTA AGA GCA AAA TTA AAT AAT GTA TGT GCT AAT GAT TAT TGT CAA ATA CCT TTC AAT CTT AAA ATT CGT GCA AAT GAA TTA GAC GTA CTT AAA AAA CTT GTG TTC GGA TAT AGA AAA CCA TTA GAC AAT ATT AAA GAT AAT GTA GGA AAA ATG GAA GAT TAC ATT AAA AAA AAT AAA AAA ACC ATA GAA AAT ATA AAT GAA TTA ATT GAA GAA AGT AAG AAA ACA ATT GAT AAA AAT AAG AAT GCA ACT AAA GAA GAA GAA AAA AAA AAA TTA TAC CAA GCT CAA TAT GAT CTT TTT ATT TAC AAT AAA CAA TTA GAA GAA GCA CAT AAT TTA ATA AGC GTT TTA GAA AAA CGT ATT GAC ACT TTA AAA AAA AAT GAA AAC ATT AAG GAA TTA CTT GAT AAG ATA AAT GAA ATT AAA AAT CCC CCA CCG GCC GGT GGA CTC CTG TTG ATA GAT CCA GTA ATG ACC TCA GAA CTC CAT CTG GAT TTG TTC AGA ACG CTC GGT TGC CGC CGG GCG TTT TTT ATT GGT GAG AAT CCA AGC TAG;

ATG CAT CAC GCC CCC GGA TCC GCT GAA ATA GCA GAA ACT GAA AAC ACA TTA GAA AAC ACA AAA ATA TTA TTG AAA CAT TAT AAA GGA CTT GTT AAA TAT TAT AAT GGT GAA TCA TCT CCA TTA AAA ACT TTA AGT GAA GAA TCA ATT CAA ACA GAA GAT AAT TAT GCC AGT TTA GAA AAC TTT AAA GTA TTA AGT AAA TTA GAA GGA AAA TTA AAG GAT AAT TTA AAT TTA GAA AAG AAA AAA TTA TCA TAC TTA TCA AGA GGT TTA CAT CAT TTA ATT GCT GAA TTA AAA GAA GTA ATA AAA AAT AAA AAT TAT ACA GGT AAT TCT CCA AGC GTA AAT AAT ACG GAT GTT AAC AAT GCA TTA GAA TCT TAC AAA AAA TTT CTC CCA GAA GGA ACA GAT GTT GCA ACA GTT GTA AGT GAA AGT GGA TCC GTC GAC CTG CAG CCA AGC TTG GAC TCC TGT TGA;

ATG CAT CAC GCC CCC GCT GAA ATA GCA GAA ACT GAA AAC ACA TTA
GAA AAC ACA AAA ATA TTA TTG AAA CAT TAT AAA GGA CTT GTT AAA
TAT TAT AAT GGT GAA TCA TCT CCA TTA AAA ACT TTA AGT GAA GAA
TCA ATT CAA ACA GAA GAT AAT TAT GCC AGT TTA GAA AAC TTT AAA
GTA TTA AGT AAA TTA GAA GGA AAA TTA AAG GAT AAT TTA AAT TTA
GAA AAG AAA AAA TTA TCA TAC TTA TCA AGA GGT TTA CAT CAT TTA
ATT GCT GAA TTA AAA GAA GTA ATA AAA AAT AAA AAT TAT ACA GGT
AAT TCT CCA AGC GTA AAT AAT ACG GAT GTT AAC AAT GCA TTA GAA
TCT TAC AAA AAA TTT CTC CCA GAA GGA ACA GAT GTT GCA ACA GTT
GTA AGT GAA AGT GGA TCC GTC GAC CTG CAG CCA AGC TTG GAC TCC
TGT TGA;

ATG CAT CAC GCC CCC GGA TCC GCT GAA ATA GCA GAA ACT GAA AAC
ACA TTA GAA AAC ACA AAA ATA TTA TTG AAA CAT TAT AAA GGA CTT
GTT AAA TAT TAT AAT GGT GAA TCA TCT CCA TTA AAA ACT TTA AGT
GAA GAA TCA ATT CAA ACA GAA GAT AAT TAT GCC AGT TTA GAA AAC
TTT AAA GTA TTA AGT AAA TTA GAA GGA AAA TTA AAG GAT AAT TTA
AAT TTA GAA AAG AAA AAA TTA TCA TAC TTA TCA AGA GGT TTA CAT
CAT TTA ATT GCT GAA TTA AAA GAA GTA ATA AAA AAT AAA AAT TAT
ACA GGT AAT TCT CCA AGC GTA AAT AAT ACG GAT GTT AAC AAT GCA
TTA GAA TCT TAC AAA AAA TTT CTC CCA GAA GGA ACA GAT GTT GCA
ACA GTT GTA AGT GAA AGT GGA TCC GGA ACT TTG TGT GAT AAT ATT
CAT GGT TTC AAA TAT TTA ATT GAT GGA TAT GAA GAA ATT AAT GAA
TTA TTA TAT AAA TTA AAC TTT TAT TTT GAT TTA TTA AGA GCA AAA
TTA AAT AAT GTA TGT GCT AAT GAT TAT TGT CAA ATA CCT TTC AAT
CTT AAA ATT CGT GCA AAT GAA TTA GAC GTA CTT AAA AAA CTT GTG
TTC GGA TAT AGA AAA CCA TTA GAC AAT ATT AAA GAT AAT GTA GGA
AAA ATG GAA GAT TAC ATT AAA AAA AAT AAA AAA ACC ATA GAA AAT
ATA AAT GAA TTA ATT GAA GAA AGT AAG AAA ACA ATT GAT AAA AAT
AAG AAT GCA ACT AAA GAA GAA GAA AAA AAA AAA TTA TAC CAA GCT
CAA TAT GAT CTT TTT ATT TAC AAT AAA CAA TTA GAA GAA GCA CAT
AAT TTA ATA AGC GTT TTA GAA AAA CGT ATT GAC ACT TTA AAA AAA
AAT GAA AAC ATT AAG GAA TTA CTT GAT AAG ATA AAT GAA ATT AAA
AAT CCC CCA CCG GCC GGT GGA CTC CTG TTG ATA GAT CCA GTA ATG
ACC TCA GAA CTC CAT CTG GAT TTG TTC AGA ACG CTC GGT TGC CGC
CGG GCG TTT TTT ATT GGT GAG AAT CCA AGC TAG.

Um die Polypeptide in einem Wirtsorganismus zu exprimieren, muss die DNA-Sequenz, die für dieses Polypeptid kodiert, operativ verbunden sein mit einer Expressionskontrollsequenz. Geeignete Expressionskontrollsequenzen sind dem Fachmann bekannt (Europäische Patentanmeldung, Publikations-Nr. 186 069, publiziert am 2. Juli 1986). Der Fachmann kann ohne weiteres von diesen Expressionskontrollsequenzen diejenigen aussuchen, die für die Expression der erfindungsgemässen Polypeptide am geeignetsten sind.

Die bevorzugte Expressionskontrollsequenz ist diejenige, die in pDS6/RBSII,SphI-His,His enthalten ist. Dieser Expressionsvektor ist hergeleitet von den Expressionsvektoren pDS6/RBSII,3A+5A und pDS8/RBSII,SphI. Der Expressionsvektor pDS6/RBSII,3A+5A wurde am 3. Oktober 1985 bei der Deutschen Sammlung von Mikroorganismen (DSM) in Form einer Probe von E.coli M15(pDS6/RBSII,3A+5A;pDMI,1) in Uebereinstimmung mit den Budapester Vertrag hinterlegt und hat die Hinterlegungsnummer DSM 3518. Der Expressionsvektor pDS8/RBSII,SphI wurde am 6. August 1986 bei der Deutschen Sammlung von Mikroorganismen (DSM) in Form einer Probe von E.coli M15(pDS8/RBSII,SphI;pDMI,1) ebenfalls in Uebereinstimmung mit dem Budapester Vertrag hinterlegt und hat die Hinterlegungsnummer DSM 3809. Die Konstruktion des Expressionsvektors pDS6/RBSII,SphI-His,His ist in Beispiel 1 erklärt. Nach dem Einbau des DNA-Fragments mit einer Nukleotidsequenz, die von einem der Isolate von P. falciparum hergeleitet ist, in den oben genannten Expressionsvektor, entsteht ein neuer replizierbarer mikrobieller Vektor, der fähig ist, ein erfindungsgemässes Polypeptid zu exprimieren. Die Konstruktion solcher Vektoren ist in den Beispielen 2, 3 und 4 beschrieben. Die erhaltenen Vektoren pGC1, pGC2a, pGC2b und pGC3 sind die bevorzugten erfindungsgemässen Vektoren.

Der Expressionvektor, der eine DNA-Sequenz enthält, die für ein erfindungsgemässes Polypeptid kodiert, kann, unter Verwendung von dem Fachmann bekannten Methoden, in einen geeigneten Wirt, der fähig ist, die genannten Polypeptide zu exprimieren, eingebaut werden. Die so erhaltenen Transformanten können unter Bedingungen kultiviert werden, die die Herstellung von grossen Mengen von Polypeptid erlauben. Die rekombinanten Polypeptide können anschliessend mit bekannten Methoden isoliert und gereinigt werden. Die vorliegende Erfindung betrifft auch solche Transformanten.

Die Auswahl eines bestimmten Wirtsorganismus zur Verwendung in der vorliegenden Erfindung hängt von einer Anzahl Faktoren ab, die dem Fachmann bekannt sind. Diese umfassen zum Beispiel die Kompatibilität mit einem ausgewählten Expressionsvektor, die toxischen Auswirkungen der rekombinanten Polypeptide auf den Wirt, die Leichtigkeit, mit der die gewünschten Polypeptide isoliert werden können, die charakteristischen Merkmale der Expression, die biologische Sicherheit und die Produktionskosten. Innerhalb dieser generellen Rahmenbedingungen sind gram-negative und gram-positive Bakterien, insbesondere Stämme von E.coli und B.subtilis, als Wirtsorganismen geeignet. Bevorzugter Wirtsorganismus ist E.coli M15 (von Villarejo et al. in J. Bacteriol. 120, 466-474 [1974] als DZ291 beschrieben). Weitere geeignete Wirtsorganismen sind E.coli 294 (ATCC Nr. 31446), E.coli RR1 (ATCC Nr. 31343) und E.coli W3110 (ATCC Nr. 27325).

Sind die Transformanten, die zur Expression der erfindungsgemässen Polypeptide befähigt sind, hergestellt, kann das erfindungsgemässe Verfahren auf verschiedene Art und Weise durchgeführt werden und zwar in Abhängigkeit von der Art und Weise der Konstruktion des Expressionsvektors und von den Wachstumseigenschaften des Wirtsorganismus. Ueblicherweise werden die Transformanten unter Bedingungen wachsen gelassen, die für die Herstellung grosser Mengen von Zellen günstig sind. Wenn sich eine grosse Menge an Zellen angehäuft hat, werden durch geeignete Induktoren oder Derepressoren oder durch eine Temperaturänderung Kontrollsequenzen, die auf einer solchen DNA vorhanden sind aktiviert, was die Transkription und Translation der kodierenden Sequenz zur Folge hat. In der vorliegenden Erfindung ist die Expression des DNA-Fragmentes, das das erfindungsgemässe Polypeptid kodiert, durch den lac-Repressor gehemmt. Wenn sich eine grosse Menge an Zellen angehäuft hat, wird die Kontrollsequenz durch die Zugabe von Isopropyl-$\beta$-D-thiogalactopyranosid (IPTG) dereprimiert. Die von den rekombinanten Zellen hergestellten Polypeptide können durch Lyse der Zellen mittels üblicher Verfahren aus den Zellen gewonnen werden. Die Art und Weise wie die Zellen geöffnet werden, hängt von der verwendeten Wirtszelle ab. Wenn ein geeigneter Wirtsorganismus verwendet wird, können die erfindungsgemässen Polypeptide durch die Transformanten auch direkt ins Medium sezerniert werden.

Wenn die erfindungsgemässen Polypeptide in einem mikrobiellen Wirt hergestellt werden, stammt der Methioninrest am N-Terminus vom mRNA Translationsstartsignal AUG, das auf der DNA vom Startkodon ATG kodiert wird. Dieses AUG kodiert die Aminosäure Methionin. In gewissen Expressionssystemen wird dieser Methioninrest posttranslatorisch entfernt. Die An- oder Abwesenheit des N-terminalen Methionins hat keinen Einfluss auf die biologische Aktivität der meisten rekombinanten Polypeptide (Winnacker, in "Gene und Klone", Seite 255, Verlag Chemie (VCH), Weinheim [1985]).

Die erfindungsgemässen Polypeptide können mittels bekannter Methoden gereinigt werden, wie zum Beispiel mittels Zentrifugation bei unterschiedlichen Geschwindigkeiten, mittels Präzipitation mit Ammoniumsulfat, mittels Dialyse (bei Normaldruck oder bei reduziertem Druck), mittels präparativer Isoelektrofokussierung, mittels präparativer Gelelektrophorese oder mittels verschiedener chromatographischer Methoden wie Gelfiltration, Hochleistungs-Flüssigchromatographie (HPLC), Ionenaustauschchromatographie, Umkehrphasenchromatographie und Affinitätschromatographie (z.B. an Sepharose Blau CL-6B, an trägergebundenen, gegen das Polypeptid gerichteten monoklonalen Antikörpern oder an Metallchelatharzen wie sie in

der vorliegenden Erfindung beschrieben sind).

Eine bevorzugte Reinigungsmethode für die erfindungsgemässen Polypeptide ist die affinitätschromatographische Reinigung an Metallchelatharzen (Sulkowsky, Trends in Biotechn. 3, 1-7 [1985]). Dabei wird die selektive Bindung von benachbarten Histidinresten an Nitrilotriessigsäure-Nickelchelatharze (NTA-Harze) ausgenützt. Bevorzugte NTA-Harze bestehen aus den Nitrilotriessigsäurederivaten N-[3-Amino-1-carboxypropyl]iminodiessigsäure oder N-[5-Amino-1-carboxypentyl]iminodiessigsäure. Diese NTA-Derivate werden mittels eines bifunktionellen Reagenzes als Abstandhalter ("spacer") an ein Trägermaterial gebunden. In der vorliegenden Erfindung sind die NTA-Derivate bevorzugt über die Radikale $-O-CH_2-CH(OH)-CH_2-$ oder -O-CO-als Abstandhalter an die Trägermatrix gebunden. Bevorzugte Trägermaterialien sind vernetzte Dextrane, Agarose (z.B. Sepharose®) und Polyacrylamide. Die Herstellung des NTA-Harzes der Formel [Sepharose® CL-6B]$-O-CH_2-CH(OH)-CH_2-NH-(CH_2)_4-CH(COOH)-N(CH_2COO^-)_2Ni^{2+}$ ist in Beispiel 2,F beschrieben.

Die Polypeptide der vorliegenden Erfindung können in Form von Multimeren, z.B. in Form von Dimeren, Trimeren oder Tetrameren vorliegen, oder können auch Teil sein von Fusionspolypeptiden. Multimere können entstehen wenn Polypeptide in prokaryotischen Wirtsorganismen hergestellt werden, zum Beispiel durch die Bildung von Disulfidbrücken zwischen Cysteinresten (vergl. Fig. 9). Fusionspeptide können mittels rekombinanter DNA-Technologie hergestellt werden. Dabei werden DNA-Fragmente, die für ein erfindungsgemässes Polypeptid kodieren, mit einer weiteren DNA-Sequenz verknüpft. Diese weitere DNA-Sequenz kann aus einer grösseren Anzahl von DNA Sequenzen, die eine grosse Anzahl prokaryotischer oder eukaryotischer Polypeptide kodieren, ausgewählt werden. Nach der Expression der Fusionspolypeptide, die von den kombinierten DNA Sequenzen kodiert werden, in einem geeigneten Wirtsorganismus, können die Fusionspolypeptide, mittels Affinitätschromatographie unter Verwendung eines Liganden der spezifisch ist für das genannte prokaryotische oder eukaryotische Polypeptid, gereinigt werden.

Ein Beispiel für ein derartiges Fusionspolypeptid ist eines, das ein erfindungsgemässes Polypeptid sowie ein Polypeptid mit $\beta$-Galactosidaseaktivität enthält. Solche Fusionsproteine können, wie von Rüther et al., EMBO J., 2, 1791-1794 [1983], beschrieben, hergestellt und gereinigt werden.

Ein weiteres Beispiel für ein Fusionspolypeptid ist eines, das zwei oder mehrere der erfindungsgemässen Polypeptide enthält. Ein Beispiel eines solchen Polypeptids ist p190-3. Die Aminosäuresequenz dieses Polypeptids enthält die Untersequenzen B in der Aminosäuresequenz der Polypeptide p190-1 und p190-2a. Im Rahmen der Erfindung können die Aminosäuresequenzen des Fusionspolypeptids direkt über eine Peptidbindung oder eine andere kovalente Bindung oder über ein Peptidfragment mit einer oder mehreren Aminosäuren verbunden sein. Ein solches Peptidfragment kann allein aus technischen Gründen anlässlich der Konstruktion des Expressionsvektors, der das Fusionspolypeptid kodiert, eingebaut worden sein.

Die vorliegende Erfindung betrifft auch immunogene Kompositionen, die ein Polypeptid gemäss der vorliegenden Erfindung, sowie ein pharmazeutisch akzeptables Adjuvans, enthalten. Diese immunogenen Kompositionen sind fähig Antikörper, die gegen den p190-Vorläufer der vorwiegenden Merzozoiten Oberflächenantigene von P. falciparum gerichtet sind, zu induzieren. Da diese Antigene immunologisch reaktive Determinanten des Malariaparasiten darstellen, können die oben beschriebenen Polypeptide und immunogenen Kompositionen als Vakzine in Säugern verwendet werden, um diese gegen Malaria zu schützen. Der Ausdruck "pharmazeutisch akzeptables Adjuvans" kann entweder eine Standard-Zusammensetzung sein, die für die Behandlung von Menschen geeignet ist oder eines der typischen Adjuvantien, die bei der Tiervakzinierung verwendet werden können.

Adjuvantien die für die Vakzinierung von Tieren geeignet sind, sind neben andern Freund's komplettes oder inkomplettes Adjuvans (beide nicht für den Gebrauch beim Menschen und beim Vieh geeignet), Adjuvans 65 (enthaltend Erdnussöl, Mannit-monoolein und Aluminiummonostearat), Mineralgele, wie z.B. Aluminiumhydroxid, Aluminiumphosphat und Alaun, oberflächenaktive Substanzen, wie z.B. Hexadecylamin, Octadecylamin, Lysolecithin, Dimethyldioctadecylammoniumbromid, N,N-Dioctadecyl-N′,N′-bis(2-hydroxyethyl)propandiamin, Methoxyhexadecylglycerin und Pluronic Polyole, Polyanionen, wie z.B. Pyran, Dextransulfat, polyIC, Polyacrylsäure und Carbopol, Aminosäuren und Peptide, wie z.B. Muramyldipeptid, Dimethylglycin, Tuftsin, sowie Oel-Emulsionen. Die erfindungsgemässen Polypeptide können auch nach Einbau in Liposomen oder in andere Mikro-Trägermaterialien oder nach Kopplung an Polysaccharide, an andere Proteine oder an andere Polymere oder in Kombination mit Quil-A, wobei "Iscoms" (= immunostimulierende Komplexe, Morein et al., Nature 308, 457-460 [1984]) gebildet werden, verabreicht werden.

Die vorliegende Erfindung betrifft auch eine Methode zur Immunisierung von Säugern gegen Malaria, wobei die Säuger mit einer immunosierenden Menge der Polypeptide oder der immunogenen Kompositionen behandelt werden.

Die Art der Verabreichung, die Antigendosis, sowie die Häufigkeit der Injektionen, sind Faktoren, die vom Fachmann auf bekannte Art und Weise optimiert werden können. Typischerweise folgt auf die erste

Injektion nach einigen Wochen eine oder mehrere "booster"-Impfungen, die die Bildung von hohen Titern an Antikörpern, die gegen die Merozoitenform des Malariaparasiten gerichtet sind, zur Folge haben.

Die erfindungsgemässen Polypeptide und immunogenen Kompositionen können zur Bildung von Antikörpern gegen die vorwiegenden Merozoiten-Oberflächenantigene von P. falciparum, in einem Wirtsorganismus verwendet werden. Geeignete Wirtsorganismen für die Erzeugung von Antikörpern sind: Affen, Kaninchen, Pferde, Ziegen, Meerschweinchen, Ratten, Mäuse, Kühe, Schafe, etc. Das erzeugte Antiserum enthält Antikörper, die selektiv mit den obengenannten Oberflächenantigenen von P. falciparum reagieren oder daran binden. Das Antiserum kann entweder direkt verwendet werden oder die spezifischen Antikörper können mittels bekannter Methoden, z.B. mittels Ammoniumsulfatfällung, daraus isoliert werden. Das Antiserum oder die spezifischen Antikörper können auf bekannte Art und Weise für diagnostische Zwecke oder zu Reinigungszwecken (z.B. Affinitätschromatographie) verwendet werden.

Nachdem die vorliegende Erfindung allgemein beschrieben worden ist, sollen die nachfolgenden Beispiele zum besseren Verständnis der Erfindung beitragen, insbesondere bei Bezugnahme auf die folgenden Abbildungen, worin die folgenden Abkürzungen und Symbole verwendet werden:

B, E, H, P, S, Sa, Sc, X und Xb bezeichnen Schnittstellen für die Restriktionsenzyme BamHI, EcoRI, HindIII, PstI, SphI, SalI, ScaI, XhoI bzw. XbaI.

In Figuren 1, 3 und 5 repräsentiert

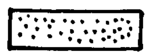

die Promotoren der Gene bla, lacI und neo;

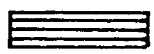

repräsentiert die ribosamalen Bindungsstellen der Gene bla, cat, neo und lacI;

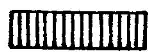

repräsentiert die Terminatoren $t_o$ und T1;

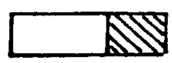

repräsentiert das regulierbare Promotor/Operator Element $P_{N25X/O}$;

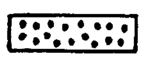

repräsentiert die ribosomale Bindungsstellen RBSII,SphI und RBSII,3A+5A; → repräsentiert die kodierende Region unter Kontrolle dieser ribosomalen Bindungsstellen;

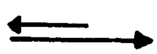

repräsentiert die für die DNA-Replikation benötigte Region (repl.);

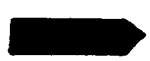

repräsentiert Regionen, die für Dihydrofolatreduktase (dhrf), Chloramphenicolacetyltransferase (cat), β-Lactamase (bla), lac-Repressor (lacI) oder Neomycinphosphotransferase (neo) kodieren.

Fig. 1

Schematische Darstellung des Plasmids pDS8/RBSII,SphI.

Fig. 2

Nukleotidsequenz des XhoI/XbaI-Fragments des Plasmids pDS8/RBSII,SphI das das regulierbare Promotor/Operator-Element $P_{N25}x_{/O}$, die ribosomale Bindungstelle RBSII,SphI, das dhfr-Gen, den Terminator $t_o$, das cat-Gen und den Terminator T1 enthält. Die in Fig. 1 angegebenen Restriktionsenzymschnittstellen sind überstrichen und die Region unter der Kontrolle von RBSII,SphI, die für die Dihydrofolat-Reduktase kodiert, ist unterstrichen. Zusätzlich ist der pBR322 Teil von pDS8/RBSII,SphI schematisch gezeichnet, wobei die angegebenen Zahlen sich auf die Nukleotidsequenz von pBR322 (Sutcliffe, Cold Spring Harbor Symp. Quant. Biol. 43, 77-90 [1979]) beziehen.

Fig. 3

Schematische Darstellung des Plasmids pDS6/RBSII,3A + 5A.

Fig. 4

Nukleotidsequenz des XhoI/XbaI-Fragments des Plasmids pDS6/RBSII,3A + 5A, das das regulierbare Promoter/Operator-Element $P_{N25}x_{/O}$, die ribosomale Bindungstelle RBSII,3A + 5A, den Terminator $t_o$, das cat-Gen und den Terminator T1 enthält. Die in Fig.3 angegebenen Restriktionsenzymschnittstellen sind überstrichen und die Region unter der Kontrolle von RBSII,3A + 5A ist unterstrichen. Zusätzlich ist der pBR322 Teil von pDS6/RBSII,3A + 5A schematisch gezeigt, wobei die angegebenen Zahlen sich auf die Nukleotidsequenz von pBR322 (Sutcliffe, supra) beziehen.

Fig. 5

Schematische Darstellung des Plasmids pDMI,1.

Fig. 6

DNA-Sequenz des Plasmids pDMI,1. Die in Fig.5 angegebenen Restriktionsendonukleaseschnittstellen sind überstrichen und die Regionen, die für die Neomycinphosphotransferase (neo) und den lac-Repressor (lacI) kodieren, sind unterstrichen.

Fig. 7

Reinigung von p190-1 auf der NTA-Nickelchelatsäule. Die Spur des UV-Aufzeichnungsgerätes (280 nm) ist als durchgehende oder gepunktete Linie angegeben. Der pH-Gradient ist mit offenen Symbolen (o) und der $(NH_4)_2SO_4$-Gradient mit geschlossenen Symbolen (o) dargestellt. Pufferwechsel sind mit grossen Buchstaben bezeichnet. A: Auftragung des Rohextraktes. B: Waschen mit 0,1 M Tris/HCl (pH 7,5), 0,2 M NaCl. C: Waschen mit 0,1 M Tris/HCl (pH 6,0), 0,2 M NaCl. D: Washen mit 0,1 M Tris/HCl (pH 6,0), 1 M $(NH_4)_2SO_4$. E: Washen mit 0,1 M Tris/HCl (pH 4,5), 1 M $(NH_4)_2SO_4$. F: Gradientenelution von p190-1 mit 0,1 M Tris/HCl (pH 4,5), 0,2 M NaCl.

Fig. 8

Analytische SDS-Polyacrylamidgelelektrophorese (SDS-PAGE) von gereinigtem p190-1. Spuren 3 bis 6 zeigen, dass das Polypeptid in homogener Form vorliegt. Spur 1 zeigt ein E.coli M15(pGC1,pDMI,1)-Lysat, woraus p190-1 gereinigt wurde. Spur 2 zeigt eine Molekulargewichtsmarkiermischung (Biorad). Die elektrophoretischen Mobilitäten der Markierproteine Phosphorylase b [$M_r$ = 92'500], Rinderserumalbumin [$M_r$ = 66'200], Ovalbumin [$M_r$ = 45'000], Carboanhydrase [$M_r$ = 31'0000], Soyabohnen-Trypsininhibitor [$M_r$ = 21'500] und Lysozym [$M_r$ = 14'400] sind durch die $M_r$-Werte (x $10^3$) angegeben. Spuren 3 bis 6 enthalten 0.25, 0.5, 1.0 und 2.0 $\mu$g p190-1.

Fig. 9

Analytische SDS-PAGE von gereinigtem p190-3 unter reduzierenden (Spuren 2 und 3) und nichtreduzierenden (Spuren 4 und 5) Bedingungen. Das Polypeptid ist in homogener Form (vgl. Spuren 2 und 3). Spuren 4 und 5 zeigen, dass das E.coli Lysat Multimere des Polypeptids enthält, die durch Bildung von Disulfidbrücken zwischen Cysteinseitenketten in p190-3 entstehen. Spuren 2 und 4 enthalten 5 μg und Spuren 3 und 5 10 μg Protein. Spur 1 ist eine Molekulargewichtsmarkiermischung (Pharmacia). Die elektrophoretischen Mobilitäten der Markierproteine (Phosphorylase b [$M_r$ = 94'000], Rinderserumalbumin [$M_r$ = 67'000], Ovalbumin [$M_r$ = 43'000], Carboanhydrase [$M_r$ = 30'000], Soyabohnen-Trypsininhibitor [$M_r$ = 20'100] und α-Lactalbumin [$M_r$ = 14'400]) sind durch ihre $M_r$-Werte (x $10^3$) angegeben.

Fig. 10

Western-Blot und Proteinanalyse von p190-1, p190-2b und p190-3. Teil A zeigt einen Western-Blot von bakteriellen Lysaten von mit pGC1, pGC2b oder pGC3 transformierten E.coli M15(pDMI,1), der mit Kaninchen anti-190 Serum analysiert wurde. Teil B zeigt die gleichen Proben, die diesmal aber mit vereinigten menschlichen Seren aus endemischen Gebieten analysiert wurden. Das Kaninchenserum reagierte mit allen drei Antigenen, währenddem das menschliche Antiserum nur mit Banden reagierte, die in der Grösse denjenigen der Polypeptide p190-1 und p190-3 entsprechen. Die Antigen-Antikörperkomplexe wurden unter Verwendung der Meerretich-Peroxidase-Reakion sichtbar gemacht. Teil C zeigt ein Coomassie-Blau gefärbtes Gel der gleichen Proben. ST bedeutet ein Molekulargewichtsstandard. Die Grössen sind in Kilo-Dalton (= 1000 Dalton) Molekulargewicht angegeben.

Fig. 11

Bakterienlysate von, mit PGC1, pGC2b oder pGC3 (Spuren 1, 2 bzw. 3) transformierten E.coli wurden auf SDS-Polyacrylamidgelen aufgetrennt und auf Nitrocellulose übertragen. Der Western-Blot wurde dann unter Verwendung eines, gegen p190-3 gerichteten Kaninchenserums, untersucht. Antigen/Antikörperkomplexe wurden mittels der Meerrettich-Peroxidasereaktion sichtbar gemacht. Das gegen p190-3 gerichtete Kaninchenantiserum reagierte wie erwartet mit allen drei Antigenen (p190-1, p190-2b und p190-3).

Beispiel 1

Konstruktion des Plasmids pDS6/RBSII,SphI,His-His

A. Prinzip

Unter Verwendung eines chemisch synthetisierten Oligonukleotidadaptors wurde ein DNA Fragment hinter der RBSII,SphI Sequenze des Vektors pDS8/RBSII,SphI eingesetzt. Dieses Fragment kodiert für ein Affinitätspeptid das zwei benachbarte Histidine enthält. Ein Polypeptid, das dieses Affinitätspeptid enthält, kann durch selektive Bindung an ein Affinitätschromatographieharz, das spezifisch ist für benachbarte Histidinreste, gereinigt werden.

B. Herstellung des synthetischen Oligonukleotides

Die synthetischen Oligonukleotide (1) und (2) mit der folgenden Sequenz

```
                  His-His
(1)  5'-        CATCACGCCCCCG       -3'
(2)  3'-GTACGTAGTGCGGGGGCCTAG-5'
        SphI                BamHI
```

wurden unter Verwendung von Glas mit kontrollierter Porengrösse (CPG) als Trägermaterial hergestellt (Sproat et al., Tetrahedr. Lett., 24, 5771-5774 [1983]); Adams et al., J. Amer. Chem. Soc., 105, 661-663

[1983]). Die lyophilisierten Oligonukleotide wurden in Wasser während einer Stunde bei 4°C gelöst und auf eine DNA Konzentration von 100 nMol/ml eingestellt. 100 pMole jedes Oligonukleotids wurden mit 1 µl [$^{32}$P]-ATP (2 pMol, 5000 Ci/mMol), 1 Einheit T4-Polynukleotidkinase (Gibco-BRL, Basel) in 10 µl 50 mM Tris/HCl (pH 8,5), 10 mM $MgCl_2$, während 10 Minuten bei 37°C behandelt. Nach Zugabe von 1 µl 5 mM ATP wurde die Reaktion durch Erhitzen der Proben aus 65°C während 7 Minuten gestoppt.

C. Konstruktion des pDS6/RBSII,SphI-His,His

4 µg des pDS8/RBSII,SphI wurden mit SphI gemäss den Angaben des Herstellers (Gibco-BRL) verdaut. Das Enzym wurde durch Erhitzen der Proben während 7 Minuten auf 65°C inaktiviert und die DNA wurde mit 2,5 Volumen Aethanol in Gegenwart von 0,3 M Kaliumacetat gefällt. Der Niederschlag wurde während 2 Minuten in einem Speed-vac-Konzentrator getrocknet und anschliessend in T4-Ligasepuffer (50 mM Tris/HCl (pH 7,8), 10 mM $MgCl_2$, 10 mM DTT, 500 µM ATP) gelöst. 50 pMole des phosphorylierten Oligonukleotids, das das SphI-BamHI-Adaptorfragment, das durch Hybridisierung der Oligonukleotide (1) und (2) (siehe oben) erhalten wurde, gelöst in 1x Ligasepuffer, wurden zugegeben und das Probevolumen auf ein Endvolumen von 25 µl gebracht. Die Ligierung wurde während 3 Stunden bei 22°C unter Verwendung von 1 µl DNA-Ligase (1 Weiss-Einheit, Boehringer Mannheim) durchgeführt. Die Reaktion wurde durch Inkubation der Probe während 7 Minuten bei 65°C gestoppt. Die DNA wurde präzipitiert, getrocknet und dann in Restriktionspuffer (50 mM Tris/HCl (pH 8), 10 mM $MgCl_2$, 50 mM NaCl) aufgelöst. Nach Zugabe von 20 Einheiten BamHI und 10 Einheiten XbaI wurde die Probe während 1 Stunde bei 37°C inkubiert. Die Verdauung wurde durch Erhitzen der Proben während 7 Minuten auf 65°C gestoppt. Nach der Zugabe von 10x Gel-Probenpuffer (100 mM Tris/HCl (pH 8), 0,5% Bromphenolblau, 0,5% Xylencyanol, 10 mM EDTA, 50% Glyzerin) wurde die DNA Fragmente auf einem 6%-igen Polyacrylamidgel getrennt. Das Gel wurde mit Ethidiumbromid (1 µg/ml) während 5 Minuten gefärbt und die DNA unter 300 nm UV-Licht sichtbar gemacht. Als Markier-DNA wurde Phagen ØX DNA die mit HaeIII verdaut worden war (Gibco-BRL, Basel) verwendet. Die DNA Bande die den regulierbaren Promotor $P_{N25X/O}$, die ribosomale Bindungsstelle RBSII,SphI, den hybridisierten Adaptor, das bla-Gen und den Replikationsursprung enthielt (Vektor DNA) wurde mit einem Skalpell aus dem Gel geschnitten und in ein 1,5 ml Eppendorfreagenzröhrchen überführt.

Mit einer Pipettenspitze wurde das Acrylamidgelstück, das die DNA-Bande enthielt, zu einem feinen Granulat zerkleinert. Nach Zugabe von 200 µl 1x TE-Puffer (10 mM Tris/HCl (pH 8,0), 1 mM EDTA) wurde die Probe über Nacht bei 4°C auf einem Eppendorf-Minischüttler geschüttelt. Die Probe wurde dann während 15 Minuten bei 12'000 rpm zentrifugiert, der Ueberstand in ein neues Eppendorfröhrchen überführt und die DNA mit 2,5 Volumen Aethanol in Gegenwart von 0,3 M Kaliumacetat gefällt. Schliesslich wurde die DNA unter Vakuum getrocknet und in 10 µl TE-Puffer aufgelöst.

4 µg des Plasmids pDS6/RBSII,3A+5A wurden mit XbaI und BamHI wie vom Hersteller (BRL-Gibco, Basel) empfohlen, verdaut. Nach der Inaktivierung des Enzyms (7 Minuten, 65°C) wurde die DNA wie oben beschrieben gefällt. Der Niederschlag wurde in 20 µl 50 mM Tris/HCl (pH 8), enthaltend zwei Einheiten Kälberdünndarmphosphatase (CIP, Boehringer Mannheim), gelöst und während einer Stunde bei 37°C inkubiert. Die Reaktion wurde durch Erhitzen der Probe während 7 Minuten auf 65°C gestoppt, Gel-Probenpuffer wurde zugegeben und die DNA wurde auf einem 6%-igen Polyacrylamidgel elektrophoretisch aufgetrennt. Das Fragment das den Polylinker, den Terminator $t_0$, das cat-Gen und den Terminator T1 enthielt, wurde wie oben beschrieben isoliert.

Die Vektor DNA und das isolierte Fragment (je 0,5 µl) wurden in einem Volumen von 30 µl mit zwei Einheiten T4-DNA-Ligase in Ligasepuffer während 3 Stunden bei 22°C ligiert. Die Ligation wurde durch Erhitzen der Probe während 7 Minuten auf 65°C gestoppt. Die Transformation wurde, wie von Morrison (Methods Enzymol. 68, 326-331 [1979]) beschrieben, durchgeführt, wobei als Wirt E.coli M15 enthaltend das Plasmid pDMI,1 verwendet wurde. Die Zellen wurden auf LB-Agarplatten die 100 µg/ml Ampicillin und 25 µg/ml Kanamycin enthielten ausplattiert. Die Platten wurden über Nacht bei 37°C inkubiert.

Wie erwartet wurden in den Kontrolligationen keine Transformanten erhalten. Die Ligation enthaltend Vektor DNA plus das Fragment ergab etwa 100 Kolonien. Einzelne Kolonien wurden mit einem Zahnstocher gepflückt und in 10 ml LB-Medium enthaltend 100 µg/ml Ampicillin und 25 µg/ml Kanamycin wachsen gelassen. Plasmid DNA wurde gemäss der Methode von Birnboim et al. (Nucleic Acids Res. 7, 1513-1523 [1979] extrahiert. Der erhaltene DNA Niederschlag wurde in 200 µl TE-Puffer aufgelöst.

D. Sequenzanalyse von pDS6/RBSII,SphI-His,His

Zur Bestätigung, dass der SphI-BamHI-Adaptor in das Plasmid pDS6/RBSII,SphI-His,His eingebaut worden war, wurde die doppelsträngige DNA unter Verwendung eines Starters, der mit [γ-$^{32}$P]-ATP markiert

worden war, sequenziert. Die Startersequenz enthält die Nukleotide von Position 199-218 von pDS8/RBSII,SphI, und endet deshalb 6 Nukleotide vor dem ATG der SphI-Schnittstelle. 15 µl der isolierten DNA (0,3 pMol) wurden mit Aethanol präzipitiert und einmal mit 80%-igem Aethanol gewaschen. Nach dem Trocknen des Niederschlags während 2 Minuten in einem Speed-vac-Konzentrator wurde der Niederschlag in 8 µl 1/4 TE-Puffer aufgelöst. Nach der Zugabe von 2 pMol der, mit [$\gamma$-$^{32}$P]-ATP endmarkierten Startersequenz wurde die Probe während 5 Minuten auf 95 °C erhitzt und dann während 5 Minuten in ein 42 °C Wasserbad getaucht. Nun wurde die Dideoxykettenabbruchmethode gemäss Sanger et al. [Proc. Natl. Acad. Sci. USA 74, 5463-5467 [1977] zur Sequenzierung verwendet.

Die Sequenzdaten zeigten, dass der Adaptor wie gewünscht in das Plasmid pDS6/RBSII,SphI-His,His eingebaut worden war.

Beispiel 2

A. Prinzip

Ein HpaI-HaeIII Fragment (527 Basenpaare lang) wurde von einem Klon der das p190 Gen des K1 Isolates von P. falciparum enthält (Mackay et al. supra) isoliert. Ein BamHI-Verbindungsstück wurde an die stumpfen Enden dieses Fragmentes ligiert. Nach der Verdauung mit BamHI wurde das Fragment in den Expressionsvektor pDS6/RBSII,SphI-His,His integriert, der zuvor mit BamHI linearisiert worden war.

B. Vorbereitung des Fragmentes 1

6 µg eines Klons der das p190 Gen des K1 Isolates von P. falciparum enthielt, wurde mit 15 Einheiten HpaI und 25 Einheiten HaeIII in 40 µl 6 mM NaCl, 6 mM Tris/HCl (pH 7,6), 6 mM $MgCl_2$ während 1 Stunde bei 37 °C verdaut. Nach Zugabe von 4,5 µl 10x Gel-Probenpuffer (100 mM Tris/HCl (ph 8), 0,5% Bromphenolblau, 0,5% Xylencyanol, 10 mM EDTA, 50% Glyzerin) wurden die DNA Fragmente auf einem 6%-igen Polyacrylamidgel in 1x TBE (89 mM Tris/HCl (pH 8,0), 89 mM Borsäure, 2 mM EDTA) als Gelelektrophoresepuffer während 3 Stunden bei 250 Volt aufgetrennt. Das Gel wurde während 5 Minuten mit Ethidiumbromid (1 µg/ml) gefärbt und die DNA unter 300 nM UV-Licht sichtbar gemacht. Als Marker wurde Phagen ØX DNA, die mit HaeIII (Gibco-BRL, Basel) verdaut worden war, verwendet. Die DNA Bande mit der gewünschten Sequenz (527 Basenpaare Länge) wurde aus dem Gel ausgeschnitten und wie oben beschrieben gereinigt.

10 pMole eines phosphorylierten BamHI-Verbindungsstücks (CCGGATCCGG) wurden zum Fragment gegeben und mit 5 Einheiten T4-DNA-Ligase während 3 Stunden bei 22 °C inkubiert. Die Reaktion wurde durch Erhitzen der Probe während 7 Minuten auf 65 °C gestoppt. Nach Aethanolpräzipitation der DNA wurde der Niederschlag in Restriktionspuffer resuspendiert und mit 20 Einheiten BamHI während 2 Stunden bei 37 °C in einem Totalvolumen von 30 µl verdaut. Nach der Inaktivierung des Enzyms (7 Minuten, 65 °C) wurde Gel-Probenpuffer zugegeben und das Fragment wurde nach der Elektrophorese wie oben beschrieben isoliert. Der so erhaltene DNA Niederschlag wurde in 30 µl TE-Puffer resuspendiert.

C. Herstellung des Plasmids pDS6/RBSII,SphI-His,His

4 µg des Plasmids pDS6/RBSII,SphI-His,His wurden während 2 Stunden bei 37 °C mit 20 Einheiten BamHI in 30 µl Restriktionspuffer verdaut. Nach Inaktiverung der Restriktionsenzyms (7 Minuten, 65 °C) wurde die DNA wie oben beschrieben präzipitiert. Der Niederschlag wurde in 20 µl 50 mM Tris/HCl (pH 8), enthaltend 2 Einheiten Kälberdünndarmphosphatase (CIP, Boehringer Mannheim), aufgelöst und während 1 Stunde bei 37 °C inkubiert. Die Reaktion wurde durch Erhitzen der Probe während 7 Minuten auf 65 °C gestoppt. Dann wurde Gel-Probenpuffer zugegeben und die DNA elektrophoretisch auf einem 6% Polyacrylamidgel aufgetrennt. Nach der Sichtbarmachung unter dem UV-Licht wurde die Vektor DNA aus dem Gel ausgeschnitten und wie oben beschrieben isoliert. Der erhaltene DNA Niederschlag wurde in 20 µl TE-Puffer resuspendiert.

D. Zusammenbau des Plasmids pGC1

10 µl der Vektor DNA und die Hälfte des isolierten Fragments 1 wurden in einem Volumen von 30 µl mit 2 Einheiten T4-DNA-Ligase bei 22 °C während 3 Stunden in Ligationspuffer ligiert. Eine Kontrolligation ohne Fragment 1 DNA wurde gleichzeitig durchgeführt. Die Reaktionen wurden durch Erhitzen der Proben während 7 Minuten auf 65 °C gestoppt. Transformationen wurden, wie von Morrison (supra) beschrieben,

unter Verwendung von E.coli M15, enthaltend das Plasmid pDMI,1, durchgeführt. Die Zellen wurden auf LB-Agarplatten enthaltend 100 µg/ml Ampicillin und 25 µg/ml Kanamycin ausplattiert. Die Platten wurden über Nacht bei 37°C inkubiert.

Wie erwartet wurden in den Kontrolligationen keine Transformanten erhalten. Die Ligation enthaltend Vektor DNA plus Fragment 1 ergab etwa 200 Kolonien. Einzelne Kolonien wurden mit einem Zahnstocher gepflückt und wie oben beschrieben anlaysiert. Die Plasmid DNAs (je 5 µl) wurden in Restriktionspuffer mit BamHI verdaut um das Fragment 1 herauszuschneiden. Alle Plasmide wurden linearisiert aber kein Fragment konnte herausgelöst werden. Dies deutet darauf hin, dass eine BamHI-Schnittstelle während der Konstruktion verloren ging (siehe unten).

Die Plasmide wurde wie oben beschrieben in E.coli M15, enthaltend das Plasmid pDMI,1, transformiert. Einzelne Kolonien wurden gepflückt und in 3 ml LB-Medium enthaltend 100 µg/ml Ampicillin und 25 µg/ml Kanamycin bis auf eine optische Dichte bei 600 nm ( $OD_{600}$) = 0,6 aufgewachsen. Eine 500 µl Probe wurde von der Kultur abgetrennt (nicht induzierte Kontrollprobe) und zum Rest IPTG, bis zu einer Endkonzentration von 1 mM, zugegeben. Die Inkubation wurde während 3 Stunden bei 37°C unter Schütteln weitergeführt (induzierte Proben). Je eine 500 µl Probe der induzierten und der nicht induzierten Kultur wurden während 3 Minuten bei 12'000 rpm zentrifugiert. Der Ueberstand wurde entnommen und der Zellniederschlag in SDS-Gel-Probenpuffer (0,1 M Tris/HCl (pH 6,8), 3% β-Mercaptoäthanol, 20% Glyzerin, 0, 1% Bromphenolblau, 3% SDS) resuspendiert. Die Proben wurden während 5 Minuten gekocht, auf Eis gesetzt und anschliessend auf einem 12,5%-igen SDS Polyacrylamidgel (Laemmli, Nature 227, 680-685 [1970]) während 3 Stunden bei 50 mA aufgetrennt. Das Gel wurde während 1 Stunde mit Coomassieblau bei Raumtemperatur gefärbt und in 10%-iger Essigsäure enthaltend 10% Methanol während 3 Stunden bei 60°C entfärbt. Die Hälfte der analysierten Kolonien zeigten eine starke Bande bei 24 kD Molekulargewicht nach der IPTG-Induktion. Die andere Hälfte der Kolonien hatte keine weitere neue Bande.

E. Sequenzanalyse von pGC1

Die Nukleotidsequenz der Plasmid DNA wurde unter Verwendung von Startersequenzen, die mit [$\gamma$-$^{32}P$] ATP markiert worden waren, bestimmt. Eine Startersequenz enthielt die Nukleotide von Position 199-218 von pDS8/RBSII,SphI und endet deshalb 6 Nukleotide vor dem ATG der SphI Restriktionsschnittstelle. Die andere Startersequenz enthielt die Nukleotide 928-896 von pDS8/RBSII,SphI. Auf diese Art und Weise können Fragmente, die in die BamHI Restriktionsenzymschnittstelle integriert sind, von beiden Seiten sequenziert werden. Die Sequenzdaten zeigten, dass das p190 Fragment in die BamHI Schnittstelle integriert war und zwar in Serie mit dem ATG des RBSII,SphI, dass aber die BamHI Restriktionsschnittstelle am Ende dieses Fragments in allen Konstruktionen deletiert war.

F. Vorbereitung des Harzes für die Reinigung der Polypeptide mit benachbarten Histidinresten

41,7 g Bromessigsäure wurden in 150 ml 2 N Natronlauge gelöst und auf 0°C gekühlt. Dazu wurde unter Rühren eine Lösung von 42 g $N^{\varepsilon}$-Z-L-Lysin in 225 ml 2 N Natronlauge bei 0°C langsam zugetropft. Nach 2 Stunden wurde die Kühlung abgestellt und über Nacht weitergerührt. Dann wurde das Reaktionsgemisch während 2 Stunden bei 50°C gehalten und anschliessend wurden 450 ml 1 N Salzsäure zugesetzt. Nachdem das Gemisch abgekühlt war, wurden die ausgeschiedenen Kristalle abfiltriert. Das Produkt wurde in 1 N Natronlauge gelöst und mit der gleichen Menge 1 N Salzsäure erneut gefällt und abfiltriert. Es wurden 40 g N-[5-Benzyloxycarbonylamino-1-carboxypentyl]-iminodiessigsäure, in Form weisser Kristalle, Smp. 172-174°C (Zers.), $[\alpha]_D$ = +9,9° (c = 1; 0,1 N NaOH), erhalten.

7,9 g des erhaltenen Lysinderivats wurden in 49 ml 1 N Natronlauge gelöst und nach Zusatz von 5% Pd/C bei Raumtemperatur und Normaldruck hydriert. Der Katalysator wurde abfiltriert und das Filtrat eingedampft. Es resultierten 6,2 g N-[5-Amino-1-carboxypentyl]-iminodiessigsäure.

100 ml Sepharose® CL-6B (Pharmacia) wurden zweimal mit ca. 500 ml Wasser gewaschen und dann mit 16 ml 4 N Natronlauge und 8,22 ml Epibromhydrin während 4 Stunden bei 30°C umgesetzt. Das Totalvolumen des Reaktionsgemisches war 200 ml. Anschliessend wurde die aktivierte Sepharose abfiltriert, mit Wasser neutral gewaschen und zurück in das Reaktionsgefäss transferiert. 6,2 g N-[5-Amino-1-carboxypentyl]-iminodiessigsäure wurden in 50 ml Wasser gelöst und zusammen mit 10,6 g festem Soda zur aktivierten Sepharose gegeben. Das Gemisch wurde bei 60°C über Nacht langsam gerührt. Das resultierende NTA-Chelatharz mit der Formel [Sepharose®CL-6B]-O-$CH_2$-CH(OH)-$CH_2$-NH-$(CH_2)_4$-CH-(COOH)-N$(CH_2COOH)_2$ wurde anschliessend nacheinander mit 500 ml Wasser, 100 ml wässrigem $NiSO_4 \cdot 6H_2O$(2 Gew.-%), 200 ml Wasser, 200 ml 0,2 M Essigsäure (enthaltend 0,2 M NaCl und 0,1 Gew./Vol.% Tween 20) und 200 ml Wasser gewaschen. Die Nickelionenkonzentration des resultierenden

Chelatharzes der Formel [Sepharose®CL-6B]-O-$CH_2$-CH(OH)-$CH_2$-NH-$(CH_2)_4$-CH(COOH)-N$(CH_2COO^-)_2$-$Ni^{2+}$ betrug etwa 7,1 Mikromol/ml.

G. Reinigung des Polypeptids p190-1

Eine Kultur von E.coli M15(pGC1, pDMI,1) wurde während 12 Stunden bei 37°C unter heftigem Schütteln in LB-Medium enthaltend 100 µg/ml Ampicillin und 25 µg/ml Kanamycin wachsen gelassen. Bei einer optischen Dichte von 0,7 bei einer Wellenlänge von 600 nm ($OD_{600}$) wurde die Expression des Polypeptids p190-1 durch Zugabe von IPTG (Endkonzentration 1 mM) induziert. Nach zusätzlichen 6 Stunden Inkubation wurden die Zellen zur Zentrifugation geerntet.

Die geernteten Zellen (46,5 g) wurden in 7 M Guanidin•HCl (140 ml) während 1 Stunde bei 4°C aufgebrochen. Die löslichen Proteine wurden durch Zentrifugation bei 10'000 x g während 15 Minuten bei 4°C von den unlöslichen Partikeln abgetrennt. Der Ueberstand wurde fünffach mit 0,1 M Tris/HCl (pH 7,5), 0,2 M NaCl verdünnt und wie oben beschrieben zentrifugiert, was einen Rohextrakt ergab.

Der Rohextrakt wurde auf die NTA-Nickelchelatsäule die oben beschrieben ist (Sepharose CL-6B mit Nitrilotriessigsäure als Liganden, Säulen-Durchmeser = 5,0 cm, Länge = 5,5 cm, Durchflussrate 500 ml/Stunde) aufgetragen. Das Harz war mit 0,1 M Tris/HCl (pH 7,5), 0,2 M NaCl äquilibriert worden. Proteinverunreinigungen, die eine schlechte Nickelchelatbindungskapazität hatten oder weniger hydrophobe Eigenschaften hatten als p190-1, wurden aus der Säule ausgewaschen und zwar zuerst mit 0,1 M Tris/HCl (ph 7,5), 0,2 M NaCl, dann mit 0,1 M Tris/HCl (pH 6,0), 0,2 M NaCl (Zerstörung der Nickel-Proteinkomplexe niederer Affinität), dann mit 0,1 M Tris/HCl (pH 6,0), 1 M $(NH_4)_2SO_4$ (Induktion der hydrophoben Bindungen) und schliesslich mit 0,1 M Tris/HCl (pH 4,5) 1 M $(NH_4)_2SO_4$ (Elution der Nickelchelat-Proteinkomplexe, die nicht über hydrophobe Wechselwirkungen binden). Die Elution von p190-1 erfolgte durch Absenken der $(NH_4)_2SO_4$) Konzentration (siehe Figur 7). Ausbeute von p190-1: 66 mg, Reinheit ca. -90%.

Die gesammelten Fraktionen der NTA-Säule wurden 15-fach mit $H_2O$ verdünnt und nach Anpassung des pH-Wertes auf pH 6,0 an eine Fractogel TSK CM-650(M) Säule (Merck, Durchmesser = 1,6 cm, Länge = 6,7 cm, Durchflussrate = 1,5 ml/Minute, Equilibrationspuffer = 50mM Natriumphosphat (pH 6,0)) absorbiert. Das p190-1 Protein wurde mit einem linearen Gradienten von 0 bis 0,6 M NaCl während 2 Stunden eluiert. Die Fraktionen enthaltend p190-1 wurden vereinigt und lyophilisiert.(Ausbeute ~50 mg, Reinheit ~95%).

Zur Entfernung von Spuren von Proteinen mit hohem Molekulargewicht eignet sich entweder präparative HPLC unter Verwendung einer Nucleosil 300-S-C18 Säule (Macherey & Nagel, Durchmesser = 0,4 cm, Länge = 33 cm, Durchflussmenge = 1 ml/Minute, mit 0,1% Trifluoracetessigsäure als Aequilibrationspuffer, Elution mit einem 0-70% Gradienten von Acetonitril) oder eine Gelfiltration an Sephacryl™ S-200 (Pharmacia, Durchmesser = 2,6 cm, Länge = 81 cm, Durchflussrate = 40 ml/Stunde, Puffer = 1% Ameisensäure). Das gereinigte p190-1 Protein wurde nach Lyophilisation als ein salzfreies Pulver erhalten.

Beispiel 3

A. Prinzip

Ein 407 Basenpaare langes AluI-BamHI Fragment wurde von einem Klon, der das p190 Gen des K1 Isolates von P. falciparum (Mackay et al., supra) enthält, isoliert. Ein BamHI Verbindungsstück wurde an das stumpfe Ende des Fragmentes angebracht. Nach Verdauung mit BamHI wurde das Fragment in den Expressionsvektor pDS6/RBSII,SphI-His,His, der zuvor mit BamHI linearisiert worden war, integriert.

B. Herstellung des Fragmentes 2

6 µg eines Klons enthaltend das p190 Gen des K1 Isolates von P. falciparum wurden mit 30 Einheiten AluI und 15 Einheiten BamHI in Restriktionspuffer verdaut. Nach der Isolation der Fragmente und dem Anknüpfen eines BamHI Verbindungsstücks (CGGATCCG) wurde das Fragment wie oben beschrieben gereinigt. Der erhaltene DNA Niederschlag wurde in 30 µl TE-Puffer resuspendiert.

C. Vorbereitung des Plasmids pDS6/RBSII,SphI-His,His

Die Vorbereitung des Expressionsvektors wurde in Beispiel 2,C beschrieben.

D. Zusammenbau des Plasmids pGC2

Die Ligierung, die Transformation und die DNA Analyse wurden wie in Beispiel 2,D beschrieben durchgeführt. In 4 Plasmiden konnte durch BamHI Verdauung ein Fragment der erwarteten Grösse (410 Basenpaare) herausschnitten werden. Die anderen Plasmide wurden nur linearisiert.

E. Sequenzanalyse von pGC2

Die Plasmid DNA's wurden wie oben beschrieben sequenziert. Das Vorhandensein von 2 verschiedenen Plasmidtypen wurde beobachtet. In pGC2a war das Fragment 2 wie erwartet in der richtigen Orientiertung, in Serie mit dem ATG der ribosomalen Bindungsstelle RBSII,SphI integriert und enthielt an beiden Enden BamHI Restriktionsenzymschnittstellen. Der andere Typ Plasmide wurde mit pGC2b bezeichnet, und enthielt eine kleine Deletion vor der Malaria spezifischen Sequenz. Trotz dieser Deletion sind die ganze p190 Gensequenz und das Affinitätspeptid, das die benachbarten Histidinreste enthält, in diesem Plasmid integriert. Die Analyse der gesamten zellulären Proteine auf einem SDS-Polyacrylamidgel zeigte, dass alle Klone, die die p190 Sequenzin der richtigen Orientierung und in Serie mit dem ATG der RBSII,SphI enthielten, ein deutlich sichtbares Protein von etwa 20 kD Molekulargewicht exprimierten. Das Plasmid mit der kleinen Deletion zwischen RBSII,SphI und der p190 Sequenz (siehe oben) exprimierte etwa fünfmal mehr dieses Polypeptids im Vergleich zu den Konstruktionen ohne Deletion. Die Polypeptide, die von pGC2a hergeleitet sind, wurden mit p190-2a bezeichnet und die Polypeptide, die von pGC2b hergeleitet sind, wurden mit p190-2b bezeichnet.

Wie oben erklärt, wurde das Plasmid pGC2b, das die kleine Deletion enthält, durch Zufall durch eine Deletionsmutation erhalten. Ein Fachmann kann ebenfalls ein Plasmid des Typs pGC2b aus dem Plasmid pGC2a erhalten, indem er das Plasmid durch gezielte Mutagenese (Morinaga et al., Bio/Technology 7, 636-639 [1984] unter Verwendung einer kleinen synthetischen Oligonukleotidstartersequenz, die der Nukleotidsequenz, die zur gewünschten Deletion benachbart ist aber bei der die Nukleotide, die zu deletieren sind, fehlen, herstellt.

F. Reinigung des Polypeptids p190-2b

Das Polypeptid p190-2b wurde auf gleiche Art und Weise gereinigt wie das Polypeptid p190-1 (siehe Beispiel 2). E.coli-Zellen (16,6 g) wurden in 50 ml 7M Guanidin•HCl aufgebrochen und der Rohextrakt wurde auf eine NTA-Nickelchelatsäule (Durchmesser 2,6 cm, Länge 8,8 cm, Durchflussrate 160 ml/Stunde) appliziert. Eine Ausbeute von 14 mg p190-2b mit einer Reinheit von ungefähr 90% wurde erhalten.

Die vereinigten Fraktionen der NTA-Säule wurden 15-fach mit $H_2O$ verdünnt und nach Anpassung des pH's auf 7,5 an eine Fractogel TSK DEAE-650$^{(M)}$ Säule (Durchmesser = 1,6 cm, Länge = 8,8 cm, Durchflussrate = 120 ml/Stunde Aequilibrationspuffer = 25 mM Tris/HCl (pH 7,5)) adsorbiert. Das Protein p190-2b wurde mit einem linearen Gradienten von 0-0,6 M NaCl während 3 Stunden eluiert. Die p190-2b enthaltenden Fraktionen wurden vereinigt und lyophilisiert. Ausbeute: 9 mg, Reinheit >95%.

Eine abschliessende Reinigung wurde ähnlich wie in Beispiel 2 beschrieben mit präparativer HPLC durchgeführt, wobei eine RP-18 Säule (Macherey & Nagel, Durchmesser 10 mm, Länge 250 mm, Durchflussrate 5 ml/Minute, Elution mit einem Gradienten von Acetonitril, Probelösung 2-5 mg des DEAE-Pools) verwendet wurde. Ausbeute: ~5 mg.

Das Polypeptid p190-2a kann auf gleich Art und Weise gereinigt werden.

Beispiel 4

A. Prinzip

Das BamHI-Fragment (Fragment 3) mit der Sequenz, die vom p190-Gen hergeleitet ist, wurde aus dem Plasmid pGC2a, das beide BamHI Restriktionsschnittstellen besitzt, isoliert. Dieses Fragment wurde in den BamHI verdauten Vektor pGC1 integriert, worauf das Plasmid pGC3 entstand, das ein Fusionspolypeptid exprimieren kann.

B. Vorbereitung des Fragments 3

4 $\mu$g des Plasmids pGC2a wurden mit 15 Einheiten BamHI in 20 $\mu$l Restriktionspuffer während 1 Stunde bei 37 °C verdaut. Nach der Hitzeinaktivierung des Enzyms (7 Minuten, 65 °C) wurde das Fragment

wie oben beschrieben isoliert und in 20 µl TE-Puffer resuspendiert.

C. Vorbereitung des Plasmids pGC1

4 µg des Plasmids pGC1 wurden mit 15 Einheiten BamHI verdaut. Die DNA wurde präzipitiert und mit Kälberdünndarmphosphatase wie oben beschrieben behandelt. Die linearisierte Plasmid DNA wurde auf einem 6%-igen präparativen Polyacrylamidgel (siehe oben) gereinigt und in 10 µl TE-Puffer resuspendiert.

D. Zusammenbau von pGC3

Das mit BamHI linearisierte Plasmid pGC1 (= Vektor DNA) wurde mit 10 µl des isolierten Fragmentes 3 ligiert (2 Einheiten T4-DNA-Ligase, Ligationspuffer, 3 Stunden, 22 °C, 30 µl Totalvolumen). Eine Kontrolligierung ohne Fragment 3 DNA wurde parallel durchgeführt. Die Ligationen wurden durch Erhitzen der Proben während 7 Minuten auf 65 °C gestoppt. Die Transformationen wurden wie von Morrison (supra) beschrieben durchgeführt. Die Ligation enthaltend Vektor DNA plus Fragment 3 ergab etwa 50 Kolonien. Die Analyse der Kolonien wurde wie oben beschrieben durchgeführt. Die Plasmid DNA wurde mit BamHI verdaut. Von den Plasmiden die analysiert wurden, enthielten 10 ein BamHI Fragment mit einer Länge von etwa 400 Basenpaaren.

E. Sequenzanalyse des Plasmids pGC3 und Expression des Polypeptids p190-3

Die DNA Sequenzen von 4 Plasmiden wurden wie oben beschrieben bestimmt. Ein Plasmid enthielt das BamHI Fragment in der richtigen Orientierung. Sequenzanalyse bewies, dass das Fragment 3 und das Fragment 1 im gleichen Translationsleseraster fusioniert worden waren. Das Plasmid wurde mit pGC3 bezeichnet. Die Analyse der induzierten Kulturen, unter Verwendung von SDS-PAGE, zeigte, dass Klone, die das pGC3 enthalten, ein Polypeptid von etwa 40'000 Dalton Molekulargewicht produzieren. Für Details siehe Figur 9.

F. Reinigung des Polypeptids p190-3

Bakteriensedimente, enthaltend p190-3 wurden in 0.01 M Hepes (pH 7,8), 0,01 M $MgSO_4$, 0,15 M NaCl, 10% Glyzerin in einer Konzentration von 4 x $10^{10}$ Zellen pro ml resuspendiert. 1 µg/ml DNase 1 und 100 Einheiten pro ml Proteaseinhibitor Trasylol® (Bayer) wurden zugefügt und die Bakterienzellen in einer Frenchpresse bei einem Druck von 20'000 lb/$in^2$ (~1,379 x $10^8$ Pa) aufgebrochen. Das Aufbrechen der Zellen wurde am Kontrastmikroskop überprüft und war grösser als 98%.

Ein rohes Lysat wurde durch differentielle Zentrifugation bei 2'000 rpm (480 x g) während 15 Minuten, 8'000 rpm (8'000 x g) während 15 Minuten, 15'000 rpm (20'000 x g) während 15 Minuten und 42'000 rpm (150'000 x g) während 60 Minuten erhalten. Die Verteilung von p190-3 in den verschiedenen Fraktionen wurde durch Polyacrylamidgelelektrophorese in Gegenwart von SDS (SDS-PAGE) und durch die Immunoblottechnik bestimmt.

Mehr als 80% des p190-3 wurde in dem Sediment der 8'000 rpm- und der 5'000 rpm-Zentrifugation gefunden. Die Sedimente der 2'000 rpm-Zentrifugation enthielten Bakterienbruchstücke, das Sediment der 42'000 rpm-Zentrifugation enthielt Bakterienmembranen und der 42'000 rpm-Ueberstand enthielt bakterielle, cytoplasmatische Proteine. Die Sedimente der 8'000- und der 15'000 rpm-Zentrifugation wurden in 25 mM Imidazol-HCl (pH 7,5) enthaltend 10% Glyzerin, 5 mM EDTA und 5 mM DTT mit einem Magnetrührer resuspendiert. Ein gleiches Volumen 6 M Harnstoff wurde im gleichen Puffer hergestellt und zur Suspension gegeben. Die Suspension wurde bei 15'000 rpm (20'000 x g) während 15 Minuten zentrifugiert. Dieser Extraktionsschritt solubilisierte einige der bakteriellen Verunreinigungen, währenddem das p190-3 in Niederschlag verblieb. Der Niederschlag wurde dann in 9 M Harnstoff, der in 25 mM Imidazol-HCl (pH 7,5), 10% Glyzerin, 5 mM EDTA und 5 mM DTT hergestellt worden war, resuspendiert. Die Lösung wurde bei 42'000 rpm (150'000 x g) während 60 Minuten zentrifugiert und auf eine Chromatofokusiersäule (PBE 94, Pharmacia) die zuvor mit 9 M Harnstoff in 25 mM Imidazol-HCl (pH 7,5) äquilibriert worden war, aufgetragen. Nicht gebundenes Material wurde mit Aequilibrationspuffer aus der Säule ausgewaschen und das p190-3 wurde mit Polypuffer 74-HCl (Pharmacia), pH 4,0, 1:8 verdünnt mit Wasser und enthaltend 9 M Harnstoff, eluiert. Die Verteilung von p190-3 in den Säulenfraktionen wurden durch SDS-PAGE bestimmt. Fraktionen die p190-3 enthielten wurden vereinigt und durch Zugabe von 65% gesättigtem Ammoniumsulfat präzipitiert. Die Mischung wurde während 2 Stunden auf Eis inkubiert und der Niederschlag wurde durch Zentrifugation bei 10'000 rpm (12'000 x g) während 15 Minuten gesammelt. Der Niederschlag wurde in 1

mM Natriumphosphatpuffer (pH 6,8) enthaltend 4 M Guanidinium-HCl solubilisiert. Die Lösung wurde gegen 1 mM Natriumphosphatpuffer, pH 6,8, 4 M Guanidin-HCl während 16 Stunden bei 4°C dialysiert. Das Dialysat wurde bei 42'000 rpm während 60 Minuten zentrifugiert und der Ueberstand wurde auf eine Hydroxylapatitsäule (HA-Ultragel, LKB), die mit 1 mM Natriumphosphatpuffer (pH 6,8) enthaltend 4 M Guanidin-HCl äquilibriert worden war, aufgetragen. Nicht gebundenes Material wurde mit Aequilibrierungspuffer aus der Säule gewaschen und p190-3 wurde mit einem linearen Gradienten von 0-0,4 M Kaliumphosphatpuffer (pH 6,8) enthaltend 4 M Guanidin-HCl, bei einer Durchflussrate von 0,2 ml/Minute, eluiert. Die Verteilung von p190-3 in den Säulenfraktionen wurde durch SDS-PAGE bestimmt. Da Guanidin-HCl ein unlösliches Präzipitat mit SDS bildet wurde zuerst ein Aliquot jeder Fraktion zuerst mit Trichloressigsäure (TCA) präzipitiert (10% Endkonzentration). Die Präzipitate wurden in einer Eppendorfzentrifuge während 3 Minuten zentrifugiert und die Niederschläge wurden frei von TCA gewaschen, indem die Niederschläge in Aceton resuspendiert wurden und erneut zentrifugiert wurden.

Fraktionen der HA-Ultragelsäule, die p190-3 enthielten, wurden vereinigt und mit 65% gesättigtem Ammoniumsulfat, wie oben beschrieben, gefällt. Die präzipitierten Proteine wurden zentrifugiert und der Ueberstand in 0,9%-iger Kochsalzlösung resuspendiert. Da p190-3 im Gegensatz zu Ammoniumsulfat in Kochsalzlösung nicht löslich ist, konnte durch eine zusätzliche Zentrifugation das Ammoniumsulfat im Ueberstand abgetrennt und reines p190-3 aus dem Niederschlag isoliert werden. Ein Immunoblot (Western-Blot, Towbin et al. Proc. Natl. Acad. Sci. U.S.A. 76, 4350-4354 [1979]) zeigte, dass p190-3 nicht nur mit Kaninchenpolyklonalem antiauthentischem p190 Antiserum sondern auch mit monoklonalen Maus anti-p190-1 und anti-p190-2 Antikörpern reagierte.

Das Polypeptid p190-3 kann durch präparative SDS-PAGE weitergereinigt werden (Takacs, Immunol. Methods 1, 81-105 [1979]). Das Resultat einer solchen Reinigung ist in Figur 9 gezeigt. Die analytische SDS-PAGE von gereinigtem p190-3 unter reduzierenden Bedingungen (Probepuffer enthaltend 5% β-Mercaptoäthanol) und nicht reduzierenden Bedingungen (Probenpuffer ohne β-Mercaptoäthanol) zeigte, dass in der Abwesenheit von β-Mercaptoäthanol, p190-3 rasch Dimere, Trimere und Tetramere bildet. Dies könnte die Ursache dafür sein, dass p190-3 in den meisten wässrigen Puffern nicht löslich ist.

Das Kochsalz gewaschene, Ammoniumsulfat präzipitierte 190-3 Sediment das oben beschrieben ist, wurde als Immunogen für die Produktion von Antisera verwendet. Der Niederschlag wurde in Kochsalzlösung mit Hilfe eines Glas-Teflonhomogenisators resuspendiert. Die Suspension wurde mit komplettem Freund's Adjuvans (CFA) vermischt oder an Aluminiumhydroxid adsorbiert, bevor es zur Injektion verwendet wurde.

## Beispiel 5

### Immunogenitätsdaten für p190-1, p190-2b und p190-3

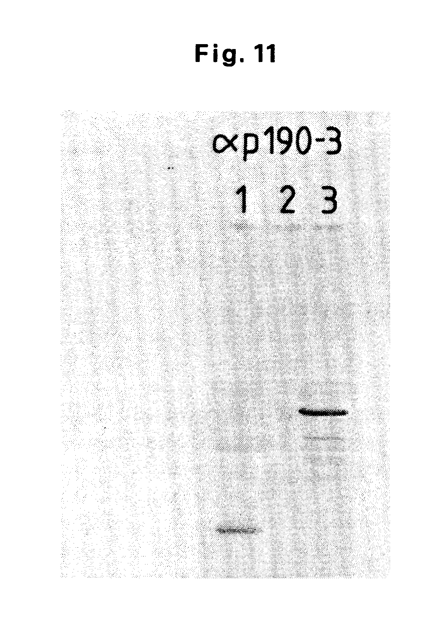

Einzelne Kolonien von E.coli M15, die entweder mit pGC1, pGC2b oder pGC3 transformiert worden sind, wurden in ein Röhrchen, das 10 ml LB-Medium mit 100 µg/ml Ampicillin und 25 µg/ml Kanamycin enthielt, überführt und während 12 Stunden bei 37°C unter heftigem Schütteln wachsen gelassen. Bei einer optischen Dichte von 0,7 bei einer Wellenlänge von 600 nm ($OD_{600}$) wurden die Kulturen mit IPTG (Endkonzentration 1 mM) induziert. Nach weiterer 6-stündiger Inkubation der Zellen wurden diese mittels Zentrifugation gesammelt. Zellen, die aus einem Volumen von 40 µl Kulturmedium isoliert worden waren, wurden in Probenpuffer enthaltend 3% Natriumdodecylsulfat (SDS), 3% β-Mercaptoäthanol, 20% Glyzerin und 125 mM Tris/HCl (pH 6,8) resuspendiert. Die Proben wurden während 5 Minuten gekocht, auf Eis abgekühlt und während 30 Sekunden bei 12'000 x g zentrifugiert. Anschliessend wurden die Proben auf einem SDS-Polyacrylamidgel gemäss Laemmli (supra) während 3 Stunden bei 30 mA elektrophoretisch aufgetrennt (12,5% Acrylamid, Verhältnis Acrylamid/Bisacrylamid 30/0,8). Die Polypeptide wurden vom SDS-Polyacrylamidgel gemäss der Western-Blot-Technik (Towbin et al., supra) auf Nitrocellulosefilterpapier übertragen. Nach dem Transfer wurde der Filter während 10 Minuten in 1 x TBS (15 mM Tris/HCl (pH 7,4), 150 mM NaCl) gewaschen und anschliessend während 30 Minuten in 1 x TBS, 5% fettfreiem Milchpulver inkubiert. Der Filter wurde dann während 2 Stunden mit Kaninchen-anti-p190-Antiserum (1:1000 Verdünnung in 1 x TBS, 5% fettfreiem Milchpulver) inkubiert. Ungebundene Antikörper wurden mittels 5 Waschungen mit 1 x TBS entfernt. Der Filter wurde dann mit dem zweiten Antikörper (Ziege-anti-Kaninchen gekoppelt an Meerrettichperoxidase [Biorad], 1:1000 Verdünnung in 1 x TBS, 5% fettfreier Milch) während einer Stunde inkubiert. Nach dieser Inkubation wurde der Filter fünfmal in 1 x TBS gewaschen. Anschliesslich wurde der Filter in 50 ml 1 x TBS gelegt und 10 ml 4-Chlornaphtol (Sigma, 4 mg/ml in Methanol) zugefügt. Nach Zugabe von 50 µl Wasserstoffperoxid wurden dann die Banden, die mit dem Antiserum reagiert hatten, sichtbar. Wie in Figur 10 Teil A Spuren 1-3 gezeigt, reagierte das Kaninchenserum, das

gegen das natürliche Parasiten p190 Protein erzeugt worden war, spezifisch mit einer einzigen Bande in jeder Spur. Diese Bande wanderte gleichweit wie eine starke Bande auf einem SDS Polyacrylamidgel, das parallel dazu mit den gleichen Proben gemacht worden war und das mit Coomassie brilliant Blau R-250 (Figur 10, Teil C) gefärbt worden war. Die rekombinanten Polypeptide hatten das erwartete Molekulargewicht. In einem zweiten Western-Blot wurden vereinigte, menschliche Malariaseren aus endemischen Gebieten verwendet. Wieder ergaben die gleichen Banden ein starkes Signal, sogar bei einer Verdünnung des Serums auf 1:2000 (Figur 10, Teil B) was darauf hindeutet, dass p190-1 (Spur 1) und p190-3 (Spur 3) ein wichtiges Epitop des 190 kD Vorläufers der vorwiegenden Merozoitenoberflächenantigene von P. falciparum darstellt. Im Gegensatz zu p190-1 und p190-3 reagierte das Polypeptid p190-2b nicht mit menschlichen Malariaseren aus endemischen Gebieten (Fig. 10, Teil B, Spur 2). Dies könnte darauf zurckzuführen sein, dass das Epitop, das durch p190-2b dargestellt wird, sich nahe bei der Membran des p190 Polypeptids befindet und deshalb nicht dem menschlichen Immunsystem ausgesetzt ist. Kaninchenseren gegen natives p190 reagierten mit diesem Epitop. Dies kann darin begründet sein, dass die Tiere mit ungefaltetem p190 immunisiert worden waren und ist nicht darin begründet, dass dieses Epitop auf diesem Polypeptid fehlt.

Die Resultate des Western-Blots wurden durch einen ELISA-Test unter Verwendung von Kaninchen-anti-p190-Serum bestätigt (Tabelle I). Die negativen Kontrollen Rinderserumalbumin (BSA) und ein Lysat eines E.coli der eine grosse Menge eines nicht malariarekombinanten Proteins exprimiert, ergaben O.D. Messungen von 0,01 und 0,02. Die positive Kontrolle, nämlich natives p190 Protein, das aus dem Parasiten isoliert worden war, ergab einen Wert von 0,13. Bakterielle Lysate von E.coli M15(pGC1; pDMI,1) und E.coli M15(pGC2b; pDMI,1) ergaben Werte von 0,15 und 0,13. Diese Werte waren vergleichbar mit den Werten des nativen p190 Proteins (positive Kontrolle), das einen Wert von 0,13 ergab. Daraus lässt sich vermuten, dass die rekombinanten Polypeptide p190-1 und p190-2b einige wichtige Epitope des p190 Proteins tragen.

## Tabelle I

### ELISA-Analyse von p190-1 und p190-2b

| Probe | $O.D.^{450}$ |
|---|---|
| Rinderserumalbumin | 0.01 |
| E. coli M15/pMF-1 | 0.02 |
| natives p190 Protein | 0.13 |
| E. coli M15(pGC1;pDMI,1) | 0.15 |
| E. coli M15(pGC2b;pDMI,1) | 0.13 |

Antigene wurden unter Verwendung eines anti-p190-Kaninchenserums (Verdünnung 1:2000) nachgewiesen. 10 ng BSA und p190 wurden für die Beschichtung der Platten verwendet. Die bakteriellen Lysate enthielten etwa 1 µg Gesamtprotein. Verdünnungskurven wurden für jede Probe gemacht, um die optimale Beschichtungskonzentration zu erhalten.

In einem weiteren Experiment wurden Balb/c Mäuse mit zwei subcutanen Injektionen, im Abstand von einem Monat, mit je etwa 50 µg p190-1 oder 200 µg p190-2b, immunisiert. Die erste Injektion wurde in komplettem Freund's Adjuvans gemacht, die zweite in inkomplettem Freund's Adjuvans. Eine dritte Injektion von 50 µg Antigen in komplettem Freund's Adjuvans wurde einen Monat (p190-1) oder zwei Wochen (p190-2b) nach der zweiten Injektion gegeben. Drei Tage (p190-1) oder vier Tage (p190-2b) später, wurde das Serum genommen und mittels ELISA-Test auf die Reaktion mit den gereinigten Polypeptiden p190-1, p190-2b oder p190-3 überprüft oder mittels indirekter Immunofluoreszenz unter Verwendung von fixierten P. falciparum Blut Stadium Parasiten von verschiedenen Isolaten, untersucht.

Für den ELISA Test, wurden Platten mit Antigen beschichtet (1 µg/ml in 0,14 M NaCl) und mit Rinderserumalbumin (10 mg/ml in 0,1 M Borat, 0,14 M NaCl, pH 8,4) blockiert. Serumproben (10-fache Verdünnungen) wurden für 2 Stunden zugegeben. Die Platte wurde dann mit Borat/Kochsalzpuffer gewaschen und die Antikörperbindung wurde, nach einer Waschung mit alkalischer Phosphatase, die an Kaninchen anti-Maus Ig (Sigma) gebunden ist, gemäss den Instruktionen des Herstellers, bestimmt. Tabelle II zeigt, dass p190-1 und p190-2b in Balb/c Mäusen immunogen sind, dass Antiserum gegen p190-1 nicht signifikant mit p190-2b kreuzreagiert (oder umgekehrt) und dass anti-p190-1 und anti-p190-2b mit p190-3 reagieren.

## Tabelle II

| | Polypeptid | | |
|---|---|---|---|
| **Serum** | **p190-1** | **p190-2b** | **p190-3** |
| Anti p190-1 | $10^4$ (0.28) | $10^2$ (0.22) | $10^6$ (0.37) |
| Anti p190-2b | $10^2$ (0.11) | $>10^6$ (1.6) | $10^6$ (0.25) |

Tabelle II zeigt ELISA Titer von Maus-anti-p190-1 und -anti-p190-2b auf den Polypeptiden p190-1, p190-2b und p190-3. Die Nummern in Zahlen entsprechen der optischen Dichte dieser Titer. Optische Dichten von <0,1 wurden als negativ betrachtet.

Für den Immunofluoreszenztest wurden Tropfenpräparationen von mit P. falciparum infizierten Erythrocyten von asynchronen Kulturen des Parasiten vorbereitet. Die luftgetrockneten Objektträger (12 Tropfen per Objektträger) wurden bei -20 °C gelagert. Unmittelbar vor Gebrauch wurden die Objektträger mit Aceton fixiert. Die Parasiten wurden dann während 20 Minuten bei 37 °C mit den Testseren behandelt. Die Objektträger wurden in PBS gewaschen, Fluorescein konjugiertes Ziegen-anti-Maus-Ig-Antiserum wurde zugegeben und die Proben wurden während weiteren 20 Minuten inkubiert. Die Objektträger wurden dann gewaschen, mit 50% Glyzerin in PBS überschichtet und nach Zugabe eines Deckglases unter dem UV-Licht untersucht. Die folgenden P. falciparum Isolate wurden geprüft: K1, MAD-20, FCH-5, Ro-33, Ro-58. Alle Isolate färbten sich positiv mit Seren von Mäusen welche entweder mit p190-1 oder p190-2b immunisiert worden waren (Serumverdünnung 1:100).

**Patentansprüche**
**Patentansprüche für folgende Vertragsstaaten : AT, BE, CH, DE, FR, GB, IT, LI, NL, SE**

**1.** Ein Polypeptid der Formel

A-B-C (I)

worin
A ein Affinitätspeptidrest ist oder fehlen kann,
B

ThrLeuCysAspAsnIleHisGlyPheLysTyrLeuIleAspGlyTyrGluGluIle
AsnGluLeuLeuTyrLysLeuAsnPheTyrPheAspLeuLeuArgAlaLysLeuAsn
AsnValCysAlaAsnAspTyrCysGlnIleProPheAsnLeuLysIleArgAlaAsn
GluLeuAspValLeuLysLysLeuValPheGlyTyrArgLysProLeuAspAsnIle
LysAspAsnValGlyLysMetGluAspTyrIleLysLysAsnLysLysThrIleGlu
AsnIleAsnGluLeuIleGluGluSerLysLysThrIleAspLysAsnLysAsnAla
ThrLysGluGluGluLysLysLysLeuTyrGlnAlaGlnTyrAspLeuPheIleTyr
AsnLysGlnLeuGluGluAlaHisAsnLeuIleSerValLeuGluLysArgIleAsp
ThrLeuLysLysAsnGluAsnIleLysGluLeuLeuAspLysIleAsnGluIleLys
AsnProProPro

oder ein Fragment davon, oder

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

oder ein Fragment davon ist, oder eine Kombination dieser Sequenzen oder Fragmente, vorausgesetzt, dass die obengenannten Fragmente mindestens ein Epitop des 190kD Vorläufers der vorwiegenden Merozoiten Oberflächenantigene von P. falciparum darstellen oder enthalten, und

C ein Peptidrest ist oder fehlen kann, dadurch gekennnzeichnet, dass das Polypeptid fähig ist eine Immunantwort gegen verschiedene Isolate von P. falciparum hervorzurufen.

**2.** Ein Polypeptid der Formel

A-B-C (I)

worin

A ein Affinitätspeptidrest ist oder fehlen kann,

B

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

oder ein Fragment davon ist, wobei dieses Fragment mindestens ein Epitop des 190kD Vorläufers der vorwiegenden Merozoiten Oberflächenantigene von P. falciparum darstellt oder enthält, und

C ein Peptidrest ist oder fehlen kann, dadurch gekennnzeichnet, dass das Polypeptid fähig ist eine Immunantwort gegen verschiedene Isolate von P. falciparum hervorzurufen.

**3.** Ein Polypeptid gemäss Anspruch 1 oder 2, dadurch gekennzeichnet, dass der Affinitätspeptidrest A zwei oder mehrere benachbarte Histidinreste enthält.

**4.** Ein Polypeptid gemäss Anspruch 3 , dadurch gekennzeichnet, dass der Affinitätspeptidrest A
MetHisHisAlaProGlySerGly,
MetHisHisAlaProGlySer, oder
MetHisHisAlaPeoGlySer, oder MetHisHisAlaPro ist.

**5.** Ein Polypeptid gemäss Anspruch 1 oder Anspruch 2, dadurch gekennzeichnet, dass der Peptidrest C

AlaGlyGlyLeuLeuLeuIleAspProValMetThrSerGluLeuHisLeuAsp-
LeuPheArgThrLeuGlyCysArgArgAlaPhePheIleGlyGluAsnProSer,

oder
ValAspLeuGlnProSerLeuAspSerCys ist.

**6.** Ein Polypeptid mit der Aminosäuresequenz

MetHisHisAlaProGlySerGlyThrLeuCysAspAsnIleHisGlyPheLysTyrLeu
IleAspGlyTyrGluGluIleAsnGluLeuLeuTyrLysLeuAsnPheTyrPheAspLeu
LeuArgAlaLysLeuAsnAsnValCysAlaAsnAspTyrCysGlnIleProPheAsnLeu
LysIleArgAlaAsnGluLeuAspValLeuLysLysLeuValPheGlyTyrArgLysPro
LeuAspAsnIleLysAspAsnValGlyLysMetGluAspTyrIleLysLysAsnLysLys
ThrIleGluAsnIleAsnGluLeuIleGluGluSerLysLysThrIleAspLysAsnLys
AsnAlaThrLysGluGluGluLysLysLysLeuTyrGlnAlaGlnTyrAspLeuPheIle
TyrAsnLysGlnLeuGluGluAlaHisAsnLeuIleSerValLeuGluLysArgIleAsp
ThrLeuLysLysAsnGluAsnIleLysGluLeuLeuAspLysIleAsnGluIleLysAsn
ProProProAlaGlyGlyLeuLeuLeuIleAspProValMetThrSerGluLeuHisLeu
AspLeuPheArgThrLeuGlyCysArgArgAlaPhePheIleGlyGluAsnProSer.

**7.** Ein Polypeptid mit der Aminosäuresequenz

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys
LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerValAspLeuGlnProSerLeuAspSerCys.

**8.** Ein Polypeptid mit der Aminosäuresequenz

MetHisHisAlaProAlaGluIleAlaGluThrGluAsnThrLeuGluAsnThrLysIle LeuLeuLysHisTyrLysGlyLeuValLysTyrTyrAsnGlyGluSerSerProLeuLys ThrLeuSerGluGluSerIleGlnThrGluAspAsnTyrAlaSerLeuGluAsnPheLys ValLeuSerLysLeuGluGlyLysLeuLysAspAsnLeuAsnLeuGluLysLysLysLeu SerTyrLeuSerArgGlyLeuHisHisLeuIleAlaGluLeuLysGluValIleLysAsn LysAsnTyrThrGlyAsnSerProSerValAsnAsnThrAspValAsnAsnAlaLeuGlu SerTyrLysLysPheLeuProGluGlyThrAspValAlaThrValValSerGluSerGly SerValAspLeuGlnProSerLeuAspSerCys.

**9.** Ein Polypeptid mit der Aminosäuresequenz

MetHisHisAlaProGlySerAlaGluIleAlaGluThrGluAsnThrLeuGluAsnThr LysIleLeuLeuLysHisTyrLysGlyLeuValLysTyrTyrAsnGlyGluSerSerPro LeuLysThrLeuSerGluGluSerIleGlnThrGluAspAsnTyrAlaSerLeuGluAsn PheLysValLeuSerLysLeuGluGlyLysLeuLysAspAsnLeuAsnLeuGluLysLys LysLeuSerTyrLeuSerArgGlyLeuHisHisLeuIleAlaGluLeuLysGluValIle LysAsnLysAsnTyrThrGlyAsnSerProSerValAsnAsnThrAspValAsnAsnAla LeuGluSerTyrLysLysPheLeuProGluGlyThrAspValAlaThrValValSerGlu SerGlySerGlyThrLeuCysAspAsnIleHisGlyPheLysTyrLeuIleAspGlyTyr GluGluIleAsnGluLeuLeuTyrLysLeuAsnPheTyrPheAspLeuLeuArgAlaLys LeuAsnAsnValCysAlaAsnAspTyrCysGlnIleProPheAsnLeuLysIleArgAla AsnGluLeuAspValLeuLysLysLeuValPheGlyTyrArgLysProLeuAspAsnIle LysAspAsnValGlyLysMetGluAspTyrIleLysLysAsnLysLysThrIleGluAsn IleAsnGluLeuIleGluGluSerLysLysThrIleAspLysAsnLysAsnAlaThrLys GluGluGluLysLysLysLeuTyrGlnAlaGlnTyrAspLeuPheIleTyrAsnLysGln LeuGluGluAlaHisAsnLeuIleSerValLeuGluLysArgIleAspThrLeuLysLys AsnGluAsnIleLysGluLeuLeuAspLysIleAsnGluIleLysAsnProProProAla GlyGlyLeuLeuLeuIleAspProValMetThrSerGluLeuHisLeuAspLeuPheArg ThrLeuGlyCysArgArgAlaPhePheIleGlyGluAsnProSer.

**10.** Ein Polypeptid gemäss einem der Ansprüche 1-9, das an ein Trägermaterial adsorbiert oder kovalent daran gebunden ist.

**11.** Ein DNA Fragment, das für ein Polypeptid gemäss einem der Ansprüche 1 bis 9 kodiert.

**12.** Ein replizierbarer, mikrobieller Vektor, der ein DNA Fragment gemäss Anspruch 11 enthält.

**13.** Ein replizierbarer Vektor gemäss Anspruch 12, dadurch gekennzeichnet, dass das DNA Fragment operativ mit einer Expressionskontrollsequenz verbunden ist.

**14.** Ein Mikroorganismus, der mit einem Vektor gemäss Anspruch 12 oder 13 transformiert ist.

**15.** Ein Polypeptid gemäss einem der Ansprüche 1 bis 10 zur Immunisierung von Säugern gegen Malaria.

**16.** Ein Verfahren zur Herstellung von Polypeptiden gemäss einem der Ansprüche 1 bis 9, dadurch gekennzeichnet, dass Mikroorganismen, die mit einem Vektor gemäss Anspruch 12 oder 13 transformiert sind, unter Bedingungen kultiviert werden, die die Expression des kodierten Polypeptids erlauben.

**17.** Ein Verfahren zur Herstellung von Mikroorganismen gemäss Anspruch 14, dadurch gekennzeichnet, dass ein Mikroorganismus in an sich bekannter Art und Weise mit einem Vektor gemäss Anspruch 12 oder 13 transformiert wird.

**18.** Ein Verfahren zur Herstellung von Antikörpern, die gegen ein konserviertes Epitop des 190kD Vorläufers der vorwiegenden Merozoiten Oberflächenantigene von P. falciparum gerichtet sind, dadurch gekennzeichnet, dass ein Polypeptid gemäss einem der Ansprüche 1 bis 10 in einen geeigneten Wirsorganismus, der fähig ist eine Immunantwort gegen das Polypeptid auszulösen, injiziert wird und die gebildeten Antikörper nach bekannten Methoden isoliert werden.

**19.** Eine immunogene Komposition, die fähig ist, in einem Wirt Antikörper hervorzurufen, die spezifisch sind für p190, dem Vorläufer der vorwiegenden Merozoiten Oberflächenantigene von P. falciparum, wobei diese immunogenen Kompositionen ein Polypeptid gemäss einem der Ansprüche 1-10 sowie ein pharmazeutisch akzeptables Adjuvans enthalten.

**20.** Eine Komposition gemäss Anspruch 19 als ein Vakzin.

**21.** Verwendung eines Polypeptids gemäss einem der Ansprüche 1 bis 9, zur Herstellung einer immunogenen Komposition zur Immunisierung von Säugern gegen Malaria.

**Patentansprüche für folgenden Vertragsstaat : ES**

**1.** Ein Verfahren zur Herstellung von Polypeptiden der Formel

A-B-C (I)

worin
A ein Affinitätspeptidrest ist oder fehlen kann,
B

ThrLeuCysAspAsnIleHisGlyPheLysTyrLeuIleAspGlyTyrGluGluIle
AsnGluLeuLeuTyrLysLeuAsnPheTyrPheAspLeuLeuArgAlaLysLeuAsn
AsnValCysAlaAsnAspTyrCysGlnIleProPheAsnLeuLysIleArgAlaAsn
GluLeuAspValLeuLysLysLeuValPheGlyTyrArgLysProLeuAspAsnIle
LysAspAsnValGlyLysMetGluAspTyrIleLysLysAsnLysLysThrIleGlu
AsnIleAsnGluLeuIleGluGluSerLysLysThrIleAspLysAsnLysAsnAla
ThrLysGluGluGluLysLysLysLeuTyrGlnAlaGlnTyrAspLeuPheIleTyr
AsnLysGlnLeuGluGluAlaHisAsnLeuIleSerValLeuGluLysArgIleAsp
ThrLeuLysLysAsnGluAsnIleLysGluLeuLeuAspLysIleAsnGluIleLys
AsnProProPro

oder ein Fragment davon, oder

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

oder ein Fragment davon ist, oder eine Kombination dieser Sequenzen oder Fragmente, vorausgesetzt, dass die obengenannten Fragmente mindestens ein Epitop des 190kD Vorläufers der vorwiegenden Merozoiten Oberflächenantigene von P. falciparum darstellen oder enthalten, und

C ein Peptidrest ist oder fehlen kann,

wobei das Polypeptid fähig ist eine Immunantwort gegen verschiedene Isolate von P. falciparum hervorzurufen, dadurch gekennzeichnet, dass Mikroorganismen, die mit einem Vektor, der für dieses Polypeptid kodiert, transformiert sind, unter Bedingungen kultiviert werden, die die Expression des kodierten Polypeptids erlauben und das Polypeptid isoliert wird.

2. Ein Verfahren zur Herstellung von Polypeptiden der Formel

A-B-C (I)

worin

A ein Affinitätspeptidrest ist oder fehlen kann,

B

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

oder ein Fragment davon ist, wobei dieses Fragment mindestens ein Epitop des 190kD Vorläufers der vorwiegenden Merozoiten Oberflächenantigene von P. falciparum darstellt oder enthält, und

C ein Peptidrest ist oder fehlen kann,

wobei das Polypeptid fähig ist eine Immunantwort gegen verschiedene Isolate von P. falciparum hervorzurufen, dadurch gekennzeichnet, dass Mikroorganismen, die mit einem Vektor, der für dieses Polypeptid kodiert, transformiert sind, unter Bedingungen kultiviert werden, die die Expression des kodierten Polypeptids erlauben und das Polypeptid isoliert wird.

3. Ein Verfahren gemäss Anspruch 1 oder 2, dadurch gekennzeichnet, dass der Affinitätspeptidrest A zwei oder mehrere benachbarte Histidinreste enthält.

4. Ein Verfahren gemäss Anspruch 3 , dadurch gekennzeichnet, dass der Affinitätspeptidrest A
MetHisHisAlaProGlySerGly,
MetHisHisAlaProGlySer, oder
MetHisHisAlaPro ist.

5. Ein Verfahren gemäss Anspruch 1 oder 2, dadurch gekennzeichnet, dass der Peptidrest C

AlaGlyGlyLeuLeuLeuIleAspProValMetThrSerGluLeuHisLeuAsp-
LeuPheArgThrLeuGlyCysArgArgAlaPhePheIleGlyGluAsnProSer,

oder
ValAspLeuGlnProSerLeuAspSerCys ist.

6. Ein Verfahren zur Herstellung eines Polypeptids mit der Aminosäuresequenz

MetHisHisAlaProGlySerGlyThrLeuCysAspAsnIleHisGlyPheLysTyrLeu
IleAspGlyTyrGluGluIleAsnGluLeuLeuTyrLysLeuAsnPheTyrPheAspLeu
LeuArgAlaLysLeuAsnAsnValCysAlaAsnAspTyrCysGlnIleProPheAsnLeu
LysIleArgAlaAsnGluLeuAspValLeuLysLysLeuValPheGlyTyrArgLysPro
LeuAspAsnIleLysAspAsnValGlyLysMetGluAspTyrIleLysLysAsnLysLys
ThrIleGluAsnIleAsnGluLeuIleGluGluSerLysLysThrIleAspLysAsnLys
AsnAlaThrLysGluGluGluLysLysLysLeuTyrGlnAlaGlnTyrAspLeuPheIle
TyrAsnLysGlnLeuGluGluAlaHisAsnLeuIleSerValLeuGluLysArgIleAsp
ThrLeuLysLysAsnGluAsnIleLysGluLeuLeuAspLysIleAsnGluIleLysAsn
ProProProAlaGlyGlyLeuLeuLeuIleAspProValMetThrSerGluLeuHisLeu
AspLeuPheArgThrLeuGlyCysArgArgAlaPhePheIleGlyGluAsnProSer,

dadurch gekennzeichnet, dass Mikroorganismen, die mit einem Vektor, der für dieses Polypeptid kodiert, transformiert sind, unter Bedingungen kultiviert werden, die die Expression des kodierten Polypeptids erlauben und das Polypeptid isoliert wird.

7. Ein Verfahren zur Herstellung eines Polypeptids mit der Aminosäuresequenz

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys
LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerValAspLeuGlnProSerLeuAspSerCys

dadurch gekennzeichnet, dass Mikroorganismen, die mit einem Vektor, der für dieses Polypeptid kodiert, transformiert sind, unter Bedingungen kultiviert werden, die die Expression des kodierten Polypeptids erlauben und das Polypeptid isoliert wird.

**8.** Ein Verfahren zur Herstellung eines Polypeptids mit der Aminosäuresequenz

MetHisHisAlaProAlaGluIleAlaGluThrGluAsnThrLeuGluAsnThrLysIle
LeuLeuLysHisTyrLysGlyLeuValLysTyrTyrAsnGlyGluSerSerProLeuLys
ThrLeuSerGluGluSerIleGlnThrGluAspAsnTyrAlaSerLeuGluAsnPheLys
ValLeuSerLysLeuGluGlyLysLeuLysAspAsnLeuAsnLeuGluLysLysLysLeu
SerTyrLeuSerArgGlyLeuHisHisLeuIleAlaGluLeuLysGluValIleLysAsn
LysAsnTyrThrGlyAsnSerProSerValAsnAsnThrAspValAsnAsnAlaLeuGlu
SerTyrLysLysPheLeuProGluGlyThrAspValAlaThrValValSerGluSerGly
SerValAspLeuGlnProSerLeuAspSerCys

dadurch gekennzeichnet, dass Mikroorganismen, die mit einem Vektor, der für dieses Polypeptid kodiert, transformiert sind, unter Bedingungen kultiviert werden, die die Expression des kodierten Polypeptids erlauben und das Polypeptid isoliert wird.

**9.** Ein Verfahren zur Herstellung eines Polypeptids mit der Aminosäuresequenz

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys

LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerGlyThrLeuCysAspAsnIleHisGlyPheLysTyrLeuIleAspGlyTyr
GluGluIleAsnGluLeuLeuTyrLysLeuAsnPheTyrPheAspLeuLeuArgAlaLys
LeuAsnAsnValCysAlaAsnAspTyrCysGlnIleProPheAsnLeuLysIleArgAla
AsnGluLeuAspValLeuLysLysLeuValPheGlyTyrArgLysProLeuAspAsnIle
LysAspAsnValGlyLysMetGluAspTyrIleLysLysAsnLysLysThrIleGluAsn
IleAsnGluLeuIleGluGluSerLysLysThrIleAspLysAsnLysAsnAlaThrLys
GluGluGluLysLysLysLeuTyrGlnAlaGlnTyrAspLeuPheIleTyrAsnLysGln
LeuGluGluAlaHisAsnLeuIleSerValLeuGluLysArgIleAspThrLeuLysLys
AsnGluAsnIleLysGluLeuLeuAspLysIleAsnGluIleLysAsnProProProAla
GlyGlyLeuLeuLeuIleAspProValMetThrSerGluLeuHisLeuAspLeuPheArg
ThrLeuGlyCysArgArgAlaPhePheIleGlyGluAsnProSer

dadurch gekennzeichnet, dass Mikroorganismen, die mit einem Vektor, der für dieses Polypeptid kodiert, transformiert sind, unter Bedingungen kultiviert werden, die die Expression des kodierten Polypeptids erlauben und das Polypeptid isoliert wird.

**10.** Ein Verfahren gemäss einem der Ansprüche 1-9, dadurch gekennzeichnet, dass das Polypeptid zusätzlich an ein Trägermaterial adsorbiert oder kovalent daran gebunden wird.

**11.** Ein Verfahren zur Herstellung eines replizierbaren, mikrobiellen Vektors, der für ein Polypeptid, wie es in den Ansprüchen 1 bis 9 definiert ist, kodiert, dadurch gekennzeichnet, dass ein DNA Fragment, das für ein solches Polypeptid kodiert, in einen replizierbaren, mikrobiellen Vektor integriert wird.

**12.** Ein Verfahren gemäss Anspruch 11, dadurch gekennzeichnet, dass das DNA Fragment, das für ein Polypeptid, wie es in den Ansprüchen 1 bis 9 definiert ist, kodiert, operativ mit einer Expressionskontrollsequenz verbunden wird.

**13.** Ein Verfahren zur Herstellung eines transformierten Mikroorganismus, der fähig ist ein Polypeptid, wie es in den Ansprüchen 1 bis 9 definiert ist, zu produzieren, dadurch gekennzeichnet, dass ein Mikroorganismus in an sich bekannter Art und Weise mit einem Vektor, wie er in Anspruch 11 oder 12 definiert ist, transformiert wird.

**14.** Ein Verfahren zur Herstellung von Antikörpern, die gegen ein konserviertes Epitop des 190kD Vorläufers der vorwiegenden Merozoiten Oberflächenantigene von P. falciparum gerichtet sind, dadurch gekennzeichnet, dass ein Polypeptid, wie es in einem der Ansprüche 1 bis 10 definiert ist, in einen geeigneten Wirsorganismus, der fähig ist eine Immunantwort gegen das Polypeptid auszulösen, injiziert wird und die Antikörper nach bekannten Methoden isoliert werden.

**15.** Ein Verfahren zur Herstellung einer immunogene Komposition die fähig ist, in einem Wirt Antikörper, die spezifisch sind für p190, dem Vorläufer der vorwiegenden Merozoiten Oberflächenantigene von P. falciparum, hervorzurufen, dadurch gekennzeichnet, dass ein Polypeptid, wie es in einem der Ansprüche 1 bis 10 definiert ist, mit einem pharmazeutisch akzeptablen Adjuvans vermischt wird.

**16.** Verwendung eines Polypeptids gemäss einem der Ansprüche 1 bis 9, zur Herstellung einer immunogenen Komposition zur Immunisierung von Säugern gegen Malaria.

**Claims**
**Claims for the following Contracting Stages : AT, BE, CH, DE, FR, GB, IT, LI, NL, SE**

**1.** A polypeptide of the formula

A-B-C (I)

wherein
A is an affinity peptide residue or may be absent,
B is

**ThrLeuCysAspAsnIleHisGlyPheLysTyrLeuIleAspGlyTyrGlu**
**GluIleAsnGluLeuLeuTyrLysLeuAsnPheTyrPheAspLeuLeuArg**
**AlaLysLeuAsnAsnValCysAlaAsnAspTyrCysGlnIleProPheAsn**
**LeuLysIleArgAlaAsnGluLeuAspValLeuLysLysLeuValPheGly**
**TyrArgLysProLeuAspAsnIleLysAspAsnValGlyLysMetGluAsp**
**TyrIleLysLysAsnLysLysThrIleGluAsnIleAsnGluLeuIleGlu**
**GluSerLysLysThrIleAspLysAsnLysAsnAlaThrLysGluGluGlu**
**LysLysLysLeuTyrGlnAlaGlnTyrAspLeuPheIleTyrAsnLysGln**
**LeuGluGluAlaHisAsnLeuIleSerValLeuGluLysArgIleAspThr**
**LeuLysLysAsnGluAsnIleLysGluLeuLeuAspLysIleAsnGluIle**
**LysAsnProProPro**

or a fragment thereof, or

AlaGluIleAlaGluThrGluAsnThrLeuGluAsnThrLysIleLeuLeu
LysHisTyrLysGlyLeuValLysTyrTyrAsnGlyGluSerSerProLeu
LysThrLeuSerGluGluSerIleGlnThrGluAspAsnTyrAlaSerLeu
GluAsnPheLysValLeuSerLysLeuGluGlyLysLeuLysAspAsnLeu
AsnLeuGluLysLysLysLeuSerTyrLeuSerArgGlyLeuHisHisLeu
IleAlaGluLeuLysGluValIleLysAsnLysAsnTyrThrGlyAsnSer
ProSerValAsnAsnThrAspValAsnAsnAlaLeuGluSerTyrLysLys
PheLeuProGluGlyThrAspValAlaThrValValSerGluSerGlySer

or a fragment thereof, or a combination of these sequences or fragments, provided that the fragments mentioned above represent or contain at least one epitope of the 190 kD precursor to the major merozoite surface antigens of P. falciparum, and

C is a peptide residue or may be absent, characterized in that the polypeptide is capable of eliciting an immune response aganist different isolates of P. falciparum.

**2.** A polypeptide of the formula

A-B-C (1)

wherein
A is an affinity peptide residue or may be absent,
B is

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

or a fragment thereof, this fragment representing or containing at least one epitope of the 190 kD precursor to the major merozoite surface antigens of P. falciparum, and

C is a peptide residue or may be absent, characterized in that the polypeptide is capable of eliciting an imune response against different isolates of P. falciparum.

**3.** A polypeptide according to claim 1 or 2, characterized in that the affinity peptide residue A contains two or more adjacent histidine residues.

**4.** A polypeptide according to claim 3, characterized in that the affinity peptide residue A is
MetHisHisAlaProGlySerGly,
MetHisHisAlaProGlySer or
MetHisHisAlaPro.

**5.** A polypeptide according to claim 1 or claim 2, characterized in that the peptide residue C is

AlaGlyGlyLeuLeuLeuIleAspProValMetThrSerGluLeuHisLeuAsp-LeuPheArgThrLeuGlyCysArgArgAlaPhePheIleGlyGluAsnProSer,

or

ValAspLeuGlnProSerLeuAspSerCys

**6.** A polypeptide having the ammino acid sequence

MetHisHisAlaProGlySerGlyThrLeuCysAspAsnIleHisGlyPheLysTyrLeu
IleAspGlyTyrGluGluIleAsnGluLeuLeuTyrLysLeuAsnPheTyrPheAspLeu
LeuArgAlaLysLeuAsnAsnValCysAlaAsnAspTyrCysGlnIleProPheAsnLeu
LysIleArgAlaAsnGluLeuAspValLeuLysLysLeuValPheGlyTyrArgLysPro
LeuAspAsnIleLysAspAsnValGlyLysMetGluAspTyrIleLysLysAsnLysLys
ThrIleGluAsnIleAsnGluLeuIleGluGluSerLysLysThrIleAspLysAsnLys
AsnAlaThrLysGluGluGluLysLysLysLeuTyrGlnAlaGlnTyrAspLeuPheIle
TyrAsnLysGlnLeuGluGluAlaHisAsnLeuIleSerValLeuGluLysArgIleAsp
ThrLeuLysLysAsnGluAsnIleLysGluLeuLeuAspLysIleAsnGluIleLysAsn
ProProProAlaGlyGlyLeuLeuLeuIleAspProValMetThrSerGluLeuHisLeu
AspLeuPheArgThrLeuGlyCysArgArgAlaPhePheIleGlyGluAsnProSer.

**7.** A polypeptide having the amino acid sequence

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys
LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerValAspLeuGlnProSerLeuAspSerCys.

**8.** A polypeptide having the amino acid sequence

MetHisHisAlaProAlaGluIleAlaGluThrGluAsnThrLeuGluAsnThrLysIle
LeuLeuLysHisTyrLysGlyLeuValLysTyrTyrAsnGlyGluSerSerProLeuLys
ThrLeuSerGluGluSerIleGlnThrGluAspAsnTyrAlaSerLeuGluAsnPheLys
ValLeuSerLysLeuGluGlyLysLeuLysAspAsnLeuAsnLeuGluLysLysLysLeu
SerTyrLeuSerArgGlyLeuHisHisLeuIleAlaGluLeuLysGluValIleLysAsn
LysAsnTyrThrGlyAsnSerProSerValAsnAsnThrAspValAsnAsnAlaLeuGlu
SerTyrLysLysPheLeuProGluGlyThrAspValAlaThrValValSerGluSerGly
SerValAspLeuGlnProSerLeuAspSerCys.

**9.** A polypeptide having the amino acid sequence

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys
LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerGlyThrLeuCysAspAsnIleHisGlyPheLysTyrLeuIleAspGlyTyr
GluGluIleAsnGluLeuLeuTyrLysLeuAsnPheTyrPheAspLeuLeuArgAlaLys
LeuAsnAsnValCysAlaAsnAspTyrCysGlnIleProPheAsnLeuLysIleArgAla
AsnGluLeuAspValLeuLysLysLeuValPheGlyTyrArgLysProLeuAspAsnIle
LysAspAsnValGlyLysMetGluAspTyrIleLysLysAsnLysLysThrIleGluAsn
IleAsnGluLeuIleGluGluSerLysLysThrIleAspLysAsnLysAsnAlaThrLys
GluGluGluLysLysLysLeuTyrGlnAlaGlnTyrAspLeuPheIleTyrAsnLysGln
LeuGluGluAlaHisAsnLeuIleSerValLeuGluLysArgIleAspThrLeuLysLys
AsnGluAsnIleLysGluLeuLeuAspLysIleAsnGluIleLysAsnProProProAla
GlyGlyLeuLeuLeuIleAspProValMetThrSerGluLeuHisLeuAspLeuPheArg
ThrLeuGlyCysArgArgAlaPhePheIleGlyGluAsnProSer.

**10.** A polypeptide according to any one of claims 1-9 which is adsorbed on a carrier material or covalently attached thereto.

**11.** A DNA fragment coding for a polypeptide according to any one of claims 1 to 9.

**12.** A replicable microbial vector containing a DNA fragment according to claim 11.

**13.** A replicable microbial vector according to claim 12, characterized in that the DNA fragment is operably linked to an expression control sequence.

**14.** A microorganism transformed with a vector according to claim 12 or 13.

**15.** A polypeptide according to any one of claims 1 to 10 for the immunization of mammals against malaria.

**16.** A process for the production of a polypeptide according to any one of claims 1-9, characterized by culturing microorganisms transformed with a vector according to claim 12 or 13 under conditions permitting the expression of the encoded polypeptide.

**17.** A process for the production of microorganisms according to claim 14, characterized by transforming a microorganism in a manner known per se with a vector according to claims 12 or 13.

**18.** A process for the production of antibodies against a conserved epitope of the 190 kD precursor of the major merozoite surface antigens of P. falciparum, characterized by injection a polypeptide according to any one of claims 1 to 10 into a suitable host capable of eliciting an immune response against the polypeptide and isolating the antibody produced according to known methods.

**19.** An immunogenic composition capable of eliciting antibodies specific for p190, the precursor to the major merozoite surface antigens of P. falciparum, in a host, these immunogenic compositions containing a polypeptide in accordance with any one of claims 1-10 and a pharmaceutically acceptable adjuvant.

**20.** A composition according to claim 19 as a vaccine.

**21.** The use of a polypeptide according to any one of claims 1 to 9 for the production of an immunogenic composition for the immunization of mammals against malaria.

**Claims for the following Contracting State : ES**

**1.** A process for the preparation of polypeptides of the formula

A-B-C (I)

wherein
A is an affinity peptide residue or may be absent,
B

**ThrLeuCysAspAsnIleHisGlyPheLysTyrLeuIleAspGlyTyrGluGluIle**
**AsnGluLeuLeuTyrLysLeuAsnPheTyrPheAspLeuLeuArgAlaLysLeuAsn**
**AsnValCysAlaAsnAspTyrCysGlnIleProPheAsnLeuLysIleArgAlaAsn**
**GluLeuAspValLeuLysLysLeuValPheGlyTyrArgLysProLeuAspAsnIle**
**LysAspAsnValGlyLysMetGluAspTyrIleLysLysAsnLysLysThrIleGlu**
**AsnIleAsnGluLeuIleGluGluSerLysLysThrIleAspLysAsnLysAsnAla**
**ThrLysGluGluGluLysLysLysLeuTyrGlnAlaGlnTyrAspLeuPheIleTyr**
**AsnLysGlnLeuGluGluAlaHisAsnLeuIleSerValLeuGluLysArgIleAsp**
**ThrLeuLysLysAsnGluAsnIleLysGluLeuLeuAspLysIleAsnGluIleLys**
**AsnProProPro**

or a fragment thereof, or

**AlaGluIleAlaGluThrGluAsnThrLeuGluAsnThrLysIleLeuLeuLysHis TyrLysGlyLeuValLysTyrTyrAsnGlyGluSerSerProLeuLysThrLeuSer GluGluSerIleGlnThrGluAspAsnTyrAlaSerLeuGluAsnPheLysValLeu SerLysLeuGluGlyLysLeuLysAspAsnLeuAsnLeuGluLysLysLysLeuSer TyrLeuSerArgGlyLeuHisHisLeuIleAlaGluLeuLysGluValIleLysAsn LysAsnTyrThrGlyAsnSerProSerValAsnAsnThrAspValAsnAsnAlaLeu GluSerTyrLysLysPheLeuProGluGlyThrAspValAlaThrValValSerGlu SerGlySer**

or a fragment thereof, or a combination of these sequences or fragments, provided that the fragments mentioned above represent or contain at least one epitope of the 190 kD precursor to the major merozoite surface antigens of P. falciparum, and

C is a peptide residue or may be absent,

whereby the polypeptide is capable of eliciting an immune response against different isolates of P. falciparum, characterized by culturing microorganism transformed with a vector encoding this polypeptide under conditions permitting the expression of the encoded polypeptide and isolating the polypeptide.

2. A process for the preparation of polypeptides of the formula

A-B-C (I)

wherein

A is an affinity peptide residue or may be absent,

B is

**AlaGluIleAlaGluThrGluAsnThrLeuGluAsnThrLysIleLeuLeuLysHis TyrLysGlyLeuValLysTyrTyrAsnGlyGluSerSerProLeuLysThrLeuSer GluGluSerIleGlnThrGluAspAsnTyrAlaSerLeuGluAsnPheLysValLeu SerLysLeuGluGlyLysLeuLysAspAsnLeuAsnLeuGluLysLysLysLeuSer TyrLeuSerArgGlyLeuHisHisLeuIleAlaGluLeuLysGluValIleLysAsn LysAsnTyrThrGlyAsnSerProSerValAsnAsnThrAspValAsnAsnAlaLeu GluSerTyrLysLysPheLeuProGluGlyThrAspValAlaThrValValSerGlu SerGlySer**

or a fragment thereof, this fragment representing or containing at least one epitope of the 190 kD precursor to the major merozoite surface antigens of P. falciparum, and

C is a peptide residue or may be absent,

whereby the polypeptide is capable of eliciting an immune response against different isolates of P. falciparum, characterized by culturing microorganism transformed with a vector encoding this polypeptide under conditions permitting the expression of the encoded polypeptide and isolating the polypeptide.

3. A process according to claim 1 or 2, characterized in that the affinity peptide residue A contains two or more neighbouring histidine residues.

4. A process according to claim 3, characterized in that the affinity peptide residue A is MetHisHisAlaProGlySerGly,

MetHisHisAlaProGlySer, or
MetHisHisAlaPro.

**5.** A process according to claim 1 or 2, characterized in that the peptide residue C is

AlaGlyGlyLeuLeuLeuIleAspProValMetThrSerGluLeuHisLeuAsp-LeuPheArgThrLeuGlyCysArgAlaPhePheIleGlyGluAsnProSer,

or
ValAspLeuGlnProSerLeuAspSerCys.

**6.** A process for the preparation of a polypeptide having the amino acid sequence

MetHisHisAlaProGlySerGlyThrLeuCysAspAsnIleHisGlyPheLysTyrLeu
IleAspGlyTyrGluGluIleAsnGluLeuLeuTyrLysLeuAsnPheTyrPheAspLeu
LeuArgAlaLysLeuAsnAsnValCysAlaAsnAspTyrCysGlnIleProPheAsnLeu
LysIleArgAlaAsnGluLeuAspValLeuLysLysLeuValPheGlyTyrArgLysPro
LeuAspAsnIleLysAspAsnValGlyLysMetGluAspTyrIleLysLysAsnLysLys
ThrIleGluAsnIleAsnGluLeuIleGluGluSerLysLysThrIleAspLysAsnLys
AsnAlaThrLysGluGluGluLysLysLysLeuTyrGlnAlaGlnTyrAspLeuPheIle
TyrAsnLysGlnLeuGluGluAlaHisAsnLeuIleSerValLeuGluLysArgIleAsp
ThrLeuLysLysAsnGluAsnIleLysGluLeuLeuAspLysIleAsnGluIleLysAsn
ProProProAlaGlyGlyLeuLeuLeuIleAspProValMetThrSerGluLeuHisLeu
AspLeuPheArgThrLeuGlyCysArgArgAlaPhePheIleGlyGluAsnProSer,

characterized by culturing microorganisms transformed with a vector encoding this polypeptide under conditions permitting the expression of the encoded polypeptide and isolating the polypeptide.

**7.** A process for the preparation of a polypeptide having the amino acid sequence

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys
LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerValAspLeuGlnProSerLeuAspSerCys ;

characterized by culturing microorganisms transformed with a vector encoding this polypeptide under conditions permitting the expression of the encoded polypeptide and isolating the polypeptide.

**8.** A process for the preparation of a polypeptide having the amino acid sequence

MetHisHisAlaProAlaGluIleAlaGluThrGluAsnThrLeuGluAsnThrLysIle
LeuLeuLysHisTyrLysGlyLeuValLysTyrTyrAsnGlyGluSerSerProLeuLys
ThrLeuSerGluGluSerIleGlnThrGluAspAsnTyrAlaSerLeuGluAsnPheLys
ValLeuSerLysLeuGluGlyLysLeuLysAspAsnLeuAsnLeuGluLysLysLysLeu
SerTyrLeuSerArgGlyLeuHisHisLeuIleAlaGluLeuLysGluValIleLysAsn
LysAsnTyrThrGlyAsnSerProSerValAsnAsnThrAspValAsnAsnAlaLeuGlu
SerTyrLysLysPheLeuProGluGlyThrAspValAlaThrValValSerGluSerGly-
SerValAspLeuGlnProSerLeuAspSerCys -

characterized by culturing microorganisms transformed with a vector encoding this polypeptide under conditions permitting the expression of the encoded polypeptide and isolating the polypeptide.

**9.** A process for the preparation of a polypeptide having the amino acid sequence

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys
LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerGlyThrLeuCysAspAsnIleHisGlyPheLysTyrLeuIleAspGlyTyr
GluGluIleAsnGluLeuLeuTyrLysLeuAsnPheTyrPheAspLeuLeuArgAlaLys
LeuAsnAsnValCysAlaAsnAspTyrCysGlnIleProPheAsnLeuLysIleArgAla
AsnGluLeuAspValLeuLysLysLeuValPheGlyTyrArgLysProLeuAspAsnIle
LysAspAsnValGlyLysMetGluAspTyrIleLysLysAsnLysLysThrIleGluAsn
IleAsnGluLeuIleGluGluSerLysLysThrIleAspLysAsnLysAsnAlaThrLys
GluGluGluLysLysLysLeuTyrGlnAlaGlnTyrAspLeuPheIleTyrAsnLysGln
LeuGluGluAlaHisAsnLeuIleSerValLeuGluLysArgIleAspThrLeuLysLys
AsnGluAsnIleLysGluLeuLeuAspLysIleAsnGluIleLysAsnProProProAla
GlyGlyLeuLeuLeuIleAspProValMetThrSerGluLeuHisLeuAspLeuPheArg
ThrLeuGlyCysArgArgAlaPhePheIleGlyGluAsnProSer

characterized by culturing microorganisms transformed with a vector encoding this polypeptide under conditions permitting the expression of the encoded polypeptide and isolating the polypeptide.

**10.** A process according to any one of claims 1-9, characterized in that the polypeptide is additionally adsorbed on a carrier material or covalently attached thereto.

**11.** A process for the production of a replicable microbial vector encoding a polypeptide as defined in claims 1 to 9, characterized by integrating a DNA fragment encoding such a polypeptide into a

replicable microbial vector.

**12.** A process according to claim 11, characterized in that the DNA fragment encoding a polypeptide as defined in claims 1 to 9 is operably linked to an expression control sequence.

**13.** A process for the production of a transformed microorganism which is capable of producing a polypeptide as defined in claims 1 to 9, characterized by transforming a microorganism in a manner known per se with a vector as defined in claim 11 or 12.

**14.** A process for the production of antibodies against a conserved epitope of the 190 kD precursor of the major merozoite surface antigens of P. falciparum, characterized by injecting a polypeptide as defined in any one of claims 1 to 10 into a suitable host capable of eliciting an imune response against the polypeptide and isolating the antibody according to known methods.

**15.** A process for the production of an immunogenic composition capable of eliciting antibodies specific for p190, the precursor to the major merozoite surface antigens of P. falciparum, in a host, characterized by mixing a polypeptide as defined in any one of claims 1 to 10 with a pharmaceutically acceptable adjuvant.

**16.** The use of a polypeptide according to any one of claims 1 to 9 for the production of an immunogenic composition for the immunization of mammals against malaria.

**Revendications**
**Revendications pour les Etats contractants suivants : AT, BE, CH, DE, FR, GB, IT, LI, NL, SE**

**1.** Polypeptide de formule

A-B-C (I)

dans laquelle
A est un radical peptide d'affinité ou peut manquer,
B représente

ThrLeuCysAspAsnIleHisGlyPheLysTyrLeuIleAspGlyTyrGluGluIle
AsnGluLeuLeuTyrLysLeuAsnPheTyrPheAspLeuLeuArgAlaLysLeuAsn
AsnValCysAlaAsnAspTyrCysGlnIleProPheAsnLeuLysIleArgAlaAsn
GluLeuAspValLeuLysLysLeuValPheGlyTyrArgLysProLeuAspAsnIle
LysAspAsnValGlyLysMetGluAspTyrIleLysLysAsnLysLysThrIleGlu
AsnIleAsnGluLeuIleGluGluSerLysLysThrIleAspLysAsnLysAsnAla
ThrLysGluGluGluLysLysLysLeuTyrGlnAlaGlnTyrAspLeuPheIleTyr
AsnLysGlnLeuGluGluAlaHisAsnLeuIleSerValLeuGluLysArgIleAsp
ThrLeuLysLysAsnGluAsnIleLysGluLeuLeuAspLysIleAsnGluIleLys
AsnProProPro

ou un de ses fragments, ou

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

ou un de ses fragments, ou une combinaison de ces séquences ou fragments, étant entendu que les fragments mentionnés ci-dessus représentent ou contiennent au moins un épitope du précurseur à 190 kD des antigènes de surface surtout mérozoïtes de P.falciparum, et

C représente un radical peptide ou peut manquer,

caractérisé en ce que le polypeptide est apte à provoquer une réponse immunitaire contre divers isolats de P. falciparum.

**2.** Polypeptide de formule

A-B-C (I)

dans laquelle

A est un radical peptide d'affinité ou peut manquer,

B représente

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

ou un de ses fragments, où ce fragment représente ou contient au moins un épitope du précurseur à 190 kD des antigènes de surface surtout mérozoïtes de P. falciparum, et

C est un radical peptide ou peut manquer,

caractérisé en ce que le polypeptide est apte à provoquer une réponse immunitaire contre divers isolats de P. falciparum.

**3.** Polypeptide selon la revendication 1 oou 2, caractérisé en ce que le radical peptide d'affinité A contient deux ou plusieurs radicaux histidine voisins.

**4.** Polypeptide selon la revendication 3, caractérisé en ce que le radical peptide d'affinité A est
MetHisHisAlaProGlySerGly,
MetHisHisAlaProGlySer, ou
MetHisHisAlaPro.

**5.** Polypeptide salon la revendication 1 ou la revendication 2, caractérisé en ce que le radical peptide C représente

AlaGlyGlyLeuLeuLeuIleAspProValMetThrSerGluLeuHisLeuAsp-
LeuPheArgThrLeuGlyCysArgArgAlaPhePheIleGlyGluAsnProSer,

ou
ValAspLeuGlnProSerLeuAspSerCys.

**6.** Polypeptide ayant la séquence d'acides aminés

MetHisHisAlaProGlySerGlyThrLeuCysAspAsnIleHisGlyPheLysTyrLeu
IleAspGlyTyrGluGluIleAsnGluLeuLeuTyrLysLeuAsnPheTyrPheAspLeu
LeuArgAlaLysLeuAsnAsnValCysAlaAsnAspTyrCysGlnIleProPheAsnLeu
LysIleArgAlaAsnGluLeuAspValLeuLysLysLeuValPheGlyTyrArgLysPro
LeuAspAsnIleLysAspAsnValGlyLysMetGluAspTyrIleLysLysAsnLysLys
ThrIleGluAsnIleAsnGluLeuIleGluGluSerLysLysThrIleAspLysAsnLys
AsnAlaThrLysGluGluGluLysLysLysLeuTyrGlnAlaGlnTyrAspLeuPheIle
TyrAsnLysGlnLeuGluGluAlaHisAsnLeuIleSerValLeuGluLysArgIleAsp
ThrLeuLysLysAsnGluAsnIleLysGluLeuLeuAspLysIleAsnGluIleLysAsn
ProProProAlaGlyGlyLeuLeuLeuIleAspProValMetThrSerGluLeuHisLeu
AspLeuPheArgThrLeuGlyCysArgArgAlaPhePheIleGlyGluAsnProSer.

**7.** Polypeptide ayant la séquence d'acides aminés

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys
LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerValAspLeuGlnProSerLeuAspSerCys.

**8.** Polypeptide ayant la séquence d'acides aminés

MetHisHisAlaProAlaGluIleAlaGluThrGluAsnThrLeuGluAsnThrLysIle LeuLeuLysHisTyrLysGlyLeuValLysTyrTyrAsnGlyGluSerSerProLeuLys ThrLeuSerGluGluSerIleGlnThrGluAspAsnTyrAlaSerLeuGluAsnPheLys ValLeuSerLysLeuGluGlyLysLeuLysAspAsnLeuAsnLeuGluLysLysLysLeu SerTyrLeuSerArgGlyLeuHisHisLeuIleAlaGluLeuLysGluValIleLysAsn LysAsnTyrThrGlyAsnSerProSerValAsnAsnThrAspValAsnAsnAlaLeuGlu SerTyrLysLysPheLeuProGluGlyThrAspValAlaThrValValSerGluSerGly SerValAspLeuGlnProSerLeuAspSerCys.

**9.** Polypeptide ayant la séquence d'acides aminés

MetHisHisAlaProGlySerAlaGluIleAlaGluThrGluAsnThrLeuGluAsnThr LysIleLeuLeuLysHisTyrLysGlyLeuValLysTyrTyrAsnGlyGluSerSerPro LeuLysThrLeuSerGluGluSerIleGlnThrGluAspAsnTyrAlaSerLeuGluAsn PheLysValLeuSerLysLeuGluGlyLysLeuLysAspAsnLeuAsnLeuGluLysLys LysLeuSerTyrLeuSerArgGlyLeuHisHisLeuIleAlaGluLeuLysGluValIle LysAsnLysAsnTyrThrGlyAsnSerProSerValAsnAsnThrAspValAsnAsnAla LeuGluSerTyrLysLysPheLeuProGluGlyThrAspValAlaThrValValSerGlu SerGlySerGlyThrLeuCysAspAsnIleHisGlyPheLysTyrLeuIleAspGlyTyr GluGluIleAsnGluLeuLeuTyrLysLeuAsnPheTyrPheAspLeuLeuArgAlaLys LeuAsnAsnValCysAlaAsnAspTyrCysGlnIleProPheAsnLeuLysIleArgAla AsnGluLeuAspValLeuLysLysLeuValPheGlyTyrArgLysProLeuAspAsnIle LysAspAsnValGlyLysMetGluAspTyrIleLysLysAsnLysLysThrIleGluAsn IleAsnGluLeuIleGluGluSerLysLysThrIleAspLysAsnLysAsnAlaThrLys GluGluGluLysLysLysLeuTyrGlnAlaGlnTyrAspLeuPheIleTyrAsnLysGln LeuGluGluAlaHisAsnLeuIleSerValLeuGluLysArgIleAspThrLeuLysLys AsnGluAsnIleLysGluLeuLeuAspLysIleAsnGluIleLysAsnProProProAla GlyGlyLeuLeuLeuIleAspProValMetThrSerGluLeuHisLeuAspLeuPheArg ThrLeuGlyCysArgArgAlaPhePheIleGlyGluAsnProSer.

**10.** Polypeptide selon l'une des revendications 1-9, qui est lié par adsorption ou covalence à un support.

**11.** Fragment d'ADN qui code pour un polypeptide selon l'une des revendications 1 à 9.

**12.** Vecteur microbien réplicable qui contient un fragment d'ADN selon la revendication 11.

**13.** Vecteur réplicable selon la revendication 12, caractérisé en ce que le fragment d'ADN est lié de façon opératoire à une séquence de commande d'expression.

14. Microorganisme qui est transformé avec un vecteur selon la revendication 12 ou 13.

15. Polypeptide selon l'une des revendications 1 à 10 pour l'immunisation des mammifères contre la malaria.

16. Procédé de préparation de polypeptides selon l'une des revendications 1 à 9, caractérisé en ce qu'on cultive des microorganismes qui sont transformés avec un vecteur selon la revendication 12 ou 13, dans des conditions qui permettent l'expression du polypeptide codé.

17. Procédé de préparation de microorganismes selon la revendication 14, caractérisé en ce qu'on transforme un microorganisme de façon connue avec un vecteur selon la revendication 12 ou 13.

18. Procédé de préparation d'anticorps qui sont dirigés contre un épitope conservé du précurseur à 190 kD des antigènes de surface surtout mérozoïtes de P. falciparum, caractérisé en ce qu'on injecte un polypeptide selon l'une des revendications 1 à 10 à un organisme hôte approprié, qui est apte à déclencher une réponse immunitaire contre le polypeptide, et en ce qu'on isole les anticorps formés selon des procédés connus.

19. Composition immunogène qui est apte à susciter chez un hôte des anticorps qui sont spécifiques de p190, précurseur des antigènes de surface surtout mérozoïtes de P. falciparum, ces compositions immunogènes contenant un polypeptide selon l'une des revendications 1-10 ainsi qu'un adjuvant pharmaceutiquement acceptable.

20. Composition selon la revendication 19 sous forme de vaccin.

21. Application d'un polypeptide selon l'une des revendications 1 à 9 à la préparation d'une composition immunogène pour l'immunisation des mammifères contre la malaria.

**Revendications pour l'Etat contractant suivant : ES**

1. Procédé de préparation de polypeptides de formule

A-B-C (I)

dans laquelle
A est un radical peptide d'affinité ou peut manquer,
B représente

ThrLeuCysAspAsnIleHisGlyPheLysTyrLeuIleAspGlyTyrGluGluIle
AsnGluLeuLeuTyrLysLeuAsnPheTyrPheAspLeuLeuArgAlaLysLeuAsn
AsnValCysAlaAsnAspTyrCysGlnIleProPheAsnLeuLysIleArgAlaAsn
GluLeuAspValLeuLysLysLeuValPheGlyTyrArgLysProLeuAspAsnIle
LysAspAsnValGlyLysMetGluAspTyrIleLysLysAsnLysLysThrIleGlu
AsnIleAsnGluLeuIleGluGluSerLysLysThrIleAspLysAsnLysAsnAla
ThrLysGluGluGluLysLysLysLeuTyrGlnAlaGlnTyrAspLeuPheIleTyr
AsnLysGlnLeuGluGluAlaHisAsnLeuIleSerValLeuGluLysArgIleAsp
ThrLeuLysLysAsnGluAsnIleLysGluLeuLeuAspLysIleAsnGluIleLys
AsnProProPro

ou un de ses fragments, ou

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

ou un de ses fragments, ou une combinaison de ces séquences ou fragments, étant entendu que les fragments mentionnés ci-dessus représentent ou contiennent au moins un épitope du précurseur à 190 kD des antigènes de surface surtout mérozoïtes de P. falciparum,et

C représente un radical peptide ou peut manquer,

où le polypeptide est apte à provoquer une réponse immunitaire contre divers isolats de P. falciparum, caractérisé en ce qu'on cultive des microorganismes qui sont transformés avec un vecteur qui code pour ce polypeptide, dans des conditions qui permettent l'expression du polypeptide codé, et en ce qu'on isole le peptide.

**2.** Procédé de préparation de polypeptides de formule

A-B-C (I)

dans laquelle

A est un radical peptide d'affinité ou peut manquer,

B représente

AlaGluIleAlaGluThrGluAsnThrLeuGluAsnThrLysIleLeuLeuLysHis
TyrLysGlyLeuValLysTyrTyrAsnGlyGluSerSerProLeuLysThrLeuSer
GluGluSerIleGlnThrGluAspAsnTyrAlaSerLeuGluAsnPheLysValLeu
SerLysLeuGluGlyLysLeuLysAspAsnLeuAsnLeuGluLysLysLysLeuSer
TyrLeuSerArgGlyLeuHisHisLeuIleAlaGluLeuLysGluValIleLysAsn
LysAsnTyrThrGlyAsnSerProSerValAsnAsnThrAspValAsnAsnAlaLeu
GluSerTyrLysLysPheLeuProGluGlyThrAspValAlaThrValValSerGlu
SerGlySer

ou un de ses fragments, où ce fragment représente ou contient au moins un épitope du précurseur à 190 kD des antigènes de surface surtout mérozoïtes de P. falciparum, et

C est un radical peptide ou peut manquer,

où le polypeptide est apte à susciter une réponse immunitaire contre divers isolats de P. falciparum, caractérisé en ce qu'on cultive des microorganismes qui sont transformés avec un vecteur qui code pour ce polypeptide, dans des conditions qui permettent l'expression du polypeptide codé, et en ce qu'on isole le polypeptide.

**3.** Procédé selon la revendication 1 ou 2, caractérisé en ce que le radical peptide d'affinité A contient deux ou plusieurs radicaux histidine voisins.

**4.** Procédé selon la revendication 3, caractérisé en ce que la radical peptide d'affinité A est MetHisHisAlaProGlySerGly,

MetHisHisAlaProGlySer ou
MetHisHisAlaPro.

5. Procédé selon la revendication 1 ou 2, caractérisé en ce que le radical peptide C est

AlaGlyGlyLeuLeuLeuIleAspProValMetThrSerGluLeuHisLeuAsp-
LeuPheArgThrLeuGlyCysArgArgAlaPhePheIleGlyGluAsnProSer,

ou
ValAspLeuGlnProSerLeuAspSerCys.

6. Procédé de préparation d'un polypeptide ayant la séquence d'acides aminés

MetHisHisAlaProGlySerGlyThrLeuCysAspAsnIleHisGlyPheLysTyrLeu
IleAspGlyTyrGluGluIleAsnGluLeuLeuTyrLysLeuAsnPheTyrPheAspLeu
LeuArgAlaLysLeuAsnAsnValCysAlaAsnAspTyrCysGlnIleProPheAsnLeu
LysIleArgAlaAsnGluLeuAspValLeuLysLysLeuValPheGlyTyrArgLysPro
LeuAspAsnIleLysAspAsnValGlyLysMetGluAspTyrIleLysLysAsnLysLys
ThrIleGluAsnIleAsnGluLeuIleGluGluSerLysLysThrIleAspLysAsnLys
AsnAlaThrLysGluGluGluLysLysLysLeuTyrGlnAlaGlnTyrAspLeuPheIle
TyrAsnLysGlnLeuGluGluAlaHisAsnLeuIleSerValLeuGluLysArgIleAsp
ThrLeuLysLysAsnGluAsnIleLysGluLeuLeuAspLysIleAsnGluIleLysAsn
ProProProAlaGlyGlyLeuLeuLeuIleAspProValMetThrSerGluLeuHisLeu
AspLeuPheArgThrLeuGlyCysArgArgAlaPhePheIleGlyGluAsnProSer,

caractérisé en ce qu'on cultive des microorganismes qui sont transformés avec un vecteur qui code pour ce polypeptide, dans des conditions qui permettent l'expression du polypeptide codé, et en ce qu'on isole le polypeptide.

7. Procédé de préparation d'un polypeptide ayant la séquence d'acides aminés

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys
LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerValAspLeuGlnProSerLeuAspSerCys

caractérisé en ce qu'on cultive des microorganismes qui sont transformés avec un vecteur qui code pour ce polypeptide, dans des conditions qui permettent l'expression du polypeptide codé, et en ce qu'on isole le polypeptide.

**8.** Procédé de préparation d'un polypeptide ayant la séquence d'acides aminés

MetHisHisAlaProAlaGluIleAlaGluThrGluAsnThrLeuGluAsnThrLysIle
LeuLeuLysHisTyrLysGlyLeuValLysTyrTyrAsnGlyGluSerSerProLeuLys
ThrLeuSerGluGluSerIleGlnThrGluAspAsnTyrAlaSerLeuGluAsnPheLys
ValLeuSerLysLeuGluGlyLysLeuLysAspAsnLeuAsnLeuGluLysLysLysLeu
SerTyrLeuSerArgGlyLeuHisHisLeuIleAlaGluLeuLysGluValIleLysAsn
LysAsnTyrThrGlyAsnSerProSerValAsnAsnThrAspValAsnAsnAlaLeuGlu
SerTyrLysLysPheLeuProGluGlyThrAspValAlaThrValValSerGluSerGly-
SerValAspLeuGlnProSerLeuAspSerCys

caractérisé en ce qu'on cultive des microorganismes qui son transformés avec un vecteur qui code pour ce polypeptide, dans des conditions qui permettent l'expression du polypeptide codé, et en ce qu'on isole le polypeptide.

**9.** Procédé de préparation d'un polypeptide ayant la séquence d'acides aminés

MetHisHisAlaProGlySerAlaGluIleAlaGluThrGluAsnThrLeuGluAsnThr
LysIleLeuLeuLysHisTyrLysGlyLeuValLysTyrTyrAsnGlyGluSerSerPro
LeuLysThrLeuSerGluGluSerIleGlnThrGluAspAsnTyrAlaSerLeuGluAsn
PheLysValLeuSerLysLeuGluGlyLysLeuLysAspAsnLeuAsnLeuGluLysLys
LysLeuSerTyrLeuSerArgGlyLeuHisHisLeuIleAlaGluLeuLysGluValIle
LysAsnLysAsnTyrThrGlyAsnSerProSerValAsnAsnThrAspValAsnAsnAla
LeuGluSerTyrLysLysPheLeuProGluGlyThrAspValAlaThrValValSerGlu
SerGlySerGlyThrLeuCysAspAsnIleHisGlyPheLysTyrLeuIleAspGlyTyr
GluGluIleAsnGluLeuLeuTyrLysLeuAsnPheTyrPheAspLeuLeuArgAlaLys
LeuAsnAsnValCysAlaAsnAspTyrCysGlnIleProPheAsnLeuLysIleArgAla
AsnGluLeuAspValLeuLysLysLeuValPheGlyTyrArgLysProLeuAspAsnIle
LysAspAsnValGlyLysMetGluAspTyrIleLysLysAsnLysLysThrIleGluAsn
IleAsnGluLeuIleGluGluSerLysLysThrIleAspLysAsnLysAsnAlaThrLys
GluGluGluLysLysLysLeuTyrGlnAlaGlnTyrAspLeuPheIleTyrAsnLysGln
LeuGluGluAlaHisAsnLeuIleSerValLeuGluLysArgIleAspThrLeuLysLys
AsnGluAsnIleLysGluLeuLeuAspLysIleAsnGluIleLysAsnProProProAla
GlyGlyLeuLeuLeuIleAspProValMetThrSerGluLeuHisLeuAspLeuPheArg
ThrLeuGlyCysArgArgAlaPhePheIleGlyGluAsnProSer

caractérisé en ce qu'on cultive des microorganismes qui sont transformés avec un vecteur qui code pour ce polypeptide, dans des conditions qui permettent l'expression du polypeptide codé, et en ce qu'on isole le polypeptide.

**10.** Procédé selon l'une des revendications 1-9, caractérisé en ce que le polypeptide est en outre lié par adsorption ou covalence à un support.

**11.** Procédé de préparation d'un vecteur microbien réplicable qui code pour un polypeptide tel que défini dans les revendications 1 à 9, caractérisé en ce qu'on intègre un fragment d'ADN qui code pour un tel polypeptide dans un vecteur microbien réplicable.

**12.** Procédé selon la revendication 11, caractérisé en ce que le fragment d'ADN qui code pour un polypeptide tel que défini dans les revendications 1 à 9 est lié de façon opératoire à une séquence de commande d'expression.

**13.** Procédé de préparation d'un microorganisme transformé qui est apte à produire un polypeptide tel que défini dans les revendications 1 à 9, caractérisé en ce qu'on transforme un microorganisme de façon connue avec un vecteur tel que défini dans la revendication 11 ou 12.

**14.** Procédé de préparation d'anticorps dirigés contre un épitope conservé du précurseur à 190 kD des antigènes de surface surtout mérozoïtes de P. falciparum, caractérisé en ce qu'on injecte un polypeptide tel que défini dans l'une des revendications 1 à 10, à un organisme hôté approprié apte à déclencher une réponse immunitaire contre le polypeptide, et en ce qu'on isole les anticorps selon des procédés connus.

**15.** Procédé de préparation d'une composition immunogène apte à susciter chez un hôte des anticorps spécifiques de p190, précurseur des antigènes de surface surtout mérozoïtes de P. falciparum, caractérisé en ce qu'on mélange un polypeptide tel que défini dans l'une des revendications 1 à 10 avec un adjuvant pharmaceutiquement acceptable.

**16.** Application d'un polypeptide selon l'une des revendications 1 à 9 , a la préparation d'une composition immunogène pour l'immunisation des mammifères contre la malaria.

FIG. 1

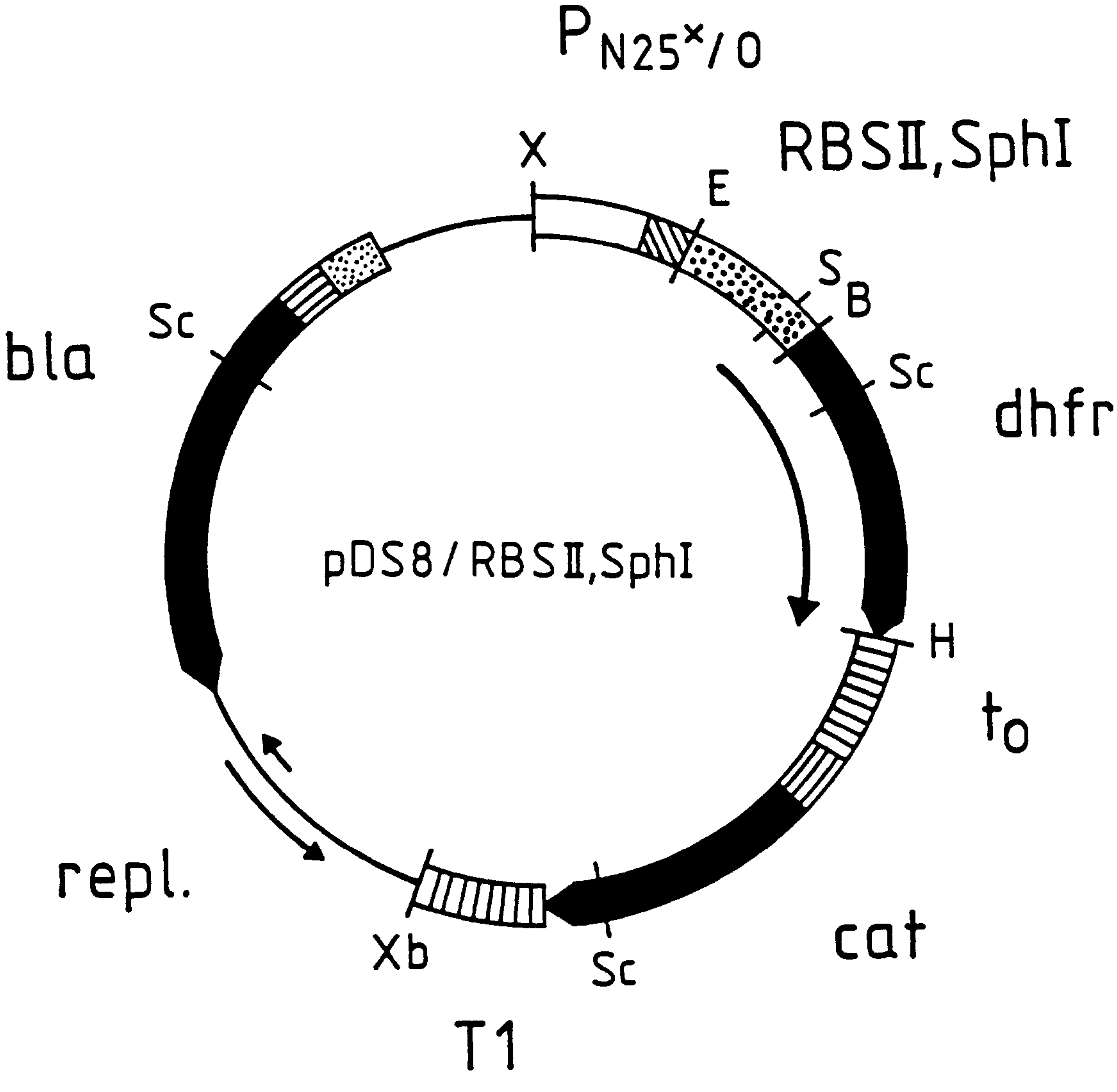
$P_{N25}$x/0
X
E
RBSII,SphI
S
B
Sc
Sc
bla
dhfr
pDS8/RBSII,SphI
H
$t_o$
repl.
Xb
Sc
cat
T1

FIG. 2

| | 10 | 20 | 30 | 40 | 50 |
|---|---|---|---|---|---|
| 0 | CCTCGAGGCT | GGCATCCCTA | ACATATCCGA | ATGGTTACTT | AAACAACGGA |
| 50 | GGACTAGCGT | ATCCCTTCGC | ATAGGGTTTG | AGTTAGATAA | AGTATATGCT |
| 100 | GAACTTTCTT | CTTTGCTCAA | AGAATCATAA | AAAATTTATT | TGCTTTCAGG |
| 150 | AAAATTTTTC | TGTATAATAG | ATTCAAATTG | TGAGCGGATA | ACAATTTGAA |
| 200 | TTCATTAAAG | AGGAGAAATT | AAGCATGCGA | GGATCCGGCA | TCATGGTTCG |
| 250 | ACCATTGAAC | TGCATCGTCG | CCGTGTCCCA | AAATATGGGG | ATTGGCAAGA |
| 300 | ACGGAGACCT | ACCCTGGCCT | CCGCTCAGGA | ACGAGTTCAA | GTACTTCCAA |
| 350 | AGAATGACCA | CAACCTCTTC | AGTGGAAGGT | AAACAGAATC | TGGTGATTAT |
| 400 | GGGTAGGAAA | ACCTGGTTCT | CCATTCCTGA | GAAGAATCGA | CCTTTAAAGG |
| 450 | ACAGAATTAA | TATAGTTCTC | AGTAGAGAAC | TCAAAGAACC | ACCACGAGGA |
| 500 | GCTCATTTTC | TTGCCAAAAG | TTTGGATGAT | GCCTTAAGAC | TTATTGAACA |
| 550 | ACCGGAATTG | GCAAGTAAAG | TAGACATGGT | TTGGATAGTC | GGAGGCAGTT |
| 600 | CTGTTTACCA | GGAAGCCATG | AATCAACCAG | GCCACCTTAG | ACTCTTTGTG |
| 650 | ACAAGGATCA | TGCAGGAATT | TGAAAGTGAC | ACGTTTTTCC | CAGAAATTGA |
| 700 | TTTGGGGAAA | TATAAACTTC | TCCCAGAATA | CCCAGGCGTC | CTCTCTGAGG |
| 750 | TCCAGGAGGA | AAAAGGCATC | AAGTATAAGT | TTGAAGTCTA | CGAGAAGAAA |
| 800 | GACTAACAGG | AAGATGCTTT | CAAGTTCTCT | GCTCCCCTCC | TAAAGCTATG |
| 850 | CATTTTTATA | AGACCATGGG | ACTTTTGCTG | GCTTTAGATC | CGGCCAAGCT |
| 900 | TGGACTCCTG | TTGATAGATC | CAGTAATGAC | CTCAGAACTC | CATCTGGATT |
| 950 | TGTTCAGAAC | GCTCGGTTGC | CGCCGGGCGT | TTTTTATTGG | TGAGAATCCA |
| 1000 | AGCTAGCTTG | GCGAGATTTT | CAGGAGCTAA | GGAAGCTAAA | ATGGAGAAAA |
| 1050 | AAATCACTGG | ATATACCACC | GTTGATATAT | CCCAATGGCA | TCGTAAAGAA |
| 1100 | CATTTTGAGG | CATTTCAGTC | AGTTGCTCAA | TGTACCTATA | ACCAGACCGT |
| 1150 | TCAGCTGGAT | ATTACGGCCT | TTTTAAAGAC | CGTAAAGAAA | AATAAGCACA |

FIG. 2 (CONT.)

| | 10 | 20 | 30 | 40 | 50 |
|---|---|---|---|---|---|
| 1200 | AGTTTTATCC | GGCCTTTATT | CACATTCTTG | CCCGCCTGAT | GAATGCTCAT |
| 1250 | CCGGAATTTC | GTATGGCAAT | GAAAGACGGT | GAGCTGGTGA | TATGGGATAG |
| 1300 | TGTTCACCCT | TGTTACACCG | TTTTCCATGA | GCAAACTGAA | ACGTTTTCAT |
| 1350 | CGCTCTGGAG | TGAATACCAC | GACGATTTCC | GGCAGTTTCT | ACACATATAT |
| 1400 | TCGCAAGATG | TGGCGTGTTA | CGGTGAAAAC | CTGGCCTATT | TCCCTAAAGG |
| 1450 | GTTTATTGAG | AATATGTTTT | TCGTCTCAGC | CAATCCCTGG | GTGAGTTTCA |
| 1500 | CCAGTTTTGA | TTTAAACGTG | GCCAATATGG | ACAACTTCTT | CGCCCCCGTT |
| 1550 | TTCACCATGG | GCAAATATTA | TACGCAAGGC | GACAAGGTGC | TGATGCCGCT |
| 1600 | GGCGATTCAG | GTTCATCATG | CCGTCTGTGA | TGGCTTCCAT | GTCGGCAGAA |
| 1650 | TGCTTAATGA | ATTACAACAG | TACTGCGATG | AGTGGCAGGG | CGGGGCGTAA |
| 1700 | TTTTTTTAAG | GCAGTTATTG | GTGCCCTTAA | ACGCCTGGGG | TAATGACTCT |
| 1750 | CTAGCTTGAG | GCATCAAATA | AAACGAAAGG | CTCAGTCGAA | AGACTGGGCC |
| 1800 | TTTCGTTTTA | TCTGTTGTTT | GTCGGTGAAC | GCTCTCCTGA | GTAGGACAAA |
| 1850 | TCCGCCGCTC | TAGAGC | | | |

2068

pBR322 A

4358

FIG. 3

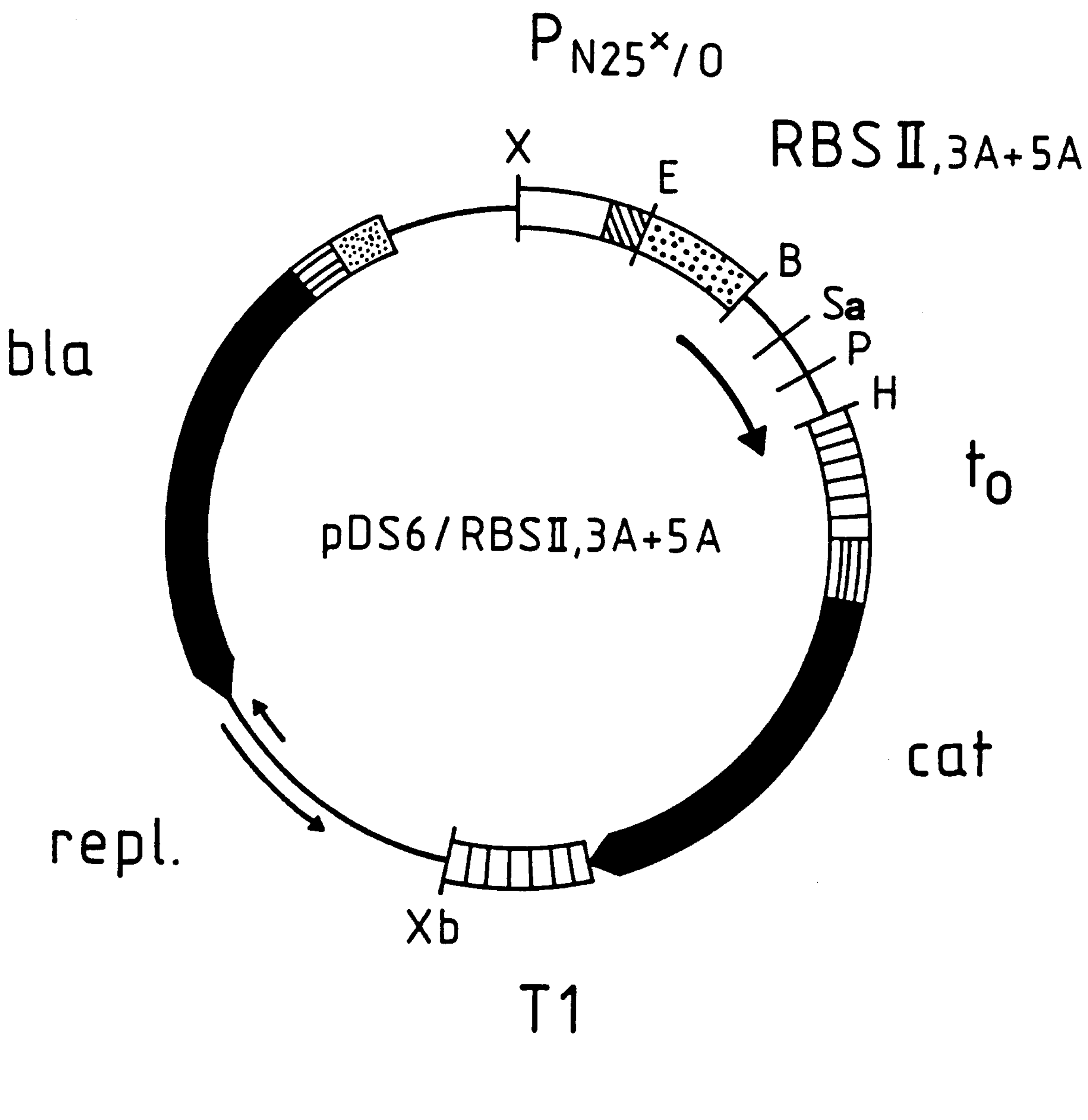
P N25x/0
X
E
RBS II,3A+5A
B
Sa
P
H
bla
t0
pDS6/RBSII,3A+5A
cat
repl.
Xb
T1

FIG. 4

```
          10         20         30         40         50
   0 CCTCGAGGCT GGCATCCCTA ACATATCCGA ATGGTTACTT AAACAACGGA
  50 GGACTAGCGT ATCCCTTCGC ATAGGGTTTG AGTTAGATAA AGTATATGCT
 100 GAACTTTCTT CTTTGCTCAA AGAATCATAA AAAATTTATT TGCTTTCAGG
 150 AAAATTTTTC TGTATAATAG ATTCAAATTG TGAGCGGATA ACAATTTGAA
 200 TTCATTAAAG AGGAGAAATT AACTATGAGG GGATCCGTCG ACCTGCAGCC
 250 AAGCTTGGAC TCCTGTTGAT AGATCCAGTA ATGACCTCAG AACTCCATCT
 300 GGATTTGTTC AGAACGCTCG GTTGCCGCCG GGCGTTTTTT ATTGGTGAGA
 350 ATCCAAGCTA GCTTGGCGAG ATTTTCAGGA GCTAAGGAAG CTAAAATGGA
 400 GAAAAAAATC ACTGGATATA CCACCGTTGA TATATCCCAA TGGCATCGTA
 450 AAGAACATTT TGAGGCATTT CAGTCAGTTG CTCAATGTAC CTATAACCAG
 500 ACCGTTCAGC TGGATATTAC GGCCTTTTTA AAGACCGTAA AGAAAAATAA
 550 GCACAAGTTT TATCCGGCCT TTATTCACAT TCTTGCCCGC CTGATGAATG
 600 CTCATCCGGA ATTTCGTATG GCAATGAAAG ACGGTGAGCT GGTGATATGG
 650 GATAGTGTTC ACCCTTGTTA CACCGTTTTC CATGAGCAAA CTGAAACGTT
 700 TTCATCGCTC TGGAGTGAAT ACCACGACGA TTTCCGGCAG TTTCTACACA
 750 TATATTCGCA AGATGTGGCG TGTTACGGTG AAAACCTGGC CTATTTCCCT
 800 AAAGGGTTTA TTGAGAATAT GTTTTTCGTC TCAGCCAATC CCTGGGTGAG
 850 TTTCACCAGT TTTGATTTAA ACGTGGCCAA TATGGACAAC TTCTTCGCCC
 900 CCGTTTTCAC CATGGGCAAA TATTATACGC AAGGCGACAA GGTGCTGATG
 950 CCGCTGGCGA TTCAGGTTCA TCATGCCGTC TGTGATGGCT TCCATGTCGG
1000 CAGAATGCTT AATGAATTAC AACAGTACTG CGATGAGTGG CAGGGCGGGG
1050 CGTAATTTTT TTAAGGCAGT TATTGGTGCC CTTAAACGCC TGGGGTAATG
1100 ACTCTCTAGC TTGAGGCATC AAATAAAACG AAAGGCTCAG TCGAAAGACT
1150 GGGCCTTTCG TTTTATCTGT TGTTTGTCGG TGAACGCTCT CCTGAGTAGG
1200 ACAAATCCGC CGCTCTAGAG C ——————————————————
                        |
                       2068

     ———— pBR322 ——————————————————— A
                                     |
                                    4358
```

FIG. 5

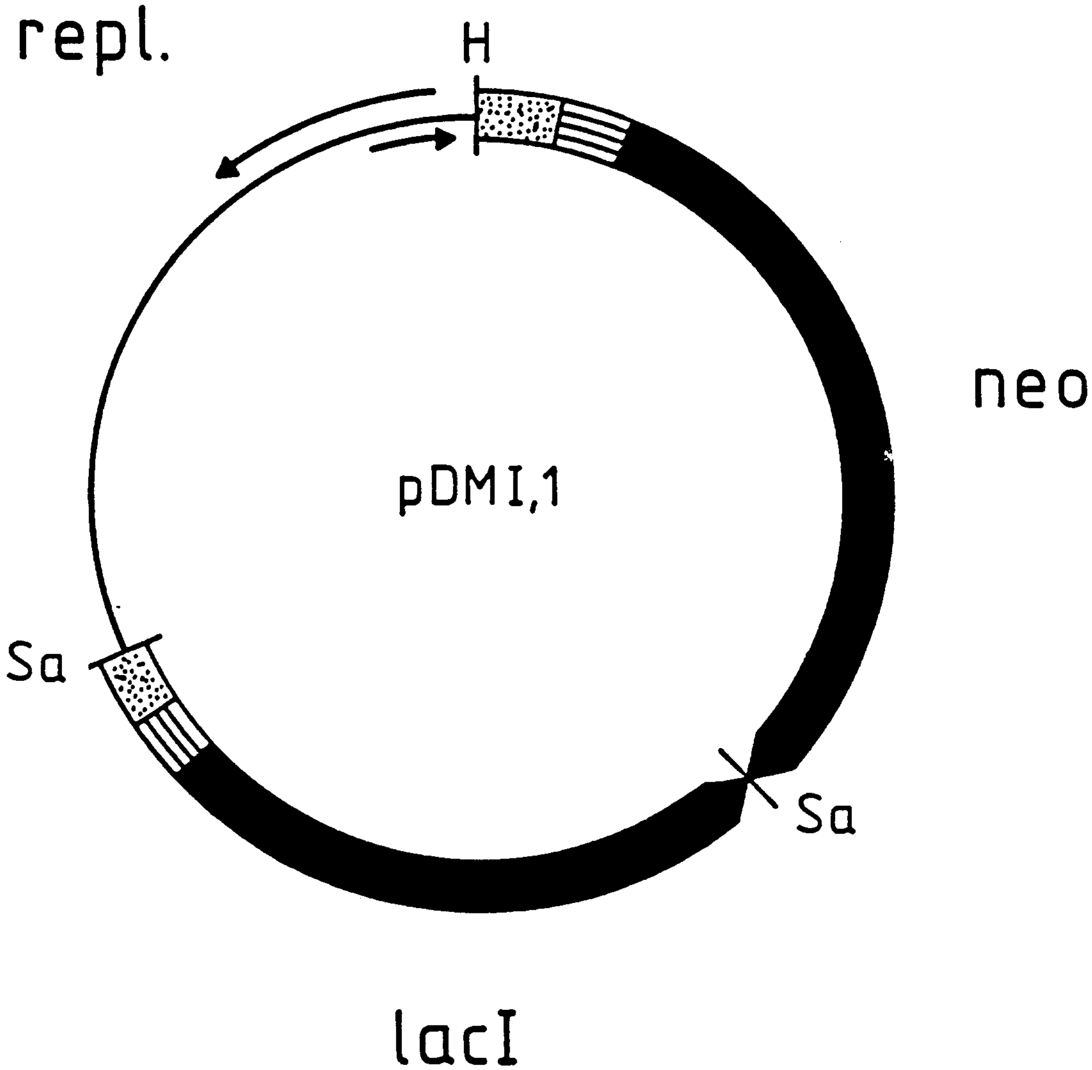

FIG. 6

| | 10 | 20 | 30 | 40 | 50 |
|---|---|---|---|---|---|
| 0 | AAGCTTCACG | CTGCCGCAAG | CACTCAGGGC | GCAAGGGCTG | CTAAAGGAAG |
| 50 | CGGAACACGT | AGAAAGCCAG | TCCGCAGAAA | CGGTGCTGAC | CCCGGATGAA |
| 100 | TGTCAGCTAC | TGGGCTATCT | GGACAAGGGA | AAACGCAAGC | GCAAAGAGAA |
| 150 | AGCAGGTAGC | TTGCAGTGGG | CTTACATGGC | GATAGCTAGA | CTGGGCGGTT |
| 200 | TTATGGACAG | CAAGCGAACC | GGAATTGCCA | GCTGGGGCGC | CCTCTGGTAA |
| 250 | GGTTGGGAAG | CCCTGCAAAG | TAAACTGGAT | GGCTTTCTTG | CCGCCAAGGA |
| 300 | TCTGATGGCG | CAGGGGATCA | AGATCTGATC | AAGAGACAGG | ATGAGGATCG |
| 350 | TTTCGCATGA | TTGAACAAGA | TGGATTGCAC | GCAGGTTCTC | CGGCCGCTTG |
| 400 | GGTGGAGAGG | CTATTCGGCT | ATGACTGGGC | ACAACAGACA | ATCGGCTGCT |
| 450 | CTGATGCCGC | CGTGTTCCGG | CTGTCAGCGC | AGGGGCGCCC | GGTTCTTTTT |
| 500 | GTCAAGACCG | ACCTGTCCGG | TGCCCTGAAT | GAACTGCAGG | ACGAGGCAGC |
| 550 | GCGGCTATCG | TGGCTGGCCA | CGACGGGCGT | TCCTTGCGCA | GCTGTGCTCG |
| 600 | ACGTTGTCAC | TGAAGCGGGA | AGGGACTGGC | TGCTATTGGG | CGAAGTGCCG |
| 650 | GGGCAGGATC | TCCTGTCATC | TCACCTTGCT | CCTGCCGAGA | AAGTATCCAT |
| 700 | CATGGCTGAT | GCAATGCGGC | GGCTGCATAC | GCTTGATCCG | GCTACCTGCC |
| 750 | CATTCGACCA | CCAAGCGAAA | CATCGCATCG | AGCGAGCACG | TACTCGGATG |
| 800 | GAAGCCGGTC | TTGTCGATCA | GGATGATCTG | GACGAAGAGC | ATCAGGGGCT |
| 850 | CGCGCCAGCC | GAACTGTTCG | CCAGGCTCAA | GGCGCGCATG | CCCGACGGCG |
| 900 | AGGATCTCGT | CGTGACCCAT | GGCGATGCCT | GCTTGCCGAA | TATCATGGTG |
| 950 | GAAAATGGCC | GCTTTTCTGG | ATTCATCGAC | TGTGGCCGGC | TGGGTGTGGC |
| 1000 | GGACCGCTAT | CAGGACATAG | CGTTGGCTAC | CCGTGATATT | GCTGAAGAGC |
| 1050 | TTGGCGGCGA | ATGGGCTGAC | CGCTTCCTCG | TGCTTTACGG | TATCGCCGCT |
| 1100 | CCCGATTCGC | AGCGCATCGC | CTTCTATCGC | CTTCTTGACG | AGTTCTTCTG |
| 1150 | AGCGGGACTC | TGGGGTTCGA | AATGACCGAC | CAAGCGACGC | CCAACCTGCC |
| 1200 | ATCACGAGAT | TTCGATTCCA | CCGCCGCCTT | CTATGAAAGG | TTGGGCTTCG |
| 1250 | GAATCGTTTT | CCGGGACGCC | GGCTGGATGA | TCCTCCAGCG | CGGGGATCTC |
| 1300 | ATGCTGGAGT | TCTTCGCCCA | CCCCGGGCTC | GATCCCCTCG | CGAGTTGGTT |
| 1350 | CAGCTGCTGC | CTGAGGCTGG | ACGACCTCGC | GGAGTTCTAC | CGGCAGTGCA |
| 1400 | AATCCGTCGG | CATCCAGGAA | ACCAGCAGCG | GCTATCCGCG | CATCCATGCC |
| 1450 | CCCGAACTGC | AGGAGTGGGG | AGGCACGATG | GCCGCTTTGG | TCGACAATTC |
| 1500 | GCGCTAACTT | ACATTAATTG | CGTTGCGCTC | ACTGCCCGCT | TTCCAGTCGG |
| 1550 | GAAACCTGTC | GTGCCAGCTG | CATTAATGAA | TCGGCCAACG | CGCGGGGAGA |
| 1600 | GGCGGTTTGC | GTATTGGGCG | CCAGGGTGGT | TTTTCTTTTC | ACCAGTGAGA |
| 1650 | CGGGCAACAG | CTGATTGCCC | TTCACCGCCT | GGCCCTGAGA | GAGTTGCAGC |
| 1700 | AAGCGGTCCA | CGCTGGTTTG | CCCCAGCAGG | CGAAAATCCT | GTTTGATGGT |
| 1750 | GGTTAACGGC | GGGATATAAC | ATGAGCTGTC | TTCGGTATCG | TCGTATCCCA |
| 1800 | CTACCGAGAT | ATCCGCACCA | ACGCGCAGCC | CGGACTCGGT | AATGGCGCGC |
| 1850 | ATTGCGCCCA | GCGCCATCTG | ATCGTTGGCA | ACCAGCATCG | CAGTGGAAC |

FIG. 6 (CONT.)

| | 10 | 20 | 30 | 40 | 50 |
|---|---|---|---|---|---|
| 1900 | GATGCCCTCA | TTCAGCATTT | GCATGGTTTG | TTGAAAACCG | GACATGGCAC |
| 1950 | TCCAGTCGCC | TTCCCGTTCC | GCTATCGGCT | GAATTTGATT | GCGAGTGAGA |
| 2000 | TATTTATGCC | AGCCAGCCAG | ACGCAGACGC | GCCGAGACAG | AACTTAATGG |
| 2050 | GCCCGCTAAC | AGCGCGATTT | GCTGGTGACC | CAATGCGACC | AGATGCTCCA |
| 2100 | CGCCCAGTCG | CGTACCGTCT | TCATGGGAGA | AAATAATACT | GTTGATGGGT |
| 2150 | GTCTGGTCAG | AGACATCAAG | AAATAACGCC | GGAACATTAG | TGCAGGCAGC |
| 2200 | TTCCACAGCA | ATGGCATCCT | GGTCATCCAG | CGGATAGTTA | ATGATCAGCC |
| 2250 | CACTGACGCG | TTGCGCGAGA | AGATTGTGCA | CCGCCGCTTT | ACAGGCTTCG |
| 2300 | ACGCCGCTTC | GTTCTACCAT | CGACACCACC | ACGCTGGCAC | CCAGTTGATC |
| 2350 | GGCGCGAGAT | TTAATCGCCG | CGACAATTTG | CGACGGCGCG | TGCAGGGCCA |
| 2400 | GACTGGAGGT | GGCAACGCCA | ATCAGCAACG | ACTGTTTGCC | CGCCAGTTGT |
| 2450 | TGTGCCACGC | GGTTGGGAAT | GTAATTCAGC | TCCGCCATCG | CCGCTTCCAC |
| 2500 | TTTTTCCCGC | GTTTTCGCAG | AAACGTGGCT | GGCCTGGTTC | ACCACGCGGG |
| 2550 | AAACGGTCTG | ATAAGAGACA | CCGGCATACT | CTGCGACATC | GTATAACGTT |
| 2600 | ACTGGTTTCA | CATTCACCAC | CCTGAATTGA | CTCTCTTCCG | GGCGCTATCA |
| 2650 | TGCCATACCG | CGAAAGGTTT | TGCACCATTC | GATGGTGTCA | ACGTAAATGC |
| 2700 | ATGCCGCTTC | GCCTTCGCGC | GCGAATTGTC | GACCCTGTCC | CTCCTGTTCA |
| 2750 | GCTACTGACG | GGGTGGTGCG | TAACGGCAAA | AGCACCGCCG | GACATCAGCG |
| 2800 | CTAGCGGAGT | GTATACTGGC | TTACTATGTT | GGCACTGATG | AGGGTGTCAG |
| 2850 | TGAAGTGCTT | CATGTGGCAG | GAGAAAAAAG | GCTGCACCGG | TGCGTCAGCA |
| 2900 | GAATATGTGA | TACAGGATAT | ATTCCGCTTC | CTCGCTCACT | GACTCGCTAC |
| 2950 | GCTCGGTCGT | TCGACTGCGG | CGAGCGGAAA | TGGCTTACGA | ACGGGGCGGA |
| 3000 | GATTTCCTGG | AAGATGCCAG | GAAGATACTT | AACAGGGAAG | TGAGAGGGCC |
| 3050 | GCGGCAAAGC | CGTTTTTCCA | TAGGCTCCGC | CCCCCTGACA | AGCATCACGA |
| 3100 | AATCTGACGC | TCAAATCAGT | GGTGGCGAAA | CCCGACAGGA | CTATAAAGAT |
| 3150 | ACCAGGCGTT | TCCCCTGGCG | GCTCCCTCGT | GCGCTCTCCT | GTTCCTGCCT |
| 3200 | TTCGGTTTAC | CGGTGTCATT | CCGCTGTTAT | GGCCGCGTTT | GTCTCATTCC |
| 3250 | ACGCCTGACA | CTCAGTTCCG | GGTAGGCAGT | TCGCTCCAAG | CTGGACTGTA |
| 3300 | TGCACGAACC | CCCCGTTCAG | TCCGACCGCT | GCGCCTTATC | CGGTAACTAT |
| 3350 | CGTCTTGAGT | CCAACCCGGA | AAGACATGCA | AAAGCACCAC | TGGCAGCAGC |
| 3400 | CACTGGTAAT | TGATTTAGAG | GAGTTAGTCT | TGAAGTCATG | CGCCGGTTAA |
| 3450 | GGCTAAACTG | AAAGGACAAG | TTTTGGTGAC | TGCGCTCCTC | CAAGCCAGTT |
| 3500 | ACCTCGGTTC | AAAGAGTTGG | TAGCTCAGAG | AACCTTCGAA | AAACCGCCCT |
| 3550 | GCAAGGCGGT | TTTTTCGTTT | TCAGAGCAAG | AGATTACGCG | CAGACCAAAA |
| 3600 | CGATCTCAAG | AAGATCATCT | TATTAATCAG | ATAAAATATT | TCTAGATTTC |
| 3650 | AGTGCAATTT | ATCTCTTCAA | ATGTAGCACC | TGAAGTCAGC | CCCATACGAT |
| 3700 | ATAAGTTGTT | AATTCTCATG | TTTGACAGCT | TATCATCGAT | |

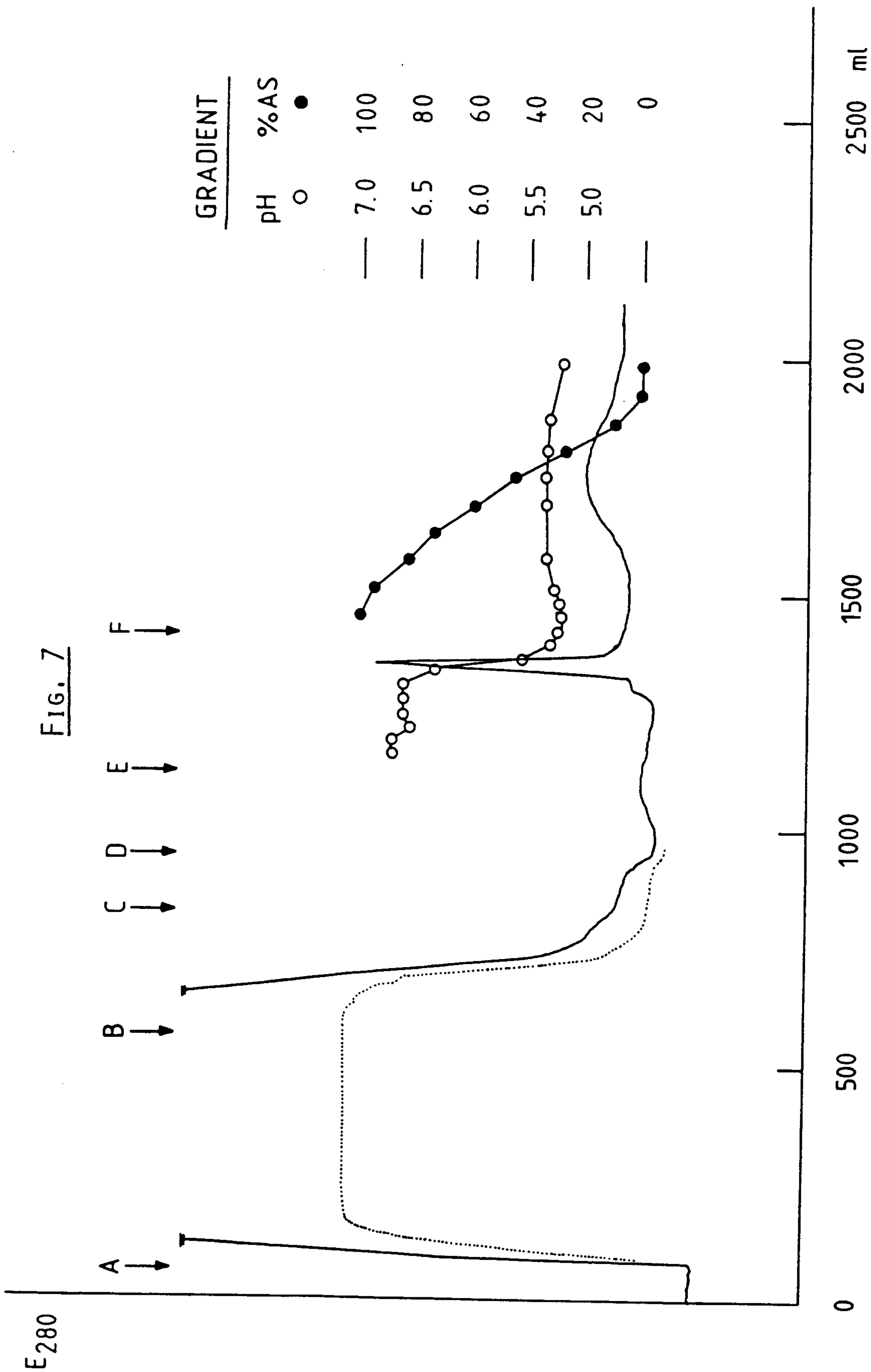
E280
A
B
C
D
E
F
GRADIENT
pH ○
%AS ●
7.0 100
6.5 80
6.0 60
5.5 40
5.0 20
0
0
500
1000
1500
2000
2500 ml

FIG. 7

Fig. 8

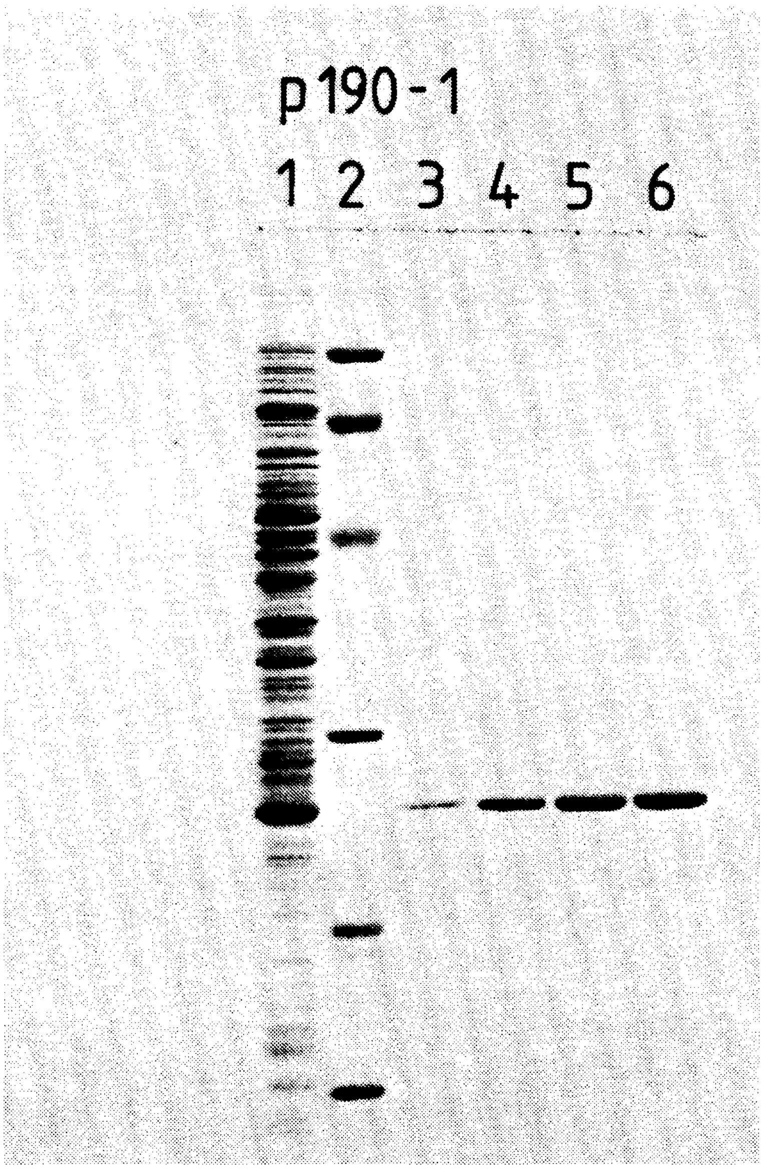

Fig. 9

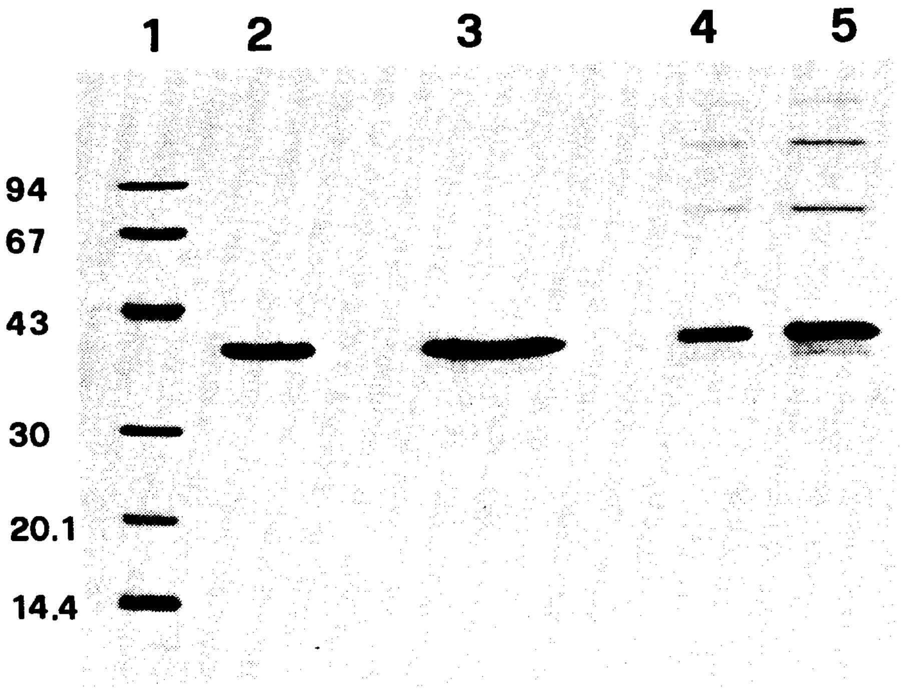

**Fig. 10**

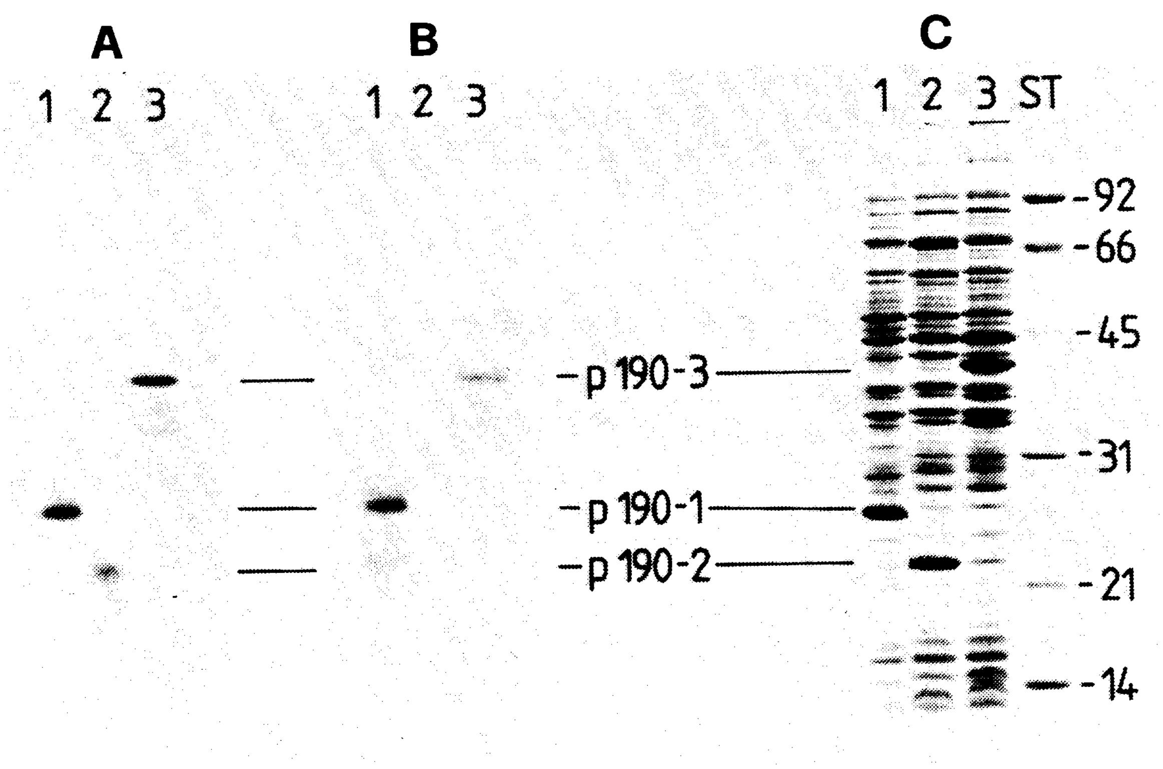

**Fig. 11**